# EUROPEAN PATENT APPLICATION

(11) **EP 4 670 789 A2**
(43) Date of publication of application: **31.12.2025**
(21) Application number: 25194955.8
(22) Date of filing: 19.06.2020
(51) Int. Cl.: A61P 19/10

(54) **MICROBIAL COMPOSITIONS AND METHODS FOR PRODUCING UPGRADED PROBIOTIC ASSEMBLAGES**

(30) Priority: 19.06.2019 US 201962863762 P; 19.06.2019 US 201962863722 P; 05.09.2019 WO PCT/US2019/049823
(62) Divisional of application: 20742977.0
(71) Applicant: Solarea Bio, Inc., Waltham, MA 02453 (US)
(72) Inventor: TOLEDO, Gerardo, V., Waltham, MA (US); SCHOTT, Eric, Michael, Waltham, MA (US); SOTO-GIRON, Maria, Juliana, Waltham, MA (US); KIM, Jinwoo, Waltham, MA (US); BUTTON, Julie, Waltham, MA (US)
(74) Representative: Heath, Abigail

(57) **Abstract**

The present invention relates to the identification of a group of microorganisms, which are relatively abundant in the microbial communities associated with fruits and vegetables typically consumed raw and therefore transient or permanent members of the human microbiota. These microbes are used to augment the effects of additional probiotic strains. The consumption of mixtures of these microbes at relevant doses will produce a beneficial effect in the host mediated in part by production of short chain fatty acids to enhance colonic butyrate production. Therapeutic methods of the invention involve the use of live microorganisms or metabolites derived from said microorganisms to establish a microbial composition in the mammalian host that will provide a health benefit to a mammal in need thereof.

## Description

### CROSS REFERENCE TO RELATED APPLICATIONS

This application claims the benefit of U.S. Provisional Application Nos. 62/863,722, filed June 19, 2019 and 62/863,762, filed June 19, 2019; and international application PCT/US2019/049823, filed September 5, 2019, each of which is hereby incorporated by reference in its entirety for all purposes.

### SEQUENCE LISTING

The instant application contains a Sequence Listing which has been submitted via EFS-Web and is hereby incorporated herein by reference in its entirety. Said ASCII copy, created on June 19, 2020, is named SBI-008WO_SL.txt, and is 382,219 bytes in size.

### BACKGROUND OF THE INVENTION

### Field of the invention

The invention relates to methods and compositions useful for producing upgraded probiotic assemblages.

### Description of the Related Art

Daily consumption of fresh fruits, vegetables, seeds and other plant-derived ingredients of salads and juices is recognized as part of a healthy diet and associated with weight loss, weight management and overall healthy life styles. This is demonstrated clinically and epidemiologically in the "China Study" (Campbell, T.C. and Campbell T.M. 2006. The China Study: startling implications for diet, weight loss and long-term health. Benbella books. pp 419) where a lower incidence of cardiovascular diseases, cancer and other inflammatory-related indications were observed in rural areas where diets are whole food plant-based. The benefit from these is thought to be derived from the vitamins, fiber, antioxidants and other molecules that are thought to benefit the microbial flora through the production of prebiotics. These can be in the form of fermentation products from the breakdown of complex carbohydrates and other plant-based polymers. There has been no clear mechanistic association between microbes in whole food plant-based diets and the benefits conferred by such a diet. The role of these microbes as probiotics, capable of contributing to gut colonization and thereby influencing a subject's microbiota composition in response to a plant-based diet, has been underappreciated.

Current probiotic technology does not take these ideas into account. Probiotics are heuristically combined according to what has previously been used, *Lactobacilli* and *Bifidobacteria* most prominently. What is needed are rationally-designed probiotic (beneficial microbes) and synbiotic (beneficial microbes combined with plant fibers) compositions and methods for designing such compositions, in order to generate more efficacious probiotics.

### SUMMARY OF THE INVENTION

The invention relates to probiotic compositions containing a plurality of viable bacteria and fungi isolated from fresh fruits, vegetables and fermented foods comprising at least one microbial entity classified as a gamma proteobacterium, fungus, or lactic acid bacterium, optionally selected from Table 4 or Table 7, at least one additional probiotic microorganism, and at least one prebiotic, wherein the prebiotic is optionally a fiber. In some aspects, the probiotic composition comprises at least one microbial entity classified as a fungus or yeast, optionally selected from Table 4 or Table 7. In some aspects, the probiotic composition further comprises at least one additional probiotic is a *Lactobacillus* or *Bifidobacterium* species. In some aspects, the additional probiotic strain is *Lactobacillus reuteri, Lactobacillus paracasei, Lactobacillus salivarius Lactobacillus plantarum, Lactobacillus acidophilus, Lactobacillus reuteri protectis, Lactobacillus bulgaricus, Lactobacillus rhamnosus, Lactobacillus casei, Lactobacillus delbreukeii, Bifidobacterium infantis, Bifidobacterium longum,* and *Bifidobacterium breve, Bifidobacterium bifidum, Bacillus cereus, Bacillus subtilis* or *Bacillus clausii, Bacillus coagulans, Clostridium butyricum, Akkermansia muciniphila, Hafnia alvei.*

In some aspects, the prebiotic contains a fiber and said fiber is oligofructose, or derived from a fiber source yielding a prebiotic fiber rich in oligofructose.
In some aspects, the microbes produce more short chain fatty acids (SCFAs) when grown together than when cultured separately as measured using gas chromatography with a Flame Ionization Detector (GC-FID), and wherein growth on the chosen prebiotic sugar results in increased synergy compared to growth on rich medium.

The invention also relates to an upgraded probiotic assemblage composition comprising a purified microbial population isolated from a first plant-based sample selected from samples in Table 3 artificially associated with a purified microbial population isolated from a second plant-based sample from selected from samples Table 3, a traditional probiotic strain, wherein the synthetic microbial consortia is capable of modulating the production of one or more branched chain fatty acids, short chain fatty acids, and/or flavones in a mammalian gut.

In some aspects, the invention relates to a fermented probiotic composition comprising a mixture of *Pediococcus pentosaceus* and/or *Leuconostoc mesenteroides* combined with non-lactic acid bacteria from Table 4 or Table 7, and an additional probiotic strain, the fermented probiotic being in a capsule or microcapsule adapted for enteric delivery.

In some aspects, the invention relates to heterologous microorganisms which can colonize the gastrointestinal tract of mammals and reduce free fatty acids absorbed into the body of a host by absorbing the free fatty acids in the gastrointestinal tract of mammals, wherein the heterologous microorganisms comprise genes encoding metabolic functions related to desirable health outcomes.

The invention also relates to a method for improving the efficacy of a probiotic strain, said method including administration of the probiotic strain along with any of the microbial and/or probiotic compositions described herein.

The invention also relates to a method to formulate an upgraded probiotic assemblage containing a purified microbial population isolated from a first plant-based sample selected from samples in Table 3 artificially associated with a purified microbial population isolated from a second plant-based sample from selected from samples Table 3, and an additional probiotic strain, wherein the purified bacterial population is predicted using a computational simulation and is capable of modulating production of one or more branched chain fatty acids, short chain fatty acids, and/or flavones in a mammalian gut.

According, provided for herein is a probiotic composition comprising a plurality of viable microbial entities, comprising: a first microbial entity, wherein the first microbial entity is a gamma proteobacterium, a fungus, or a lactic acid bacterium presented in Table 4 or Table 7; a second microbial entity, wherein the second microbial entity is a probiotic microorganism; and at least one prebiotic compound, wherein the prebiotic compound comprises a prebiotic polysaccharide or a prebiotic fiber, and wherein at least one of the first or second microbial entities comprises an agriculturally-derived microbial entity, and wherein the first and second microbial entities are distinct species.

In some aspects, the first and second microbial entity are capable of a first functional interaction. In some aspects, the first functional interaction comprises synthesis of a beneficial microbially-produced compound. In some aspects, the beneficial microbially-produced compound comprises a short chain fatty acid or vitamin selected from the group consisting of: acetate, propionate, butyrate, vitamin B12, K2, folate, and combinations thereof.

In some aspects, the composition comprises at least two distinct microbial species selected from Table 4 or Table 7. In some aspects, the composition comprises at least 3, at least 4, at least 5, or at least 6 distinct microbial species selected from Table 4 or Table 7.

In some aspects, the composition comprises at least one gamma proteobacterium, at least one fungus, and at least one lactic acid bacterium. In some aspects, each of the at least one gamma proteobacterium, the at least one fungus, and the at least one lactic acid bacterium are selected from Table 4 or Table 7.

In some aspects, the prebiotic compound is capable of being metabolized by at least one of the viable microbial entities present in the probiotic composition. In some aspects, the prebiotic compound is capable of being metabolized by the first or the second microbial entity. In some aspects, the prebiotic compound is capable of being metabolized to produce acetate, propionate, butyrate, or combinations thereof.

In some aspects, the second microbial entity is selected from the group consisting of: a *Lactobacillus sp.,* a *Bifidobacterium sp.,* a *Bacillus sp.,* and a *Clostridium sp..* In some aspects, the second microbial entity is selected from the group consisting of: *Lactobacillus reuteri, Lactobacillus paracasei, Lactobacillus salivarius Lactobacillus plantarum, Lactobacillus acidophilus, Lactobacillus reuteri protectis, Lactobacillus bulgaricus, Lactobacillus rhamnosus, Lactobacillus casei, Lactobacillus delbreukeii, Bifidobacterium infantis, Bifidobacterium longum, Bifidobacterium bifidum, Bifidobacterium breve, Streptococcus thermophilus, Escherichia coli Nissle, Lactococcus lactis, Bacillus subtilis, Bacillus clausii, Bacillus coagulans, Clostridium butyricum, Akkermansia muciniphila, Hafnia alvei,* and *Saccharomyces boulardii.* In some aspects, at least one of the viable microbial entities comprises a nucleic acid sequence having at least about 97% identity at the 16S or ITS locus with a sequence shown in SEQ ID NO: 1-170.

In some aspects, the first microbial entity is isolated from a food or food product. In some aspects, the first microbial entity is isolated from a fruit or vegetable. In some aspects, the first microbial entity is isolated from a pickled food. In some aspects, the first microbial entity is isolated from a fermented food. In some aspects, the first microbial entity is isolated from a raw agricultural product.

In some aspects, the first microbial entity is isolated from edible tissues of an agricultural product. In some aspects, the first microbial entity is isolated from an extract, fraction, tissue or portion of an agricultural product.

In some aspects, the second microbial entity is a generally regarded as safe (GRAS) probiotic microorganism or probiotic microorganism approved for human consumption. In some aspects, the second microbial entity is *Lactobacillus plantarum.* In some aspects, the second microbial entity is *Lactobacillus brevis.*

Also provided for herein is a probiotic assemblage comprising a first purified microbial entity isolated from a first plant-based sample selected from the group consisting of the samples presented in Table 3, a second purified microbial entity isolated from a second plant-based sample from selected from the group consisting of the samples presented in Table 3, wherein the first purified microbial entity is artificially-associated with the second purified microbial entity, and a third microbial entity, wherein the third microbial entity is a probiotic microorganism.

In some aspects, the composition is capable of a first functional interaction. In some aspects, the first functional interaction comprises synthesis of a beneficial microbially-produced compound. In some aspects, the wherein the beneficial microbially-produced compound comprises a short chain fatty acid or vitamin selected from the group consisting of: acetate, propionate, butyrate, vitamin B12, K2, folate, and combinations thereof.

In some aspects, the composition further comprises a prebiotic polysaccharide or a prebiotic fiber. In some aspects, the prebiotic polysaccharide is oligofructose or fructooligosaccharide. In some aspects, the prebiotic polysaccharide comprises a plant or plant extract. In some aspects, the prebiotic polysaccharide or the prebiotic fiber is capable of being metabolized by at least one microbial entities present in the probiotic composition, optionally by the first and/or the second purified microbial entities. In some aspects, the prebiotic polysaccharide or the prebiotic fiber is capable of being metabolized to produce acetate, propionate, butyrate or combinations thereof.

In some aspects, the probiotic microorganism is selected from the group consisting of: a *Lactobacillus sp.,* a *Bifidobacterium sp.,* a *Bacillus sp.,* or a *Clostridium sp.,* optionally wherein the probiotic microorganism is selected from the group consisting of: *Lactobacillus reuteri, Lactobacillus paracasei, Lactobacillus salivarius Lactobacillus plantarum, Lactobacillus acidophilus, Lactobacillus reuteri protectis, Lactobacillus bulgaricus, Lactobacillus rhamnosus, Lactobacillus casei, Lactobacillus delbreukeii, Bifidobacterium infantis, Bifidobacterium longum, Bifidobacterium bifidum, Bifidobacterium breve, Streptococcus thermophilus, Escherichia coli Nissle, Lactococcus lactis, Bacillus subtilis, Bacillus clausii, Bacillus coagulans, Clostridium butyricum, Akkermansia muciniphila, Hafnia alvei,* and *Saccharomyces boulardii.*

In some aspects, at least one of the first or the second purified microbial entities comprises a nucleic acid sequence having at least about 97% identity at the 16S or ITS locus with a sequence shown in SEQ ID NO: 1-170.

Also provided for herein is a probiotic composition comprising at least one probiotic microorganism, and at least one second microbial entity classified as a gamma proteobacterium, fungus, or lactic acid bacterium, optionally wherein the at least one second microbial entity is as a gamma proteobacterium, fungus, or lactic acid bacterium presented in Table 4 or Table 7, and wherein the at least one probiotic microorganism and the at least one second microbial entity are capable of combining with a food product to become a functional food.

In some aspects, the composition further comprises a prebiotic polysaccharide or a prebiotic fiber providing a carrier for the probiotic composition and to enhance the taste and flavor properties of the food product. In some aspects, the composition further comprises a prebiotic polysaccharide or a prebiotic fiber in dry or liquid form, wherein the prebiotic polysaccharide or the prebiotic fiber acts as a cryoprotectant.

In some aspects, the probiotic composition can be combined with a dairy product, optionally wherein the dairy product is selected from the group consisting of: yogurt, shake, smoothie, and cheese.

In some aspects, the prebiotic polysaccharide or the prebiotic fiber is in powdered form, and optionally provides flavorings, and wherein the prebiotic polysaccharide or the prebiotic fiber is capable of being mixed in a liquid to flavor the liquid, optionally wherein the flavor is selected from the group consisting of grape, strawberry, cranberry, blueberry, lime, lemon, and chocolate. In some aspects, the prebiotic polysaccharide or the prebiotic fiber comprises a plant or plant extract in the form of a juice, smoothie, shake, dry powder, tablet, granules, pellet, emulsion, paste, jelly, ferment, syrup, or brine. In some aspects, the prebiotic polysaccharide or the prebiotic fiber is derived from an agricultural product farmed using organic practices.

In some aspects, the at least one probiotic microorganism is selected from the group consisting of: a *Lactobacillus sp.,* a *Bifidobacterium sp.,* a *Bacillus sp.,* and a *Clostridium sp.*

Also provided for herein is a fermented probiotic composition comprising a *Pediococcus pentosaceus* microbial entity and/or a *Leuconostoc mesenteroides* microbial entity, a non-lactic acid bacteria microbial entity presented in Table 4 or Table 7, and a third microbial entity comprising a probiotic microorganism, wherein the fermented probiotic composition is encapsulated in a capsule or a microcapsule adapted for enteric delivery.

In some aspects, the composition further comprises a prebiotic polysaccharide or a prebiotic fiber. In some aspects, the prebiotic polysaccharide is oligofructose or fructooligosaccharide. In some aspects, the prebiotic polysaccharide comprises a plant or plant extract. In some aspects, the prebiotic polysaccharide or the prebiotic fiber is capable of being metabolized by at least one of the microbial entities present in the fermented probiotic composition. In some aspects, the prebiotic polysaccharide or the prebiotic fiber is capable of being metabolized by at least one of the microbial entities present in the probiotic composition to produce acetate, propionate, butyrate, or combinations thereof.

Also provided for herein is a fermented probiotic composition comprising a *Pichia kudriavzevii* microbial entity and/or a *Leuconostoc mesenteroides* microbial entity, a non-lactic acid bacteria microbial entity presented in Table 4 or Table 7, and a third microbial entity comprising a probiotic microorganism, wherein the fermented probiotic composition is encapsulated in a capsule or a microcapsule adapted for enteric delivery.

In some aspects, the composition further comprises a prebiotic polysaccharide or a prebiotic fiber. In some aspects, the prebiotic polysaccharide is oligofructose or fructooligosaccharide. In some aspects, the prebiotic polysaccharide comprises a plant or plant extract. In some aspects, the prebiotic polysaccharide or the prebiotic fiber is capable of being metabolized by at least one of the microbial entities present in the fermented probiotic composition. In some aspects, the prebiotic polysaccharide or the prebiotic fiber is capable of being metabolized by at least one microbial entity present in the probiotic composition to produce acetate, propionate, butyrate, or combinations thereof.

Also provided for herein is any of the compositions provided herein further comprising a drug. In some aspects, the drug is selected from the group consisting of: Metformin, Acarbose, Miglitol, Voglibose, Sitagliptin, Saxagliptin, Liraglutide, Pioglitazone, dipeptidyl peptidase-4 (DPP4)-inhibitors, glucagon-like peptide-1 (GLP-1) receptor analogs, alpha glucosidase inhibitors, thiazolidinedione, sodium/glucose cotransporter 2 (SGLT2) inhibitors, bisphosphonates, biologics (, selective estrogen receptor mediators, and anabolic agents.

Also provided for herein is a method for improving the efficacy of a probiotic microorganism in improving or maintaining health in a human subject, the method comprising administering any of compositions provided herein.

Also provided for herein is a method for treating Type 2 diabetes, the method comprising administering any of the above compositions.

Also provided for herein is a method for treating osteoporosis, the method comprising administering any of the above compositions.

Also provided for herein is a method for improving the efficacy of a probiotic microorganism in improving or maintaining health in a human subject, the method comprising co-administration of the probiotic microorganism to the subject in combination with an upgraded probiotic composition comprising a microbial entity, wherein the microbial entity is a gamma proteobacterium, a fungus, or a lactic acid bacterium presented in Table 4 or Table 7, and wherein the upgraded probiotic composition comprises a prebiotic polysaccharide or a prebiotic fiber.

Also provided for herein is a method for treating Type 2 diabetes, the method comprising co-administration of the probiotic microorganism to the subject in combination with an upgraded probiotic composition comprising a microbial entity, wherein the microbial entity is a gamma proteobacterium, a fungus, or a lactic acid bacterium presented in Table 4 or Table 7, and wherein the upgraded probiotic composition comprises a prebiotic polysaccharide or a prebiotic fiber.

Also provided for herein is a method for treating osteoporosis, the method comprising co-administration of the probiotic microorganism to the subject in combination with an upgraded probiotic composition comprising a microbial entity, wherein the microbial entity is a gamma proteobacterium, a fungus, or a lactic acid bacterium presented in Table 4 or Table 7, and wherein the upgraded probiotic composition comprises a prebiotic polysaccharide or a prebiotic fiber. In some aspects, the probiotic microorganism and the upgraded probiotic composition are co-formulated.

Also provided for herein is a method to formulate an upgraded probiotic assemblage, wherein the method comprises artificially-associating a first purified microbial entity isolated from a first plant-based sample selected from the group consisting of the samples presented in Table 3, a second purified microbial entity isolated from a second plant-based sample from selected from the group consisting of the samples presented in Table 3, and a third microbial entity, wherein the third microbial entity is a probiotic microorganism, wherein the upgraded probiotic assemblage is predicted using a computational simulation to modulate production of one or more branched chain fatty acids, short chain fatty acids, and/or flavones in a mammalian gut.

In some aspects, the probiotic microorganism is selected from the group consisting of: *Lactobacillus reuteri, Lactobacillus paracasei, Lactobacillus salivarius Lactobacillus plantarum, Lactobacillus acidophilus, Lactobacillus reuteri protectis, Lactobacillus bulgaricus, Lactobacillus rhamnosus, Lactobacillus casei, Lactobacillus delbreukeii, Bifidobacterium infantis, Bifidobacterium longum, Bifidobacterium bifidum, Bifidobacterium breve, Streptococcus thermophilus, Escherichia coli Nissle, Lactococcus lactis, Bacillus subtilis, Bacillus clausii, Bacillus coagulans, Clostridium butyricum, Akkermansia muciniphila, Hafnia alvei,* and *Saccharomyces boulardii.* In some aspects, the probiotic microorganism is a generally regarded as safe (GRAS) probiotic microorganism. In some aspects, the probiotic microorganism is approved for human consumption. In some aspects, the probiotic microorganism is *Lactobacillus plantarum.* In some aspects, the probiotic microorganism is *Lactobacillus brevis.*

In some aspects, the prebiotic polysaccharide or the prebiotic fiber acts as a cryoprotectant. In some aspects, no other cryoprotectant is present other than the prebiotic polysaccharide or the prebiotic fiber.

In some aspects, the microbial entity selected from Table 4 or Table 7 comprises a nucleic acid sequence having at least 97% identity at the 16S or ITS locus with a sequence shown in SEQ ID NO: 1-170. In some aspects, the microbial entity selected from Table 4 or Table 7 comprises a nucleic acid sequence having at least 98% identity at the 16S or ITS locus with a sequence shown in SEQ ID NO: 1-170. In some aspects, the microbial entity selected from Table 4 or Table 7 comprises a nucleic acid sequence having at least 99% identity at the 16S or ITS locus with a sequence shown in SEQ ID NO: 1-170. In some aspects, the microbial entity selected from Table 4 or Table 7 comprises a nucleic acid sequence having 100% identity at the 16S or ITS locus with a sequence shown in SEQ ID NO: 1-170.

In some aspects, each of the microbial entities selected from Table 4 or Table 7 comprise a nucleic acid sequence having at least 97% identity at the 16S or ITS locus with a sequence shown in SEQ ID NO: 1-170. In some aspects, each of the microbial entities selected from Table 4 or Table 7 comprise a nucleic acid sequence having at least 98% identity at the 16S or ITS locus with a sequence shown in SEQ ID NO: 1-170. In some aspects, each of the microbial entities selected from Table 4 or Table 7 comprise a nucleic acid sequence having at least 99% identity at the 16S or ITS locus with a sequence shown in SEQ ID NO: 1-170. In some aspects, each of the microbial entities selected from Table 4 or Table 7 comprise a nucleic acid sequence having 100% identity at the 16S or ITS locus with a sequence shown in SEQ ID NO: 1-170.

In some aspects, the microbial entities selected from Table 4 or Table 7 comprise each of the microbial entities having a nucleic acid sequence having at least 97% identity at the 16S or ITS locus with SEQ ID NO: 1, SEQ ID NO: 157, SEQ ID NO: 158, SEQ ID NO: 163, and SEQ ID NO: 165. In some aspects, the microbial entities selected from Table 4 or Table 7 comprise each of the microbial entities having a nucleic acid sequence having at least 98% identity at the 16S or ITS locus with SEQ ID NO: 1, SEQ ID NO: 157, SEQ ID NO: 158, SEQ ID NO: 163, and SEQ ID NO: 165. In some aspects, the microbial entities selected from Table 4 or Table 7 comprise each of the microbial entities having a nucleic acid sequence having at least 99% identity at the 16S or ITS locus with SEQ ID NO: 1, SEQ ID NO: 157, SEQ ID NO: 158, SEQ ID NO: 163, and SEQ ID NO: 165. In some aspects, the microbial entities selected from Table 4 or Table 7 comprise each of the microbial entities having a nucleic acid sequence having 100% identity at the 16S or ITS locus with SEQ ID NO: 1, SEQ ID NO: 157, SEQ ID NO: 158, SEQ ID NO: 163, and SEQ ID NO: 165.

### BRIEF DESCRIPTION OF THE SEVERAL VIEWS OF THE DRAWINGS

These and other features, aspects, and advantages of the present invention will become better understood with regard to the following description, and accompanying drawings, where:
**Figures 1 A-L** show plots depicting the diversity of microbial species detected in samples taken from 12 plants usually consumed raw by humans.
**Figure 1A** shows bacterial diversity observed in a green chard.
**Figure 1B** shows bacterial diversity in red cabbage.
**Figure 1C** shows bacterial diversity in romaine lettuce.
**Figure 1D** shows bacterial diversity in celery sticks.
**Figure 1E** shows bacterial diversity observed in butterhead lettuce grown hydroponically.
**Figure 1F** shows bacterial diversity in organic baby spinach.
**Figure 1G** shows bacterial diversity in green crisp gem lettuce
**Figure 1H** shows bacterial diversity in red oak leaf lettuce.
**Figure 1I** shows bacterial diversity in green oak leaf lettuce.
**Figure 1J** shows bacterial diversity in cherry tomatoes.
**Figure 1K** shows bacterial diversity in crisp red gem lettuce.
**Figure 1L** shows bacterial diversity in broccoli juice.
**Figures 2 A-C** show graphs depicting the taxonomic composition of microbial samples taken from broccoli heads (Fig. 2A), blueberries (Fig. 2B), and pickled olives (Fig. 2C).
**Figure 3** shows a schematic describing a human gut simulator experiment. The experiment comprises an *in vitro* system that represents various sections of the gastrointestinal tract. Isolates of interest are incubated in the presence of conditions that mimic particular stresses in the gastro-intestinal tract (such as low pH or bile salts), heat shock, or metformin. After incubation, surviving populations are recovered. Utilizing this system, the impact of various oral anti-diabetic therapies alone or in combination with probiotic cocktails of interest on the microbial ecosystem is tested.
**Figure 4** Genome-wide metabolic model for a DMA formulated in silico with 3 DP strains and one genome from a reference in NCBI. The predicted fluxes for acetate, propionate and butyrate under a nutrient-replete and plant fiber media are indicated.
**Figure 5** DMA experimental validation for a combination of strains DP3 and DP9 under nutrient replete and plant fiber media showing that the strains show synergy for increased SCFA production only under plant fiber media but not under rich media.
**Figure 6** Ovariectomized (OVX) mice were treated with either water (OVX), DMA4, or DMA 5 for six-weeks post-surgery. Mice received DXA scans before surgery and six-weeks post-surgery to determine the percent change in bone mineral density (A,B). DXA scans reveal a significant protection against OVX-induced bone loss by DMA5 at the lumbar spine (B), but no effect was observed when treated with DMA4 (A). Significant differences between groups in A and B were identified via 1-way ANOVA with a Tukey multiple comparison post-test (*P < 0.05, **P < 0.0005, ***P < 0.0001).
**Figure 7** Genomic analyses for glycosyl hydrolases in strains used in DMA5 and compared to reference probiotic microorganisms.
**Figure 8** Acetate production by DMA4 and DMA5 and their individual strains measured by gas chromatography.
**Figure 9** Acetate production by DMA 5 when combined with individual Bifidobacteria strains.
**Figure 10** Comparison of glycosyl hydrolase families between strains in DMA4 and DMA5.
**Figure 11** Differential abundance of glycosyl transferase families at week 6 between DMA4 and DMA5 stool metagenomes.
**Figure 12** Relative abundance values of L-rhamnose degradation pathway at week 6 between DMA4 and DMA5.
**Figure 13** Differential abundant metabolic pathways at week 6 between DMA4 and DMA5.
**Figure 14** Comparison of enzymes involved in vitamin biosynthesis identified in microbial strains from DMA4 and DMA5.

### DETAILED DESCRIPTION

### Definitions

Terms used in the claims and specification are defined as set forth below unless otherwise specified.

The term "ameliorating" refers to any therapeutically beneficial result in the treatment of a disease state, e.g., a metabolic disease state, including prophylaxis, lessening in the severity or progression, remission, or cure thereof.

The term *"in situ"* refers to processes that occur in a living cell growing separate from a living organism, e.g., growing in tissue culture.

The term *"in vivo"* refers to processes that occur in a living organism.

The term "mammal" as used herein includes both humans and non-humans and includes but is not limited to humans, non-human primates, canines, felines, murines, bovines, equines, and porcines.

As used herein, the term "derived from" includes microbes immediately taken from an environmental sample and also microbes isolated from an environmental source and subsequently grown in pure culture.

The term "percent identity," in the context of two or more nucleic acid or polypeptide sequences, refers to two or more sequences or subsequences that have a specified percentage of nucleotides or amino acid residues that are the same, when compared and aligned for maximum correspondence, as measured using one of the sequence comparison algorithms described below (e.g., BLASTP and BLASTN or other algorithms available to persons of skill) or by visual inspection. Depending on the application, the percent "identity" can exist over a region of the sequence being compared, e.g., over a functional domain, or, alternatively, exist over the full length of the two sequences to be compared. In some aspects, percent identity is defined with respect to a region useful for characterizing phylogenetic similarity of two or more organisms, including two or more microorganisms. Percent identity, in these circumstances can be determined by identifying such sequences within the context of a larger sequence, that can include sequences introduced by cloning or sequencing manipulations such as, e.g., primers, adapters, etc., and analyzing the percent identity in the regions of interest, without including in those analyses introduced sequences that do not inform phylogenetic similarity.

For sequence comparison, typically one sequence acts as a reference sequence to which test sequences are compared. When using a sequence comparison algorithm, test and reference sequences are input into a computer, subsequence coordinates are designated, if necessary, and sequence algorithm program parameters are designated. The sequence comparison algorithm then calculates the percent sequence identity for the test sequence(s) relative to the reference sequence, based on the designated program parameters.

Optimal alignment of sequences for comparison can be conducted, e.g., by the local homology algorithm of Smith & Waterman, Adv. Appl. Math. 2:482 (1981), by the homology alignment algorithm of Needleman & Wunsch, J. Mol. Biol. 48:443 (1970), by the search for similarity method of Pearson & Lipman, Proc. Nat'l. Acad. Sci. USA 85:2444 (1988), by computerized implementations of these algorithms (GAP, BESTFIT, FASTA, and TFASTA in the Wisconsin Genetics Software Package, Genetics Computer Group, 575 Science Dr., Madison, Wis.), or by visual inspection (see generally Ausubel et al., infra).

One example of an algorithm that is suitable for determining percent sequence identity and sequence similarity is the BLAST algorithm, which is described in Altschul et al., J. Mol. Biol. 215:403-410 (1990). Software for performing BLAST analyses is publicly available through the National Center for Biotechnology Information.

The term "sufficient amount" means an amount sufficient to produce a desired effect, e.g., an amount sufficient to alter the microbial content of a subject's microbiota.

The term "therapeutically effective amount" is an amount that is effective to ameliorate a symptom of a disease. A therapeutically effective amount can be a "prophylactically effective amount" as prophylaxis can be considered therapy.

As used herein the term "method" refers to manners, means, techniques and procedures for accomplishing a given task including, but not limited to, those manners, means, techniques and procedures either additional to, or readily developed from additional manners, means, techniques and procedures by practitioners of the chemical, pharmacological, biological, biochemical and medical arts.

As used herein, the term "treating" includes abrogating, inhibiting substantially, slowing, or reversing the progression of a condition, substantially ameliorating clinical or aesthetical symptoms of a condition.

As used herein, the term "preventing" includes completely or substantially reducing the likelihood or occurrence or the severity of initial clinical or aesthetical symptoms of a condition.

As used herein, the term "about" includes variation of up to approximately +/- 10% and that allows for functional equivalence in the product.

As used herein, the term "colony-forming unit" or "cfu" is an individual cell that is able to clone itself into an entire colony of identical cells.

As used herein all percentages are weight percent unless otherwise indicated.

As used herein, "viable organisms" are organisms that are capable of growth and multiplication. In some embodiments, viability can be assessed by numbers of colony-forming units that can be cultured. In some embodiments, viability can be assessed by other means, such as quantitative polymerase chain reaction.

The term "derived from" includes material isolated from the recited source, and materials obtained using the isolated materials (e.g., cultures of microorganisms made from microorganisms isolated from the recited source).

"Microbiota" refers to the community of microorganisms that occur (sustainably or transiently) in and on an animal or plant subject, typically a mammal such as a human, including eukaryotes, archaea, bacteria, and viruses (including bacterial viruses i.e., phage).

"Microbiome" refers to the genetic content of the communities of microbes that live in and on the human body, both sustainably and transiently, including eukaryotes, archaea, bacteria, and viruses (including bacterial viruses (i.e., phage)), wherein "genetic content" includes genomic DNA, RNA such as ribosomal RNA, the epigenome, plasmids, and all other types of genetic information.

The term "subject" refers to any animal subject including humans, laboratory animals (e.g., primates, rats, mice), livestock (e.g., cows, sheep, goats, pigs, turkeys, and chickens), and household pets (e.g., dogs, cats, and rodents). The subject may be suffering from a dysbiosis, including, but not limited to, an infection due to a gastrointestinal pathogen or may be at risk of developing or transmitting to others an infection due to a gastrointestinal pathogen.

The "colonization" of a host organism includes the non-transitory residence of a bacterium or other microscopic organism. As used herein, "reducing colonization" of a host subject's gastrointestinal tract (or any other microbial niche) by a pathogenic bacterium includes a reduction in the residence time of the pathogen in the gastrointestinal tract as well as a reduction in the number (or concentration) of the pathogen in the gastrointestinal tract or adhered to the luminal surface of the gastrointestinal tract. Measuring reductions of adherent pathogens may be demonstrated, e.g., by a biopsy sample, or reductions may be measured indirectly, e.g., by measuring the pathogenic burden in the stool of a mammalian host.

A "combination" of two or more bacteria includes the physical co-existence of the two bacteria, either in the same material or product or in physically connected products, as well as the temporal co-administration or co-localization of the two bacteria.

As used herein "heterologous" designates organisms to be administered that are not naturally present in the same proportions as in the therapeutic composition as in subjects to be treated with the therapeutic composition. These can be organisms that are not normally present in individuals in need of the composition described herein, or organisms that are not present in sufficient proportion in said individuals. These organisms can comprise a synthetic composition of organisms derived from separate plant sources or can comprise a composition of organisms derived from the same plant source, or a combination thereof.

"Controlled-release" refers to delayed release of an agent, from a composition or dosage form in which the agent is released according to a desired profile in which the release occurs after a period of time.

Throughout this application, various embodiments of this invention can be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range.

It is appreciated that certain features of the invention, which are, for clarity, described in the context of separate embodiments, can also be provided in combination in a single embodiment. Conversely, various features of the invention, which are, for brevity, described in the context of a single embodiment, can also be provided separately or in any suitable subcombination or as suitable in any other described embodiment of the invention. Certain features described in the context of various embodiments are not to be considered essential features of those embodiments, unless the embodiment is inoperative without those elements.

The term "agriculturally-derived" means a microorganism isolated from an edible crop, a fermented food from such edible crop or products from animal origin in a farm setting such as dairy.

The term "organic farming" refers to an alternative agricultural system avoiding the use of agrochemicals including fertilizers and pesticides and substitutes these with compost and other natural practices. In a non-limiting illustrative example, USDA standards for organic practices require:
- Land must have had no prohibited substances applied to it for at least 3 years before the harvest of an organic crop
- Soil fertility and crop nutrients will be managed through tillage and cultivation practices, crop rotations, and cover crops, supplemented with animal and crop waste materials and allowed synthetic materials
- Crop pests, weeds, and diseases will be controlled primarily through management practices including physical, mechanical, and biological controls. When these practices are not sufficient, a biological, botanical, or synthetic substance approved for use on the National List may be used
- Operations must use organic seeds and other planting stock when available
- The use of genetic engineering, ionizing radiation and sewage sludge is prohibited

The term "extract, fraction, tissue or portion of a plant" refers to the edible and non-edible components of a fresh fruit, vegetable or fermented food from which a subsample is collected for the isolation of bacteria and fungi.

The term "food" refers to edible nutritious substance that people or animals eat or drink in order to maintain life and growth.

The term "functional foods" refer to a food that has been enriched or fortified by the addition of other nutritional ingredients not found in the unenriched food item. The fortification can be done by the addition of vitamins, minerals and microorganisms (*e.g.,* a probiotic microorganism and/or any of the microorganism described herein).

The term "medical foods" are foods that are specially formulated and intended for the dietary management of a disease that has distinctive nutritional needs that cannot be met by normal diet alone

The tenn "probiotic microorganism" refers to a bacterium or fungus that when consumed live provides a health benefit to the consumer generally by improving or restoring the gut flora. Preferred probiotic microorganisms include microorganisms that are generally regarded as safe ("GRAS"), included certified GRAS microorganisms.

The term "probiotic composition" refers to compositions that include one or more probiotic microorganisms.

The term "prebiotic compounds" refer to a variety of compounds such as plant polymers, plant fibers, vitamins carbohydrate polymers found in dairy and other molecules that can act as substrates for microbial degradation and production of short chain fatty acids and other fermentable substrates.

The term "synbiotic" refers to a combination of probiotic microorganisms and prebiotic compounds in the same preparation and delivered together so the prebiotic compounds are utilized by the probiotic microorganisms.

The term "postbiotic" refers to probiotic cells that have been killed by heat or other means and are delivered to the consumer as dead cells.

"Beneficial microbially-produced compound" is any microbially-produced molecule that has a positive effect on human health and can be recognized as part of the use of "upgraded probiotic assemblages."

The term "upgraded probiotic assemblages" refers to a combination of one or more microorganisms reported here in Table 4 and Table 7 and prebiotic compounds that when combined with a probiotic composition show a clear health beneficial effect.

The term "functional interaction" means the resulting effect when two or more bacteria and/or fungi growing in a culture vessel exchange substrates and products of metabolism with a resulting improved synergistic production of a compound relative to the capacity of each of the bacteria and/or fungi grown alone; for example increased short chain fatty acid production measured by gas chromatography and the amount of short chain fatty acid by the co-cultured bacteria and/or fungi is greater than the sum of the short chain fatty acid production by the individual bacteria and/or fungi.

The term "artificially associated" means the use of rational approaches to combine microorganisms that do not naturally co-exists in the same sample, or where no demonstrations of spontaneous chance encounters, assemblies as consortium, and/or synergistic interactions are known or previously described.

The term "fermented probiotic composition" refers to an agricultural product that is subject to a natural process of microbial transformation to produce short chain fatty acids as part of preservation or flavor and that can be enriched with other microorganisms to enhance one of its properties.

It must be noted that, as used in the specification and the appended claims, the singular forms "a," "an" and "the" include plural referents unless the context clearly dictates otherwise.

As used herein "GOS" indicates one or more galacto-oligosaccharides and "FOS" indicates one or more fructo-oligosaccharide.

The following abbreviations are used in this specification and/or Figures: ac = acetic acid; but = butyric acid; ppa = propionic acid.

### Methods of the invention

The administration of the microbial composition can be accomplished orally or rectally, although administration is not limited to these methods. In some embodiments, the microbial composition is administered orally. In some embodiments, the microbial composition is delivered rectally. In some embodiments, the administration of the microbial composition occurs at regular intervals. In some embodiments, the administration occurs daily.

The microbial composition can be administered via typical pharmacological means, such as slurries, capsules, microcapsules, or solutions, although means of administration are not limited to these methods. In some embodiments, an enteric capsule or enteric microcapsule is used. In some embodiments the pharmaceutical composition involving the microbial composition described herein will be fresh or frozen prior to/upon application. In some embodiments, said pharmaceutical composition will be lyophilized or otherwise treated to increase stability or otherwise obtain a benefit from said treatment.

In some embodiments, the microbial composition is administered with an effective amount of an additional therapeutic agent or along with an effective drug regimen. For example in type 2 diabetes or metabolic syndrome, the microbial composition is administered along with anti-diabetic medications. These include, but are not limited to, metformin, Acarbose, Miglitol, Voglibose, Sitagliptin, Saxagliptin, Liraglutide, Pioglitazone, dipeptidyl peptidase-4 (DPP4)-inhibitors, glucagon-like peptide-1 (GLP-1) receptor analogs, alpha glucosidase inhibitors, thiazolidinedione, and sodium/glucose cotransporter 2 (SGLT2) inhibitors. In another example, the microbial composition is administered along with anti-osteoporosis medications including but not limited to, bisphosphonates (*e.g.,* alendronate, risedronate, ibandronate, zolendronate), biologics (*e.g.,* denosumab, romosozumab), selective estrogen receptor mediators (*e.g.,* Raloxifene), or anabolic agents (*e.g.,* teriparatide, abaloparatide).

In some embodiments, the probiotic composition is co-administered with an effective amount of an additional therapeutic agent or along with an effective drug regimen to improve the efficacy of said therapeutic agent or drug regimen.

In some embodiments, the probiotic composition is co-administered with an effective amount of an additional therapeutic agent or along with an effective drug regimen to reduce the side effects of said therapeutic agent or drug regimen.

### Compositions of the invention

In certain embodiments, compositions of the invention comprise probiotic compositions formulated for administration or consumption, with a prebiotic and any necessary or useful excipient. In other embodiments, compositions of the invention comprise probiotic compositions formulated for consumption without a prebiotic. Probiotic compositions of the invention are preferably isolated from foods normally consumed raw and isolated for cultivation. Preferably, microbes are isolated from different foods normally consumed raw, but multiple microbes from the same food source may be used.

It is additional to those of skill in the art how to identify microbial strains. Bacterial strains are commonly identified by 16S rRNA gene sequence. Fungal species can be identified by sequence of the internal transcribed space (ITS) regions of rDNA.

One of skill in the art will recognize that the 16S rRNA gene and the ITS region comprise a small portion of the overall genome, and so the sequence of the entire genome (whole genome sequence) may also be obtained and compared to additional species and/or strains.

Additionally, multi-locus sequence typing (MLST) is additional to those of skill in the art. This method uses the sequences of 7 additional bacterial genes, typically 7 housekeeping genes, to identify bacterial species based upon sequence identity of additional species as recorded in the publicly available PubMLST database. Housekeeping genes are genes involved in basic cellular functions.

In certain embodiments, bacterial entities of the invention are identified by comparison of the 16S rRNA sequence to those of additional bacterial species, as is well understood by those of skill in the art. In certain embodiments, fungal species of the invention are identified based upon comparison of the ITS sequence to those of additional species (Schoch et al PNAS 2012). In certain embodiments, microbial strains of the invention are identified by whole genome sequencing and subsequent comparison of the whole genome sequence to a database of additional microbial genome sequences. While microbes identified by whole genome sequence comparison, in some embodiments, are described and discussed in terms of their closest defined genetic match, as indicated by 16S rRNA sequence, it should be understood that these microbes are not identical to their closest genetic match and are novel microbial entities. This can be shown by examining the Average Nucleotide Identity (ANI) of microbial entities of interest as compared to the reference strain that most closely matches the genome of the microbial entity of interest. ANI is further discussed in example 6.

In other embodiments, microbial entities described herein are functionally equivalent to previously described strains with homology at the 16S rRNA or ITS region. In certain embodiments, functionally equivalent bacterial strains have 95% identity at the 16S rRNA region and functionally equivalent fungal strains have 95% identity at the ITS region. In certain embodiments, functionally equivalent bacterial strains have 96% identity at the 16S rRNA region and functionally equivalent fungal strains have 96% identity at the ITS region. In certain embodiments, functionally equivalent bacterial strains have 97% identity at the 16S rRNA region and functionally equivalent fungal strains have 97% identity at the ITS region. In certain embodiments, functionally equivalent bacterial strains have 98% identity at the 16S rRNA region and functionally equivalent fungal strains have 98% identity at the ITS region. In certain embodiments, functionally equivalent bacterial strains have 99% identity at the 16S rRNA region and functionally equivalent fungal strains have 99% identity at the ITS region. In certain embodiments, functionally equivalent bacterial strains have 99.5% identity at the 16S rRNA region and functionally equivalent fungal strains have 99.5% identity at the ITS region. In certain embodiments, functionally equivalent bacterial strains have 100% identity at the 16S rRNA region and functionally equivalent fungal strains have 100% identity at the ITS region.

The compositions disclosed herein are derived from edible plants and can comprise a mixture of microorganisms, comprising bacteria, fungi, archaea, and/or other indigenous or exogenous microorganisms, all of which work together to form a microbial ecosystem with a role for each of its members.

In certain embodiments, the probiotic composition may include selected microorganisms and other ingredients that have been approved by the United States Food and Drug Administration ("US FDA" or "FDA") and are Generally Recognized as Safe ("GRAS"). Accordingly, in embodiments where the probiotic composition is formulated to only include GRAS ingredients, the probiotic composition is suited for food products, food product contacting materials, and other compositions in which the probiotic composition will not contaminate a food or food product being produced. In certain embodiments, the probiotic composition may include selected microorganisms and other ingredients that have been approved by additional regulatory agencies and can also be considered to be Generally Recognized as Safe ("GRAS").

In some embodiments, species of interest are isolated from plant-based food sources normally consumed raw. These isolated compositions of microorganisms from individual plant sources can be combined to create a new mixture of organisms. Particular species from individual plant sources can be selected and mixed with other species cultured from other plant sources, which have been similarly isolated and grown. In some embodiments, species of interest are grown in pure cultures before being prepared for consumption or administration. In some embodiments, the organisms grown in pure culture are combined to form a synthetic combination of organisms.

In some embodiments, the microbial composition comprises proteobacteria or gamma proteobacteria. In some embodiments, the microbial composition comprises several species of *Pseudomonas.* In some embodiments, species from another genus are also present. In some embodiments, a species from the genus *Duganella* is also present. In some embodiments of said microbial composition, the population comprises at least three unique isolates selected from the group consisting of *Pseudomonas, Acinetobacter, Aeromonas, Curtobacterium, Escherichia, Lactobacillus, Leuconostoc, Pediococcus, Serratia, Streptococcus,* and *Stenotrophomonas.* In some embodiments, the bacteria are selected based upon their ability to modulate production of one or more branch chain fatty acids, short chain fatty acids, and/or flavones in a mammalian gut.

In some embodiments, microbial compositions comprise isolates that are capable of modulating production or activity of the enzymes involved in fatty acid metabolism, such as acetolactate synthase I, N-acetylglutamate synthase, acetate kinase, Acetyl-CoA synthetase, acetyl-CoA hydrolase, Glucan 1,4-alpha-glucosidase, or Bile acid symporter Acr3.

In some embodiments, the administered microbial compositions colonize the treated mammal's digestive tract. In some embodiments, these colonizing microbes comprise bacterial assemblages present in whole food plant-based diets. In some embodiments, these colonizing microbes comprise *Pseudomonas* with a diverse species denomination that is present and abundant in whole food plant-based diets. In some embodiments, these colonizing microbes reduce free fatty acids absorbed into the body of a host by absorbing the free fatty acids in the gastrointestinal tract of mammals. In some embodiments, these colonizing microbes comprise genes encoding metabolic functions related to desirable health outcomes such as increased efficacy of anti-diabetic treatments, lowered BMI, lowered inflammatory metabolic indicators, etc.

Some embodiments comprise bacteria that are not completely viable but act by releasing metabolites that act in the gastro-intestinal tract of a patient promoting weight loss, increased efficacy of diabetic regimens, bone health or other desirable outcome. Some embodiments comprise a prebiotic composition derived from metabolites present in whole food plant-based materials, identified and enriched as part of the formula for oral delivery.

### Prebiotics

Prebiotics, in accordance with the teachings of this invention, comprise compositions that promote the growth of beneficial bacteria in the intestines. Prebiotic substances can be consumed by a relevant probiotic microorganism, or otherwise assist in keeping the relevant probiotic microorganism alive or stimulate its growth. When consumed in an effective amount, prebiotics also beneficially affect a subject's naturally-occurring gastrointestinal microflora and thereby impart health benefits apart from just nutrition. Prebiotic foods enter the colon and serve as substrate for the endogenous bacteria, thereby indirectly providing the host with energy, metabolic substrates, and essential micronutrients. The body's digestion and absorption of prebiotic foods is dependent upon bacterial metabolic activity, which salvages energy for the host from nutrients that escaped digestion and absorption in the small intestine.

Prebiotics help probiotic microorganisms flourish in the gastrointestinal tract, and accordingly, their health benefits are largely indirect. Metabolites generated by colonic fermentation by intestinal microflora, such as short-chain fatty acids, can play important functional roles in the health of the host. Prebiotics can be useful agents for enhancing the ability of intestinal microflora to provide benefits to their host.

Prebiotics, in accordance with the embodiments of this invention, include, without limitation, mucopolysaccharides, oligosaccharides, polysaccharides, amino acids, vitamins, nutrient precursors, proteins, and combinations thereof.

According to particular embodiments, compositions comprise a prebiotic comprising a dietary fiber, including, without limitation, polysaccharides and oligosaccharides. These compounds have the ability to increase the number of probiotic microorganisms, and augment their associated benefits. For example, an increase of beneficial *Bifidobacteria* likely changes the intestinal pH to support the increase of *Bifidobacteria,* thereby decreasing pathogenic microorganisms.

Non-limiting examples of oligosaccharides that are categorized as prebiotics in accordance with particular embodiments include galactooligosaccharides, fructooligosaccharides, inulins, isomalto-oligosaccharides, lactilol, lactosucrose, lactulose, pyrodextrins, soy oligosaccharides, transgalacto-oligosaccharides, and xylo-oligosaccharides.

According to other particular embodiments, compositions comprise a prebiotic comprising an amino acid.

Prebiotics are found naturally in a variety of foods including, without limitation, cabbage, bananas, berries, asparagus, garlic, wheat, oats, barley (and other whole grains), flaxseed, tomatoes, Jerusalem artichoke, onions and chicory, greens (e.g., dandelion greens, spinach, collard greens, chard, kale, mustard greens, turnip greens), and legumes (e.g., lentils, kidney beans, chickpeas, navy beans, white beans, black beans). Generally, according to particular embodiments, compositions comprise a prebiotic present in a sweetener composition or functional sweetened composition in an amount sufficient to promote health and wellness.

In particular embodiments, prebiotics also can be added to high-potency sweeteners or sweetened compositions. Non-limiting examples of prebiotics that can be used in this manner include fructooligosaccharides, xylooligosaccharides, galactooligosaccharides, and combinations thereof.

Many prebiotics have been discovered from dietary intake including, but not limited to: antimicrobial peptides, polyphenols, Okara (soybean pulp by product from the manufacturing of tofu), polydextrose, lactosucrose, malto-oligosaccharides, gluco-oligosaccharides (GOS), fructo-oligosaccharides (FOS), xantho-oligosaccharides, soluble dietary fiber in general. Types of soluble dietary fiber include, but are not limited to, psyllium, pectin, or inulin. Phytoestrogens (plant-derived isoflavone compounds that have estrogenic effects) have been found to have beneficial growth effects of intestinal microbiota through increasing microbial activity and microbial metabolism by increasing the blood testosterone levels, in humans and farm animals. Phytoestrogen compounds include but are not limited to: Oestradiol, Daidzein, Formononetin, Biochainin A, Genistein, and Equol.

Dosage for the compositions described herein are deemed to be "effective doses," indicating that the probiotic or prebiotic composition is administered in a sufficient quantity to alter the physiology of a subject in a desired manner. In some embodiments, the desired alterations include reducing obesity, and or metabolic syndrome, and sequelae associated with these conditions. In some embodiments, the desired alterations are promoting rapid weight gain in livestock. In some embodiments, the prebiotic and probiotic microorganism compositions are given in addition to an anti-diabetic regimen.

### Methods of Use

### Combinations with additional probiotic microorganisms

The invention relates to rationally-designed probiotic compositions for enhancement of probiotic microorganisms, or formulated with additional probiotic microorganisms to produce upgraded probiotic assemblages. The use of probiotics in general is additional in the art, and one of skill in the art would understand that the methods described herein could be used to improve the efficacy of diverse probiotic compositions. Additional bacteria used as probiotic microorganisms are strains *of Lactobacillus* and *Bifidobacterium. Lactobacillus* species of interest include, but are not limited to, *Lactobacillus reuteri, Lactobacillus paracasei, Lactobacillus salivarius Lactobacillus plantarum, Lactobacillus acidophilus, Lactobacillus reuteri protectis, Lactobacillus bulgaricus, Lactobacillus rhamnosus, Lactobacillus casei,* and *Lactobacillus delbreukeii.* Bifidobacterial species of interest include, but are not limited to, *Bifidobacterium infantis, Bifidobacterium longum, Bifidobacterium bifidum* and *Bifidobacterium breve.* Other probiotic microorganisms of interest include *Streptococcus thermophilus, Escherichia coli Nissle, Lactococcus lactis, Bacillus subtilis, Bacillus clausii, Bacillus coagulans, Clostridium butyricum, Akkermansia muciniphila, Hafnia alvei* and *Saccharomyces boulardii.*

In some embodiments, the probiotic compositions described herein are formulated with one or more of the strains above, or strains substantially similar thereto. In some embodiments, the probiotic composition described herein is formulated for consumption along with a regimen comprising one or more of the strains above, or strains substantially similar thereto.

### Timing and Dose of Probiotic Microorganisms and Prebiotics

In an embodiment, probiotic microorganisms, such as *Lactobacillus, Leuconostoc, or Pediococcus,* are given prior to beginning treatment with a prebiotic compound. In an embodiment, probiotic microorganisms, such as *L. mesenteroides,* are given in conjunction with treatment with a prebiotic (e.g., comprising or consisting essentially of FOS, GOS, or other appropriate polysaccharide), for part or all of the duration of treatment with the prebiotic. Thus, in an embodiment, some or all doses of a prebiotic compound (e.g., comprising or consisting essentially of FOS, GOS, or other appropriate polysaccharide) are accompanied by a dose of bacteria or fungi, e.g., live cultured bacteria, e.g., *L. mesenteroides.* In an embodiment, bacteria or fungi, e.g., *L. mesenteroides,* are given initially with a prebiotic (e.g., comprising or consisting essentially of FOS, GOS, or other appropriate polysaccharide), but then use of the probiotic microorganism is discontinued. For example, the initial one, two, three, four, five, six, seven, eight, nine, ten, or more than ten days of treatment with a prebiotic (e.g., comprising or consisting essentially of FOS, GOS, or other appropriate polysaccharide) further comprises doses of bacteria or fungi, with the use of bacteria or fungi discontinued after that time. In an embodiment, bacteria or fungi, (e.g., bacteria in yogurt), or bacteria or fungi by themselves, can be given for the first two days of treatment; then the administration of bacteria or fungi is discontinued. In another embodiment, probiotic microorganisms, either alone or in combination with other substances or treatments, are used after the treatment with a prebiotic compound (comprising or consisting essentially of FOS, GOS, or other appropriate polysaccharide) is terminated. The bacteria or fungi can be taken for any suitable period after the termination of treatment with prebiotic and can be taken daily or at regular or irregular intervals. Doses can be as described below.

Any suitable amount of a probiotic microorganism per serving can be used that allows an effective microbiota in the GI as demonstrated by a reduction in weight or amelioration of other signs of metabolic syndrome measured by insulin resistance, HbA1c, body mass index (BMI), visceral adiposity, dyslipidemia, or bone mineral density. Typically, probiotic microorganisms are given as live cultured microorganisms. Herein measurement is mg indicate dry weight of purified bacteria or fungi. The dose can be about 0.001 mg to about 1 mg, or about 0.5 mg to about 5 mg, or about 1 mg to about 1000 mg, or about 2 mg to about 200 mg, or about 2 mg to about 100 mg, or about 2 mg to about 50 mg, or about 4 mg to about 25 mg, or about 5 mg to about 20 mg, or about 10 mg to about 15 mg, or about 50 mg to about 200 mg, or about 200 mg to about 1000 mg, or about 10, 11, 12, 12.5, 13, 14, or 15 mg per serving. In an embodiment, *L. mesenteroides* used in a dose of about 12.5 mg per serving. The probiotic microorganisms can also be about 0.5% w/w to about 20% w/w of the final composition. The dose of probiotic microorganisms can be given in combination with one or more prebiotics. Another common way of specifying the amount of probiotic microorganisms is as a colony forming unit (cfu). In an embodiment, one or more strains of probiotic microorganism are ingested in an amount of about 1x10^6 to about 1x10^9 cfu's, or about 1x10^6 cfu's to about 1x10^9 cfu's, or about 10x10^6 cfu's to about 0.5x10^9 cfu's, or about 113x10^5 cfu's to about 113x10^6 cfu's, or about 240x10^5 cfu's to about 240x10^6 cfu's, or about 0.3x10^9 cfu's per serving. In another embodiment, one or more strains of probiotic microorganism are administered as part of a dairy product. In an embodiment, a typical serving size for a dairy product such as fluid milk is about 240 g. In other embodiments, a serving size is about 245 g, or about 240 g to about 245 g, or about 227 to about 300 g. In an embodiment the dairy product is yogurt. Yogurt can have a serving size of about 4 oz, or about 6 oz, or about 8 oz, or about 4 oz to 10 oz, or about half cup, or about 1 cup, or about 113 g, or about 170 g, or about 227 g, or about 245 g or about 277 g, or about 100 g to about 350 g.

In an embodiment, probiotic microorganisms are given as live cultured bacteria or fungi, e.g., in combination with a prebiotic (e.g., comprising or consisting essentially of FOS, GOS, or other appropriate polysaccharide) and, optionally, other substances. The dose can be about 1 mg to about 1000 mg, or about 2 mg to about 200 mg, or about 2 mg to about 100 mg, or about 2 mg to about 50 mg, or about 4 mg to about 25 mg, or about 5 mg to about 20 mg, or about 10 mg to about 15 mg, or about 10, 11, 12, 12.5, 13, 14, or 15 mg of probiotic microorganismal cell culture dry weight. In an embodiment, *Lactobacillus (i.e. L. acidophilus), Leuconostoc (i.e. L. mesenteroides), or Pediococcus (i.e. P. pentosaceus),* is used in a dose of about 12.5 mg. In an embodiment, as the administration of a prebiotic (e.g., comprising or consisting essentially of FOS, GOS, or other appropriate polysaccharide) dose to a subject increases, the dose of bacteria or fungi increases as well. For example, an initial dose of a prebiotic (e.g., comprising or consisting essentially of FOS, GOS, or other appropriate polysaccharides) can be about 0.6 g to 1.0 g, e.g., 0.8 g, given in combination with about 10-15 mg, e.g., about 12.5 mg, of *L. mesenteroides.* The dose of a prebiotic (e.g., comprising or consisting essentially of FOS, GOS, or other appropriate polysaccharide) can be increased incrementally by about 0.6 g to 1.0 g, e.g., 0.8 g, and the accompanying dose of *L. mesenteroides* can be increased by about 10-15 mg, e.g., about 12.5 mg, of *L. mesenteroides.*

### FOS, GOS, and Other Appropriate Polysaccharide Formulations

### A. Formulations introduction

In an embodiment a prebiotic composition comprises inulin, FOS, lactulose, GOS, raffinose, stachyose, or a combination thereof. In addition, other plant-derived polysaccharides such as xylan, pectin, isomalto-oligosaccharides, gentio-oligosaccharides, 4-O-methyl glucuronoxylan (GX), neutral arabinoxylan (AX), heteroxylan (HX) can be combined with the probiotic microorganisms to enhance bacterial and fungal metabolic function. Some of these can be derived from plant material found in the plant host from which the probiotic microorganisms were isolated (i.e., the "cognate" plant). In some embodiments the prebiotics are thus adapted to be assimilated and digested by the accompanying probiotics in a manner that recapitulates the rich complexity and variety of polysaccharides present in the cognate plant and which play a role during digestion following its consumption of an animal.

In an embodiment a prebiotic composition comprises or consists of FOS, GOS, or other appropriate polysaccharide. In another embodiment a prebiotic composition comprises FOS, GOS, or other appropriate polysaccharide, in combination with one or more digestible saccharides. Digestible saccharides are saccharides that are digestible by humans and include, but are not limited to lactose, glucose, and galactose. In an embodiment a prebiotic composition comprises FOS, GOS, or other appropriate polysaccharide, and less than 20% weight/weight of one or more digestible saccharides (e.g. lactose, glucose, or galactose). In an embodiment a prebiotic composition comprises FOS, GOS, or other appropriate polysaccharide, and less than 10% of one or more digestible saccharides. In an embodiment a prebiotic composition comprises FOS, GOS, or other appropriate polysaccharide, and less than 5% of one or more digestible saccharides. In another embodiment a prebiotic composition contains less than 5% lactose. In another embodiment a prebiotic composition contains less than 4% lactose. In another embodiment a prebiotic composition contains less than 3% lactose. In another embodiment a prebiotic composition contains less than 2% lactose. In another embodiment a prebiotic composition contains less than 1% lactose. In another embodiment a prebiotic composition contains less than 0.5% lactose. In another embodiment a prebiotic composition contains less than 0.4% lactose. In another embodiment a prebiotic composition contains less than 0.3% lactose. In another embodiment a prebiotic composition contains less than 0.2% lactose. In another embodiment a prebiotic composition contains less than 0.1% lactose. In another embodiment a prebiotic composition contains less than 0.05% lactose. In another embodiment a prebiotic composition contains less than 0.01% lactose. In another embodiment a prebiotic composition contains less than 0.005% lactose. In an embodiment a prebiotic composition comprises FOS, GOS, or other appropriate polysaccharide, and essentially no lactose. In an embodiment a prebiotic composition does not contain any lactose. In another embodiment a prebiotic composition contains FOS, GOS, or other appropriate polysaccharide, and at least one probiotic microorganism. In another embodiment a prebiotic composition comprises FOS, GOS, or other appropriate polysaccharide, and optionally one or more of lactose, at least one probiotic microorganism, or a buffer. Additional ingredients include ingredients to improve handling, preservatives, antioxidants, flavorings and the like.

In an embodiment, a prebiotic composition comprises FOS, GOS, or other appropriate polysaccharide, or a probiotic. In other embodiment, a prebiotic composition is in the form of a powder, tablet, capsule, or liquid. In an embodiment, a prebiotic composition can be administered with a dairy product and is in the form of milk or other common dairy product such as a yogurt, shake, smoothie, cheese, and the like.

In embodiments where a prebiotic composition comprises less than 100% by weight of FOS, GOS, or other appropriate polysaccharide, the remaining ingredients can be any suitable ingredients intended for the consumption of the subject in need thereof, e.g., human, including, but not limited to, other prebiotics (e.g., FOS), a buffer, one or more digestible saccharides (e.g. lactose, glucose, or galactose), ingredients intended to inhibit clumping and increase pourability, such as silicone dioxide and microcrystalline cellulose, or similar ingredients as are well-additional in the art. Remaining ingredients can also include ingredients to improve handling, preservatives, antioxidants, flavorings, and the like.

### B. Buffer Components

One or more buffers, optionally with a calcium counter ion, can also be administered in methods and compositions described herein. Any buffer suitable for consumption by the subject being treated, e.g., human, are useful for the compositions herein. The buffer can partially or wholly neutralize stomach acidity, which can, e.g., allow live bacteria or fungi to reach the gut. Buffers include citrates, phosphates, and the like. One embodiment utilizes a buffer with a calcium counter ion, such as Calcium Phosphate Tribasic. The calcium can serve to restore the calcium that many lactose intolerant subjects are missing in their diet. Calcium phosphate can protect *Lactobacillus acidophilus* from bile.

In an embodiment, a buffer such as calcium phosphate is given prior to beginning treatment with a prebiotic composition (such as a composition comprising or consisting essentially of FOS, GOS, or other appropriate polysaccharide), optionally in conjunction with administration of bacteria or fungi. In an embodiment, a buffer such as calcium phosphate is given in conjunction with treatment with a prebiotic composition (e.g., a composition comprising or consisting essentially of FOS, GOS, or other appropriate polysaccharide), for part or all of the treatment with lactose. Thus, in an embodiment, some or all doses of a prebiotic composition are accompanied by a dose of a buffer such as calcium phosphate. In an embodiment, a buffer such as calcium phosphate is given initially with a prebiotic composition (such as a composition comprising or consisting essentially of FOS, GOS, or other appropriate polysaccharide), but then the buffer use is discontinued. For example, the initial one, two, three, four, five, six, seven, eight, nine, ten, or more than ten days of treatment with a prebiotic composition can include doses of a buffer such as calcium phosphate, with the use of the buffer discontinued after that time. In an embodiment, a buffer such as calcium phosphate can be given for the first two days of treatment, and then the administration of buffer is discontinued. In an embodiment, a buffer such as calcium phosphate, either alone or in combination with other substances or treatments is used after the treatment with a prebiotic composition is terminated. A buffer such as calcium phosphate can be taken for any suitable period after the termination of treatment with lactose, and can be taken daily or at regular or irregular intervals. Doses can be as described below.

Numerous buffers suitable for human consumption are additional in the art, and any suitable buffer can be used in the methods and compositions described herein. Calcium triphosphate is an exemplary buffer, and its counterion supplies a nutrient that is often lacking in lactose-intolerant subjects, i.e., calcium. In an embodiment a buffer can be used in a dose from about 2 mg to about 2000 mg, or about 4 mg to about 400 mg, or about 4 mg to about 200 mg, or about 4 mg to about 100 mg, or about 8 mg to about 50 mg, or about 10 mg to about 40 mg, or about 20 mg to about 30 mg, or about 20, 21, 22, 23, 24, 25, 26, 27, 28, 29, or 30 mg. In another embodiment a prebiotic composition further comprises an amount of a buffer from 1-50 mg, such as about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 mg. In an embodiment, buffer is used in a dose of about 25 mg. In an embodiment, calcium phosphate is used in a dose of about 25 mg. The dose can be given in combination with a prebiotic composition (e.g., a composition comprising or consisting essentially of FOS, GOS, or other appropriate polysaccharide). In an embodiment, as a prebiotic composition dose increases, the dose of buffer increases as well. For example, an initial dose of a prebiotic composition can be about 0.6 g to 1.0 g, e.g., 0.8 g, given in combination with about 20-30 mg, e.g., about 25 mg, of buffer, e.g., calcium phosphate. The dose of a prebiotic composition can be increased incrementally by about 0.6 g to 1.0 g, e.g., 0.8 g, and the accompanying dose of buffer, e.g., calcium phosphate, can be increased by about 20-30 mg, e.g., about 25 mg, of buffer, e.g., calcium phosphate.

### C. Compositions Comprising GOS and at Least One Probiotic Microorganism

In an embodiment, a prebiotic composition comprises FOS, GOS, or other appropriate polysaccharide, and at least one probiotic microorganism. The FOS, GOS, or other appropriate polysaccharide can comprise more than 1% of the weight of the composition while the at least one probiotic microorganism will typically comprise less than about 10%, 5%, 4%, 3%, or 2% by weight of the compositions. For example, the FOS, GOS, or other appropriate polysaccharide can be present at about 1-99.75% by weight and the at least one probiotic microorganism at about 0.25-2% by weight, or the FOS, GOS, or other appropriate polysaccharide can be present at about 89-96% by weight and the bacteria or fungi at about 1.2-3.7% by weight. In an embodiment, FOS, GOS, or other appropriate polysaccharide are present at about 92% by weight and at least one probiotic microorganism, (e.g., *L. mesenteroides, P. pentosaceus,* or other members from Table 4 and Table 7), is present at about 1.5% by weight. In an embodiment, FOS, GOS, or other appropriate polysaccharide are present at about 92% by weight and at least one probiotic microorganism, (e.g., *L. mesenteroides, P. pentosaceus,* or other members from Table 4), is present at about 1.5% by weight. In another embodiment, FOS, GOS, or other appropriate polysaccharide are present at about 93% by weight and at least one probiotic microorganism, (e.g., *L. mesenteroides, P. pentosaceus,* or other members from Table 4 or Table 7), is present at about 1.5% by weight. In another embodiment, FOS, GOS, or other appropriate polysaccharide are present at about 94% by weight and at least one probiotic microorganism, (e.g., *L. mesenteroides, P. pentosaceus,* or other members from Table 4 or Table 7), is present at about 1.5% by weight. In another embodiment, FOS, GOS, or other appropriate polysaccharide are present at about 95% by weight and at least one probiotic microorganism, (e.g., *L. mesenteroides, P. pentosaceus,* or other members from Table 4 or Table 7), is present at about 1.5% by weight. In another embodiment, FOS, GOS, or other appropriate polysaccharide are present at about 96% by weight and at least one probiotic microorganism, (e.g., *L. mesenteroides, P. pentosaceus,* or other members from Table 4 or Table 7), is present at about 1.5% by weight. In another embodiment, FOS, GOS, or other appropriate polysaccharide are present at about 97% by weight and at least one probiotic microorganism, (e.g., *L. mesenteroides, P. pentosaceus,* or other members from Table 4 or Table 7), is present at about 1.5% by weight. In another embodiment, FOS, GOS, or other appropriate polysaccharide are present at about 98% by weight and at least one probiotic microorganism, (e.g., *L. mesenteroides, P. pentosaceus,* or other members from Table 4 or Table 7), is present at about 1.5% by weight. In another embodiment, FOS, GOS, or other appropriate polysaccharide are present at about 98.5% by weight and at least one probiotic microorganism, (e.g., *L. mesenteroides, P. pentosaceus,* or other members from Table 4 or Table 7), is present at about 1.5% by weight. If the at least one probiotic microorganism and FOS, GOS, or other appropriate polysaccharide do not make up 100% by weight of the prebiotic composition, the remaining ingredients can be any suitable ingredients intended for consumption by the subject in need thereof, e.g., human, including, but not limited to, other prebiotics (e.g., FOS), one or more buffers, digestible saccharides (e.g. lactose, glucose, or galactose), ingredients intended to inhibit clumping and increase pourability, such as silicone dioxide and microcrystalline cellulose, or similar ingredients as are well-additional in the art. Remaining ingredients can also include ingredients to improve handling, preservatives, antioxidants, flavorings and the like.

### D. Compositions Comprising FOS, GOS, or other appropriate polysaccharide and a Buffer

In another embodiment, a prebiotic composition comprises FOS, GOS, or other appropriate polysaccharide and a buffer (e.g., calcium phosphate tribasic). For example, FOS, GOS, or other appropriate polysaccharide can be present at about 1-100% by weight and the buffer at about 0.50-4% by weight, or FOS, GOS, or other appropriate polysaccharide can be present at about 1-96% by weight and the buffer at about 1 to about 3.75% by weight. In an embodiment, FOS, GOS, or other appropriate polysaccharide are present at about 1% by weight and buffer is present at about 3% by weight. In an embodiment, FOS, GOS, or other appropriate polysaccharide are present at about 5% by weight and buffer is present at about 3% by weight. In an embodiment, FOS, GOS, or other appropriate polysaccharide are present at about 10% by weight and buffer is present at about 3% by weight. In an embodiment, FOS, GOS, or other appropriate polysaccharide are present at about 15% by weight and buffer is present at about 15% by weight. In an embodiment, FOS, GOS, or other appropriate polysaccharide are present at about 20% by weight and buffer is present at about 3% by weight. In an embodiment, FOS, GOS, or other appropriate polysaccharide are present at about 25% by weight and buffer is present at about 3% by weight. In an embodiment, FOS, GOS, or other appropriate polysaccharide are present at about 30% by weight and buffer is present at about 3% by weight. In an embodiment, FOS, GOS, or other appropriate polysaccharide are present at about 35% by weight and buffer is present at about 3% by weight. In an embodiment, FOS, GOS, or other appropriate polysaccharide are present at about 40% by weight and buffer is present at about 3% by weight. In an embodiment, FOS, GOS, or other appropriate polysaccharide are present at about 50% by weight and buffer is present at about 3% by weight. In an embodiment, FOS, GOS, or other appropriate polysaccharide are present at about 60% by weight and buffer is present at about 3% by weight. In an embodiment, FOS, GOS, or other appropriate polysaccharide are present at about 70% by weight and buffer is present at about 3% by weight. In another embodiment, FOS, GOS, or other appropriate polysaccharide are present at about 90% by weight and buffer is present at about 3% by weight. In another embodiment, FOS, GOS, or other appropriate polysaccharide are present at about 92% by weight and buffer is present at about 3% by weight. In another embodiment, FOS, GOS, or other appropriate polysaccharide are present at about 93% by weight and buffer is present at about 3% by weight. In another embodiment, FOS, GOS, or other appropriate polysaccharide are present at about 94% by weight and buffer is present at about 3% by weight. In another embodiment, FOS, GOS, or other appropriate polysaccharide are present at about 95% by weight and buffer is present at about 3% by weight. In another embodiment, FOS, GOS, or other appropriate polysaccharide are present at about 96% by weight and buffer is present at about 3% by weight. In another embodiment, FOS, GOS, or other appropriate polysaccharide are present at about 97% by weight and buffer is present at about 2% by weight. In another embodiment, FOS, GOS, or other appropriate polysaccharide are present at about 98% by weight and buffer is present at about 1% by weight. In another embodiment, FOS, GOS, or other appropriate polysaccharide are present at about 99% by weight and buffer is present at about 1% by weight. In another embodiment, FOS, GOS, or other appropriate polysaccharide are present at about 100% by weight and buffer is present at less than about 1% by weight. If the buffer and FOS, GOS, or other appropriate polysaccharide do not make up 100% by weight of the composition, the remaining ingredients can be any suitable ingredients intended for consumption by the subject (e.g., a human) including, but not limited to, probiotic microorganisms (e.g., beneficial bacteria) or other prebiotics (e.g., FOS), but also including ingredients intended to inhibit clumping and increase pourability, such as silicone dioxide and microcrystalline cellulose, or similar ingredients as are well-additional in the art. Remaining ingredients can also include ingredients to improve handling, preservatives, antioxidants, flavorings and the like.

### E. Compositions Comprising a Digestible Saccharide, a Probiotic Microorganism, and FOS, GOS, or other appropriate polysaccharide

In an embodiment, a prebiotic composition comprises a digestible saccharide (e.g. lactose, glucose, or galactose), a probiotic microorganism (e.g., *L. mesenteroides, P. pentosaceus,* or other members from Table 4 or Table 7), and FOS, GOS, or other appropriate polysaccharide. In an embodiment, lactose can be present at about 1-20% by weight, bacteria or fungi at about 0.25-20.10% by weight, and FOS, GOS, or other appropriate polysaccharide at about 1-98.75% by weight. In another embodiment lactose can be present at about 5-20% by weight, bacteria or fungi at about 0.91-1.95% by weight, and FOS, GOS, or other appropriate polysaccharide at about 1 to about 96% by weight. In another embodiment, lactose is present at about 20% by weight, bacteria or fungi at about 1.5% by weight, and FOS, GOS, or other appropriate polysaccharide are present at about 1% by weight. In another embodiment, lactose is present at about 20% by weight, bacteria or fungi at about 1.5% by weight, and FOS, GOS, or other appropriate polysaccharide are present at about 50% by weight. In another embodiment, lactose is present at about 20% by weight, bacteria or fungi at about 1.5% by weight, and FOS, GOS, or other appropriate polysaccharide are present at about 60% by weight. In another embodiment, lactose is present at about 20% by weight, bacteria or fungi at about 1.5% by weight, and FOS, GOS, or other appropriate polysaccharide are present at about 70% by weight. In another embodiment, lactose is present at about 5% by weight, bacteria or fungi at about 1.5% by weight, and FOS, GOS, or other appropriate polysaccharide are present at about 90% by weight. In another embodiment, lactose is present at about 5% by weight, bacteria or fungi at about 1.5% by weight, and FOS, GOS, or other appropriate polysaccharide are present at about 92% by weight. In another embodiment, lactose is present at about 5% by weight, bacteria or fungi at about 1.5% by weight, and FOS, GOS, or other appropriate polysaccharide are present at about 93% by weight. In another embodiment, lactose is present at about 5% by weight, bacteria or fungi at about 1% by weight, and FOS, GOS, or other appropriate polysaccharide are present at about 94% by weight. In another embodiment, lactose is present at about 4.5% by weight, bacteria or fungi at about 1.5% by weight, and FOS, GOS, or other appropriate polysaccharide are present at about 94% by weight. In another embodiment, lactose is present at about 4.5% by weight, bacteria or fungi at about 0.5% by weight, and FOS, GOS, or other appropriate polysaccharide are present at about 95% by weight. In another embodiment, lactose is present at about 3.5% by weight, bacteria or fungi at about 0.5% by weight, and FOS, GOS, or other appropriate polysaccharide are present at about 96% by weight. In another embodiment, lactose is present at about 2.5% by weight, bacteria or fungi at about 0.5% by weight, and FOS, GOS, or other appropriate polysaccharides are present at about 97% by weight. In another embodiment, lactose is present at about 1.5% by weight, bacteria or fungi at about 0.5% by weight, and FOS, GOS, or other appropriate polysaccharide are present at about 98% by weight. In another embodiment, lactose is present at about 0.5% by weight, bacteria or fungi at about 0.5% by weight, and FOS, GOS, or other appropriate polysaccharide are present at about 99% by weight. If the bacteria or fungi, FOS, GOS, or other appropriate polysaccharide and lactose do not make up 100% of the composition, the remaining ingredients can be any suitable ingredients intended for consumption by the subject, e.g., a human, including, but not limited to a buffer, digestible saccharides (e.g., lactose, glucose, or galactose), ingredients intended to inhibit clumping and increase pourability, such as silicone dioxide and microcrystalline cellulose, or similar ingredients as are well-additional in the art. Remaining ingredients can also include ingredients to improve handling, preservatives, antioxidants, flavorings and the like.

### F. Compositions Comprising FOS, GOS, or other appropriate polysaccharide, a Probiotic Microorganism, and Buffer

In an embodiment, a prebiotic composition comprises FOS, GOS, or other appropriate polysaccharide, a probiotic microorganism, and buffer. In an embodiment, FOS, GOS, or other appropriate polysaccharide can be present at about 1-100% by weight, a probiotic microorganism at about 0.25-2% by weight, and the buffer at about 0.50-4% by weight. In another embodiment, FOS, GOS, or other appropriate polysaccharide can be present at about 1-95% by weight, a probiotic microorganism at about 0.91-1.95% by weight, and the buffer at about 1.2-30.75% by weight. In another embodiment, FOS, GOS, or other appropriate polysaccharide are present at about 1% by weight, a probiotic microorganism at about 1.5% by weight, and buffer is present at about 3% by weight. In another embodiment, FOS, GOS, or other appropriate polysaccharide are present at about 5% by weight, a probiotic microorganism at about 1.5% by weight, and buffer is present at about 3% by weight. In another embodiment, FOS, GOS, or other appropriate polysaccharide are present at about 10% by weight, a probiotic microorganism at about 1.5% by weight, and buffer is present at about 3% by weight. In another embodiment, FOS, GOS, or other appropriate polysaccharide are present at about 15% by weight, a probiotic microorganism at about 1.5% by weight, and buffer is present at about 3% by weight. In another embodiment, FOS, GOS, or other appropriate polysaccharide are present at about 20% by weight, a probiotic microorganism at about 1.5% by weight, and buffer is present at about 3% by weight. In another embodiment, FOS, GOS, or other appropriate polysaccharide are present at about 25% by weight, a probiotic microorganism at about 1.5% by weight, and buffer is present at about 3% by weight. In another embodiment, FOS, GOS, or other appropriate polysaccharide are present at about 30% by weight, a probiotic microorganism at about 1.5% by weight, and buffer is present at about 3% by weight. In another embodiment, FOS, GOS, or other appropriate polysaccharide are present at about 35% by weight, a probiotic microorganism at about 1.5% by weight, and buffer is present at about 3% by weight. In another embodiment, FOS, GOS, or other appropriate polysaccharide are present at about 40% by weight, a probiotic microorganism at about 1.5% by weight, and buffer is present at about 3% by weight. In another embodiment, FOS, GOS, or other appropriate polysaccharide are present at about 50% by weight, a probiotic microorganism at about 1.5% by weight, and buffer is present at about 3% by weight. In another embodiment, FOS, GOS, or other appropriate polysaccharide are present at about 60% by weight, a probiotic microorganism at about 1.5% by weight, and buffer is present at about 3% by weight. In another embodiment, FOS, GOS, or other appropriate polysaccharide are present at about 70% by weight, a probiotic microorganism at about 1.5% by weight, and buffer is present at about 3% by weight. In another embodiment, FOS, GOS, or other appropriate polysaccharide are present at about 90% by weight, a probiotic microorganism at about 1.5% by weight, and buffer is present at about 3% by weight. In another embodiment, FOS, GOS, or other appropriate polysaccharide are present at about 92% by weight, a probiotic microorganism at about 1.5% by weight, and buffer is present at about 3% by weight. In another embodiment, FOS, GOS, or other appropriate polysaccharide are present at about 93% by weight, a probiotic microorganism at about 1.5% by weight, and buffer is present at about 3% by weight. In another embodiment, FOS, GOS, or other appropriate polysaccharide are present at about 94% by weight, a probiotic microorganism at about 1.5% by weight, and buffer is present at about 3% by weight. In another embodiment, FOS, GOS, or other appropriate polysaccharide are present at about 95% by weight, a probiotic microorganism at about 1.5% by weight, and buffer is present at about 3% by weight. In another embodiment, FOS, GOS, or other appropriate polysaccharide are present at about 96% by weight, a probiotic microorganism at about 1.5% by weight, and buffer is present at about 2% by weight. In another embodiment, FOS, GOS, or other appropriate polysaccharide are present at about 97% by weight, a probiotic microorganism at about 1.5% by weight, and buffer is present at about 1.5% by weight. In another embodiment, FOS, GOS, or other appropriate polysaccharide are present at about 99% by weight, a probiotic microorganism at about 0.5% by weight, and buffer is present at about 0.5% by weight. In another embodiment, FOS, GOS, or other appropriate polysaccharide are present at about 100% by weight, a probiotic microorganism at less than about 0.5% by weight, and buffer is present at less than about 0.5% by weight. If the probiotic microorganism, buffer, and FOS, GOS, or other appropriate polysaccharide do not make up 100% of the composition, the remaining ingredients can be any suitable ingredients intended for the consumption of a subject (e.g., human) including, but not limited to, other prebiotics (e.g., FOS), digestible saccharides (e.g., lactose, glucose or galactose), ingredients intended to inhibit clumping and increase pourability, such as silicone dioxide and microcrystalline cellulose, or similar ingredients as are well-additional in the art. Remaining ingredients can also include ingredients to improve handling, preservatives, antioxidants, flavorings and the like.

### G. Compositions Comprising a Digestible Saccharide, FOS, GOS, or other appropriate polysaccharide, and a Buffer

In an embodiment, a prebiotic composition comprises a digestible saccharide (e.g. lactose, glucose, or galactose), FOS, GOS, or other appropriate polysaccharide, and a buffer. For example, lactose can be present at about 1-20% by weight, FOS, GOS, or other appropriate polysaccharide at about 1-100% by weight, and the buffer at about 0.50-4% by weight, or the lactose can be present at about 5-20% by weight, FOS, GOS, or other appropriate polysaccharide at about 1-96% by weight, and the buffer at about 1.2-30.75% by weight. In an embodiment, lactose is present at about 20% by weight, FOS, GOS, or other appropriate polysaccharide at about 1% by weight, and buffer is present at about 3% by weight. In an embodiment, lactose is present at about 5% by weight, FOS, GOS, or other appropriate polysaccharide at about 1% by weight, and buffer is present at about 3% by weight. In an embodiment, lactose is present at about 20% by weight, FOS, GOS, or other appropriate polysaccharide at about 10% by weight, and buffer is present at about 3% by weight. In an embodiment, lactose is present at about 20% by weight, FOS, GOS, or other appropriate polysaccharide at about 15% by weight, and buffer is present at about 3% by weight. In an embodiment, lactose is present at about 20% by weight, FOS, GOS, or other appropriate polysaccharide at about 20% by weight, and buffer is present at about 3% by weight. In an embodiment, lactose is present at about 20% by weight, FOS, GOS, or other appropriate polysaccharide at about 25% by weight, and buffer is present at about 3% by weight. In an embodiment, lactose is present at about 20% by weight, FOS, GOS, or other appropriate polysaccharide at about 30% by weight, and buffer is present at about 3% by weight. In an embodiment, lactose is present at about 20% by weight, FOS, GOS, or other appropriate polysaccharide at about 35% by weight, and buffer is present at about 3% by weight. In an embodiment, lactose is present at about 20% by weight, FOS, GOS, or other appropriate polysaccharide at about 40% by weight, and buffer is present at about 3% by weight. In an embodiment, lactose is present at about 20% by weight, FOS, GOS, or other appropriate polysaccharide at about 50% by weight, and buffer is present at about 3% by weight. In an embodiment, lactose is present at about 20% by weight, FOS, GOS, or other appropriate polysaccharide at about 60% by weight, and buffer is present at about 3% by weight. In an embodiment, lactose is present at about 20% by weight, FOS, GOS, or other appropriate polysaccharide at about 70% by weight, and buffer is present at about 3% by weight. In another embodiment, lactose is present at about 5% by weight, FOS, GOS, or other appropriate polysaccharide at about 90% by weight, and buffer is present at about 3% by weight. In another embodiment, lactose is present at about 5% by weight, FOS, GOS, or other appropriate polysaccharide at about 92% by weight, and buffer is present at about 3% by weight. In another embodiment, lactose is present at about 4% by weight, FOS, GOS, or other appropriate polysaccharide at about 93% by weight, and buffer is present at about 3% by weight. In another embodiment, lactose is present at about 3% by weight, FOS, GOS, or other appropriate polysaccharide at about 94% by weight, and buffer is present at about 3% by weight. In another embodiment, lactose is present at about 2% by weight, FOS, GOS, or other appropriate polysaccharide at about 95% by weight, and buffer is present at about 3% by weight. In another embodiment, lactose is present at about 1% by weight, FOS, GOS, or other appropriate polysaccharide at about 96% by weight, and buffer is present at about 3% by weight. If a suitable prebiotic, buffer and lactose do not make up 100% of the composition by weight, the remaining ingredients can be any suitable ingredients intended for consumption by a subject (e.g., human) including, but not limited to, bacteria or fungi, ingredients intended to inhibit clumping and increase pourability, such as silicone dioxide and microcrystalline cellulose, or similar ingredients as are well-additional in the art. Remaining ingredients can also include ingredients to improve handling, preservatives, antioxidants, flavorings and the like.

### H. Compositions Comprising a Digestible Saccharide, Bacteria, Fungi, GOS, and a Buffer

In an embodiment, a composition comprises a digestible saccharide (e.g. lactose, glucose, or galactose), bacteria or fungi, FOS, GOS, or other appropriate polysaccharide, and buffer. For example, lactose can be present at about 1-20% by weight, bacteria or fungi at about 0.25-2.10% by weight, FOS, GOS, or other appropriate polysaccharide at about 1-100% by weight, and the buffer at about 0.50-4% by weight, or the lactose can be present at about 5-20% by weight, bacteria or fungi at about 0.91-1.95% by weight, FOS, GOS, or other appropriate polysaccharide at about 70-95% by weight, and the buffer at about 1.2-30.75% by weight. In an embodiment, lactose is present at about 20% by weight, bacteria or fungi at about 1.47% by weight, FOS, GOS, or other appropriate polysaccharide at about 1% by weight, and buffer is present at about 3% by weight. In an embodiment, lactose is present at about 20% by weight, bacteria or fungi at about 1.47% by weight, FOS, GOS, or other appropriate polysaccharide at about 10% by weight, and buffer is present at about 3% by weight. In an embodiment, lactose is present at about 20% by weight, bacteria or fungi at about 1.47% by weight, FOS, GOS, or other appropriate polysaccharide at about 15% by weight, and buffer is present at about 3% by weight. In an embodiment, lactose is present at about 20% by weight, bacteria or fungi at about 1.47% by weight, FOS, GOS, or other appropriate polysaccharide at about 20% by weight, and buffer is present at about 3% by weight. In an embodiment, lactose is present at about 20% by weight, bacteria or fungi at about 1.47% by weight, FOS, GOS, or other appropriate polysaccharide at about 25% by weight, and buffer is present at about 3% by weight. In an embodiment, lactose is present at about 20% by weight, bacteria or fungi at about 1.47% by weight, FOS, GOS, or other appropriate polysaccharide at about 30% by weight, and buffer is present at about 3% by weight. In an embodiment, lactose is present at about 20% by weight, bacteria or fungi at about 1.47% by weight, FOS, GOS, or other appropriate polysaccharide at about 35% by weight, and buffer is present at about 3% by weight. In an embodiment, lactose is present at about 20% by weight, bacteria or fungi at about 1.47% by weight, FOS, GOS, or other appropriate polysaccharide at about 40% by weight, and buffer is present at about 3% by weight. In an embodiment, lactose is present at about 20% by weight, bacteria or fungi at about 1.47% by weight, FOS, GOS, or other appropriate polysaccharide at about 50% by weight, and buffer is present at about 3% by weight. In an embodiment, lactose is present at about 20% by weight, bacteria or fungi at about 1.47% by weight, FOS, GOS, or other appropriate polysaccharide at about 60% by weight, and buffer is present at about 3% by weight. In an embodiment, lactose is present at about 20% by weight, bacteria or fungi at about 1.47% by weight, FOS, GOS, or other appropriate polysaccharide at about 70% by weight, and buffer is present at about 3% by weight. In an embodiment, lactose is present at about 5% by weight, bacteria or fungi at about 1.47% by weight, FOS, GOS, or other appropriate polysaccharide at about 90% by weight, and buffer is present at about 3% by weight. In an embodiment, lactose is present at about 3% by weight, bacteria or fungi at about 1.47% by weight, FOS, GOS, or other appropriate polysaccharide at about 92% by weight, and buffer is present at about 3% by weight. In an embodiment, lactose is present at about 2% by weight, bacteria or fungi at about 1.47% by weight, FOS, GOS, or other appropriate polysaccharide at about 93% by weight, and buffer is present at about 3% by weight. In an embodiment, lactose is present at about 1% by weight, bacteria or fungi at about 1.47% by weight, FOS, GOS, or other appropriate polysaccharide at about 94% by weight, and buffer is present at about 3% by weight. In an embodiment, lactose is present at about 0.5% by weight, bacteria or fungi at about 1.47% by weight, FOS, GOS, or other appropriate polysaccharide at about 95% by weight, and buffer is present at about 3% by weight. If the bacteria, fungi, FOS, GOS, or other, buffer and lactose do not make up 100% of the composition by weight, the remaining ingredients can be any suitable ingredients intended for consumption by a subject, e.g., human, including, but not limited to, ingredients intended to inhibit clumping and increase pourability, such as silicone dioxide and microcrystalline cellulose, or similar ingredients as are well-additional in the art. Remaining ingredients can also include ingredients to improve handling, preservatives, antioxidants, flavorings and the like.

### I. Additional Ingredients

Additional ingredients include ingredients to improve handling, preservatives, antioxidants, flavorings and the like. For example, in an embodiment, a prebiotic composition in powdered form can include flavorings such that when mixed in a liquid (e.g., water), the powder can flavor the liquid with various flavors such as grape, strawberry, lime, lemon, chocolate, and the like. In an embodiment, the compositions include microcrystalline cellulose or silicone dioxide. Preservatives can include, for example, benzoic acid, alcohols, for example, ethyl alcohol, and hydroxybenzoates. Antioxidants can include, for example, butylated hydroxyanisole (BHA), butylated hydroxytoluene (BHT), tocopherols (e.g., Vitamin E), and ascorbic acid (Vitamin C).

### Dosage Forms

### A. General

Compositions described herein include any suitable form, including liquid or powder. Powdered compositions can be as pure powder, or can be in the form of capsules, tablets, or the like. Powder can be packaged in bulk (e.g., in a container containing sufficient prebiotic or other substances for a subject to follow for an entire course of treatment with increasing doses of prebiotic, or a portion of a course of treatment), or as individual packets (e.g., packets containing a single dose of prebiotic plus other components, or packets containing the dose of prebiotic and other components needed for a particular day of a prebiotic treatment regimen). If packaged in bulk, the powder can be in any suitable container, such as a packet, sachet, canister, ampoule, ramekin, or bottle. The container can also include one or more scoops or similar serving devices of a size or sizes appropriate to measure and serve one or more doses of prebiotic and, optionally, other ingredients included in the powder. Liquid compositions contain prebiotic and, optionally, other ingredients, in a suitable liquid, e.g., water or buffer. Liquid compositions can be provided in bulk (e.g., in a container containing sufficient prebiotic or other substances for one subject in need thereof to follow an entire course of treatment with increasing doses of prebiotic, or a portion of a course of treatment), or as individual containers, such as cans, bottles, soft packs, and the like (e.g., containers containing a single dose of prebiotic plus other components in suitable liquid, or containers containing the dose of prebiotic and other components needed for a particular day of a prebiotic treatment regimen). The container can also include one or more measuring cups or similar serving devices of a size or sizes appropriate to measure and serve one or more doses of prebiotic and, optionally, other ingredients included in the liquid.

In an embodiment, compositions described herein comprise one or more excipients. In an embodiment, the one or more excipients comprise one or more antiadherents, one or more binders, one or more coatings, one or more disintegrants, one or more fillers, one or more flavors, one or more colors, one or more lubricants, one or more glidants, one or more sorbents, one or more preservatives, one or more sweeteners, or a combination thereof. In an embodiment, the antiadherent is magnesium stearate. In an embodiment, the one or more binders are cellulose, microcrystalline cellulose, hydroxypropyl cellulose, xylitol, sorbitol, maltitiol, gelatin, polyvinylpyrrolidone, polyethylene glycol, methyl cellulose, hydroxypropyl methylcellulose, or a combination thereof. In an embodiment, the one or more coatings are a hydroxypropyl methylcellulose film, shellac, corn protein zein, gelatin, methyl acrylate-methacrylic acid copolymers, cellulose acetate succinate, hydroxy propyl methyl cellulose phthalate, hydroxy propyl methyl cellulose acetate succinate, polyvinyl acetate phthalate, methyl methacrylate-methacrylic acid copolymers, sodium alginate, stearic acid, or a combination thereof. In an embodiment, the one or more disintegrants are crosslinked polyvinylpyrrolidone (crospovidone), crosslinked sodium carboxymethyl cellulose (croscarmellose sodium), sodium starch glycolate, or a combination thereof. In an embodiment, the one or more fillers are calcium carbonate, magnesium stearate, dibasic calcium phosphate, cellulose, vegetable oil, vegetable fat, or a combination thereof. In an embodiment, the one or more flavors are mint, cherry, anise, peach, apricot, licorice, raspberry, vanilla, or a combination thereof. In an embodiment, the one or more lubricants are talc, silica, vegetable stearin, magnesium stearate, stearic acid, or a combination thereof. In an embodiment, the one or more glidants are fumed silica, talc, magnesium carbonate, or a combination thereof. In an embodiment, the one or more sorbents are fatty acids, waxes, shellac, plastics, plant fibers, or a combination thereof. In an embodiment, the one or more preservatives are vitamin A, vitamin E, vitamin C, retinyl palmitate, selenium, cysteine, methionine, citric acid, sodium citrate, methyl paraben, propyl paraben, or a combination thereof. In an embodiment, the one or more sweeteners are stevia, sparame, sucralose, neotame, acesulfame potassium, saccharin or a combination thereof.

### B. Oral Dosage Forms and Components

In one aspect provided herein are methods and compositions formulated for oral delivery to a subject in need thereof. In an embodiment a composition is formulated to deliver a composition comprising a prebiotic to a subject in need thereof. In another embodiment, a pharmaceutical composition is formulated to deliver a composition comprising a prebiotic to a subject in need thereof. In another embodiment a composition is formulated to deliver a composition comprising prebiotic and a probiotic microorganism to a subject in need thereof.

### 1. Forms

In an embodiment, a composition is administered in solid, semi-solid, microemulsion, gel, or liquid form. Examples of such dosage forms include tablet forms disclosed in U.S. Pat. Nos. 3,048,526, 3,108,046, 4,786,505, 4,919,939, and 4,950,484; gel forms disclosed in U.S. Pat. Nos. 4,904,479, 6,482,435, 6,572,871, and 5,013,726; capsule forms disclosed in U.S. Pat. Nos. 4,800,083, 4,532,126, 4,935,243, and 6,258,380; or liquid forms disclosed in U.S. Pat. Nos. 4,625,494, 4,478,822, and 5,610,184; each of which is incorporated herein by reference in its entirety.

Forms of the compositions that can be used orally include tablets, push-fit capsules made of gelatin, as well as soft, sealed capsules made of gelatin and a plasticizer, such as glycerol or sorbitol. Tablets can be made by compression or molding, optionally with one or more accessory ingredients including freeze-dried plant material serving both as prebiotic and as a filler. Compressed tablets can be prepared by compressing in a suitable machine the active ingredient in a free-flowing form such as a powder or granules, optionally mixed with binders (e.g., povidone, gelatin, hydroxypropylmethyl cellulose), inert diluents, preservative, antioxidant, disintegrant (e.g., sodium starch glycolate, cross-linked povidone, cross-linked sodium carboxymethyl cellulose) or lubricating, surface active or dispersing agents. Molded tablets can be made by molding in a suitable machine a mixture of the powdered compound moistened with an inert liquid diluent. The tablets can optionally be coated or scored and can be formulated so as to provide slow or controlled release of the active ingredient therein. Tablets can optionally be provided with an enteric coating, to provide release in parts of the gut (e.g., colon, lower intestine) other than the stomach. All formulations for oral administration can be in dosages suitable for such administration. The push-fit capsules can contain the active ingredients in admixture with filler such as lactose, binders such as starches, and/or lubricants such as talc or magnesium stearate and, optionally, stabilizers. In soft capsules, the active compounds (prebiotics or probiotics) can be dissolved or suspended in suitable liquids, such as fatty oils, liquid paraffin, or liquid polyethylene glycols. In addition, stabilizers can be added. Dragee cores are provided with suitable coatings. For this purpose, concentrated sugar solutions can be used, which can optionally contain gum arabic, talc, polyvinyl pyrrolidone, carbopol gel, polyethylene glycol, or titanium dioxide, lacquer solutions, and suitable organic solvents or solvent mixtures. Dyestuffs or pigments can be added to the tablets or Dragee coatings for identification or to characterize different combinations of active compound doses.

Formulations for oral use can also be presented as hard gelatin capsules wherein the active ingredient is mixed with an inert solid diluent, for example, calcium carbonate, calcium phosphate or kaolin, or as soft gelatin capsules wherein the active ingredient is mixed with water soluble carrier such as polyethylene glycol or an oil medium, for example peanut oil, liquid paraffin, or olive oil.

Oral liquid preparations can be in the form of, for example, aqueous or oily suspensions, solutions, emulsions syrups or elixirs, or can be presented as a dry product for reconstitution with water or other suitable vehicle before use. Such liquid preparations can contain conventional additives, such as suspending agents, for example sorbitol, methyl cellulose, glucose syrup, gelatin, hydroxyethyl cellulose, carboxymethyl cellulose, aluminum stearate gel, fruit dry extracts, plant extracts, or hydrogenated edible fats, emulsifying agents, for example lecithin, sorbitan monooleate, acacia; nonaqueous vehicles (which can include edible oils), for example almond oil, oily esters such as glycerine, propylene glycol, or ethyl alcohol; preservatives, for example methyl or propyl p-hydoxybenzoate or sorbic acid, and, if desired, conventional flavoring or coloring agents.

In an embodiment, a provided composition includes a softgel formulation. A softgel can contain a gelatin-based shell that surrounds a liquid fill. The shell can be made of gelatin, plasticiser (e.g., glycerin and/or sorbitol), modifier, water, color, antioxidant, or flavor. The shell can be made with starch or carrageenan. The outer layer can be enteric coated. In an embodiment, a softgel formulation can include a water or oil soluble fill solution, or suspension of a composition, for example, a prebiotic composition, covered by a layer of gelatin.

An enteric coating can control the location of where a prebiotic composition is absorbed in the digestive system. For example, an enteric coating can be designed such that a prebiotic composition does not dissolve in the stomach, but rather, travels to the small intestine, where it dissolves. An enteric coating can be stable at low pH (such as in the stomach) and can dissolve at higher pH (for example, in the small intestine). Material that can be used in enteric coatings includes, for example, alginic acid, cellulose acetate phthalate, plastics, waxes, shellac, and fatty acids (e.g., stearic acid, palmitic acid). Enteric coatings are described, for example, in U.S. Pat. Nos. 5,225,202, 5,733,575, 6,139,875, 6,420,473, 6,455,052, and 6,569,457, all of which are herein incorporated by reference in their entirety. The enteric coating can be an aqueous enteric coating. Examples of polymers that can be used in enteric coatings include, for example, shellac (trade name EmCoat 120 N, Marcoat 125); cellulose acetate phthalate (trade name aquacoat CPD^{®}, Sepifilm^{™}LP, Klucel^{®}Aquacoat^{®}ECD, and Metolose^{®}); polyvinylacetate phthalate (trade name Sureteric^{®}); and methacrylic acid (trade name Eudragit^{®}).

In an embodiment, an enteric coated prebiotic composition is administered to a subject. In another embodiment, an enteric coated probiotic composition is administered to a subject. In another embodiment, an enteric coated probiotic microorganism and prebiotic composition is administered to a subject. In an embodiment, probiotic microorganisms can be administered to a subject using an enteric coating. The stomach has an acidic environment that can kill probiotics. An enteric coating can protect probiotics as they pass through the stomach and small intestine.

Enteric coatings can be used to (1) prevent the gastric juice from reacting with or destroying the active substance, (2) prevent dilution of the active substance before it reaches the intestine, (3) ensure that the active substance is not released until after the preparation has passed the stomach, and (4) prevent live bacteria contained in the preparation from being killed because of the low pH-value in the stomach.

Enteric coatings can also be used for avoiding irritation of or damage to the mucous membrane of the stomach caused by substances contained in the oral preparation, and for counteracting or preventing formation or release of substances having an unpleasant odor or taste in the stomach. Finally, such coatings can be used for preventing nausea or vomiting on intake of oral preparations.

In an embodiment a prebiotic composition is provided as a tablet, capsule, or caplet with an enteric coating. In an embodiment the enteric coating is designed to hold the tablet, capsule, or caplet together when in the stomach. The enteric coating is designed to hold together in acid conditions of the stomach and break down in non-acid conditions and therefore release the drug in the intestines.

Softgel delivery systems can also incorporate phospholipids or polymers or natural gums to entrap a composition, for example, a prebiotic composition, in the gelatin layer with an outer coating to give desired delayed/control release effects, such as an enteric coating.

Formulations of softgel fills can be at pH 2.5-7.5.

A softgel formulation can be sealed tightly in an automatic manner. A softgel formulation can easily be swallowed, allow for product identification using colors and several shapes, allow uniformity, precision and accuracy between dosages, be safe against adulteration, provide good availability and rapid absorption, and offer protection against contamination, light and oxidation. Furthermore, softgel formulations can avoid unpleasant flavors due to content encapsulation.

A composition comprising a softgel formulation can be in any of number of different sizes, including, for example, round, oblong, oval, tube, droplet, or suppositories.

In an embodiment a composition is provided in a dosage form which comprises an effective amount of prebiotic and one or more release controlling excipients as described herein. Suitable modified release dosage vehicles include, but are not limited to, hydrophilic or hydrophobic matrix devices, water-soluble separating layer coatings, enteric coatings, osmotic devices, multi-particulate devices, and combinations thereof. In an embodiment the dosage form is a tablet, caplet, capsule or lollipop. In another embodiment, the dosage form is a liquid, oral suspension, oral solution, or oral syrup. In yet another embodiment, the dosage form is a gel capsule, soft gelatin capsule, or hard gelatin capsule.

In an embodiment, the dosage form is a gelatin capsule having a size indicated in Table 1.

**Table 1**

| Gel Cap Sizes Allowable For Human Consumption Empty Gelatin Capsule Physical Specifications | | | |
|---|---|---|---|
| Outer | Height or Actual | | |
| | Diameter Size (mm) | Locked Length (mm) | Volume (ml) |
| | 9.97 | 26.14 | 1.37 |
| | 8.53 | 23.30 | 0.95 |
| | 7.65 | 21.7 | 0.68 |
| | 6.91 | 19.4 | 0.50 |
| | 6.35 | 18.0 | 0.37 |
| | 5.82 | 15.9 | 0.3 |
| | 5.31 | 14.3 | 0.21 |
| | 4.91 | 11.1 | 0.13 |

| | | | |
|---|---|---|---|
| Note: sizes and volumes are approximate. | | | |

In another embodiment a composition comprising a prebiotic is provided in effervescent dosage forms. The compositions can also comprise non-release controlling excipients.

In another embodiment, a composition comprising a prebiotic is provided in a dosage form that has at least one component that can facilitate release of the prebiotic. In a further embodiment the dosage form can be capable of giving a discontinuous release of the compound in the form of at least two consecutive pulses separated in time from 0.1 up to 24 hours. The compositions can comprise one or more release controlling and non-release controlling excipients, such as those excipients suitable for a disruptable semi-permeable membrane and as swellable substances.

In another embodiment the prebiotic mixture is a plant or plant extract, either in solid or liquid form.

In another embodiment a composition comprising a prebiotic is provided in an enteric coated dosage form. The composition can also comprise non-release controlling excipients.

In another embodiment a composition comprising a prebiotic is provided in a dosage form for oral administration to a subject in need thereof, which comprises one or more pharmaceutically acceptable excipients or carriers, enclosed in an intermediate reactive layer comprising a gastric juice-resistant polymeric layered material partially neutralized with alkali and having cation exchange capacity and a gastric juice-resistant outer layer.

In an embodiment a composition comprising a prebiotic is provided in the form of enteric-coated granules, for oral administration. The compositions can further comprise cellulose, disodium hydrogen phosphate, hydroxypropyl cellulose, hypromellose, lactose, mannitol, and sodium lauryl sulfate.

In another embodiment a composition comprising a prebiotic is provided in the form of enteric-coated pellets, for oral administration. The compositions can further comprise glyceryl monostearate 40-50, hydroxypropyl cellulose, hypromellose, magnesium stearate, methacrylic acid copolymer type C, polysorbate 80, sugar spheres, talc, and triethyl citrate.

In an embodiment a composition comprising a prebiotic is provided in the form of enteric-coated granules, for oral administration. The compositions can further comprise carnauba wax, crospovidone, diacetylated monoglycerides, ethylcellulose, hydroxypropyl cellulose, hypromellose phthalate, magnesium stearate, mannitol, sodium hydroxide, sodium stearyl fumarate, talc, titanium dioxide, and yellow ferric oxide.

In another embodiment a composition comprising a prebiotic can further comprise calcium stearate, crospovidone, hydroxypropyl methylcellulose, iron oxide, mannitol, methacrylic acid copolymer, polysorbate 80, povidone, propylene glycol, sodium carbonate, sodium lauryl sulfate, titanium dioxide, and triethyl citrate.

The compositions provided herein can be in unit-dosage forms or multiple-dosage forms. Unit-dosage forms, as used herein, refer to physically discrete units suitable for administration to human or non-human animal subject in need thereof and packaged individually. Each unit-dose can contain a predetermined quantity of an active ingredient(s) sufficient to produce the desired therapeutic effect, in association with other pharmaceutical carriers or excipients. Examples of unit-dosage forms include, but are not limited to, ampoules, syringes, and individually packaged tablets and capsules. Unit-dosage forms can be administered in fractions or multiples thereof. A multiple-dosage form is a plurality of identical unit-dosage forms packaged in a single container, which can be administered in segregated unit-dosage form. Examples of multiple-dosage forms include, but are not limited to, vials, bottles of tablets or capsules, or bottles of pints or gallons. In another embodiment the multiple dosage forms comprise different pharmaceutically active agents. For example, a multiple dosage form can be provided which comprises a first dosage element comprising a composition comprising a prebiotic and a second dosage element comprising lactose or a probiotic, which can be in a modified release form.

In this example a pair of dosage elements can make a single unit dosage. In an embodiment a kit is provided comprising multiple unit dosages, wherein each unit comprises a first dosage element comprising a composition comprising a prebiotic and a second dosage element comprising probiotic, lactose or both, which can be in a modified release form. In another embodiment the kit further comprises a set of instructions.

In an embodiment, compositions can be formulated in various dosage forms for oral administration. The compositions can also be formulated as a modified release dosage form, including immediate-, delayed-, extended-, prolonged-, sustained-, pulsatile-, controlled-, extended, accelerated-, fast-, targeted-, programmed-release, and gastric retention dosage forms. These dosage forms can be prepared according to additional methods and techniques (see, Remington: The Science and Practice of Pharmacy, supra; Modified-Release Drug Delivery Technology, Rathbone et al., Eds., Drugs and the Pharmaceutical Science, Marcel Dekker, Inc.: New York, N.Y., 2002; Vol. 126, which is herein incorporated by reference in its entirety).

In an embodiment, the compositions are in one or more dosage forms. For example, a composition can be administered in a solid or liquid form. Examples of solid dosage forms include but are not limited to discrete units in capsules or tablets, as a powder or granule, or present in a tablet conventionally formed by compression molding. Such compressed tablets can be prepared by compressing in a suitable machine the three or more agents and a pharmaceutically acceptable carrier. The molded tablets can be optionally coated or scored, having indicia inscribed thereon and can be so formulated as to cause immediate, substantially immediate, slow, controlled or extended release of a composition comprising a prebiotic. Furthermore, dosage forms of the invention can comprise acceptable carriers or salts additional in the art, such as those described in the Handbook of Pharmaceutical Excipients, American Pharmaceutical Association (1986), incorporated by reference herein in its entirety.

In an embodiment, an effective amount of a composition comprising a prebiotic is mixed with a pharmaceutical excipient to form a solid preformulation composition comprising a homogeneous mixture of compounds described herein. When referring to these compositions as "homogeneous," it is meant that the agents are dispersed evenly throughout the composition so that the composition can be subdivided into unit dosage forms such as tablets, caplets, or capsules. This solid preformulation composition can then be subdivided into unit dosage forms of the type described above comprising from, for example, about 1 g to about 20 mg of a prebiotic composition. A prebiotic composition can be formulated, in the case of caplets, capsules or tablets, to be swallowed whole, for example with water.

The compositions described herein can be in liquid form. The liquid formulations can comprise, for example, an agent in water-in-solution and/or suspension form; and a vehicle comprising polyethoxylated castor oil, alcohol, and/or a polyoxyethylated sorbitan mono-oleate with or without flavoring. Each dosage form comprises an effective amount of an active agent and can optionally comprise pharmaceutically inert agents, such as conventional excipients, vehicles, fillers, binders, disintegrants, pH adjusting substances, buffer, solvents, solubilizing agents, sweeteners, coloring agents, and any other inactive agents that can be included in pharmaceutical dosage forms for oral administration. Examples of such vehicles and additives can be found in Remington's Pharmaceutical Sciences, 17th edition (1985).

### 2. Manufacturing

The dosage forms described herein can be manufactured using processes that are well additional to those of skill in the art. For example, for the manufacture of tablets, an effective amount of a prebiotic can be dispersed uniformly in one or more excipients, for example, using high shear granulation, low shear granulation, fluid bed granulation, or by blending for direct compression. Excipients include diluents, binders, disintegrants, dispersants, lubricants, glidants, stabilizers, surfactants and colorants. Diluents, also termed "fillers," can be used to increase the bulk of a tablet so that a practical size is provided for compression. Non-limiting examples of diluents include lactose, cellulose, microcrystalline cellulose, mannitol, dry starch, hydrolyzed starches, powdered sugar, talc, sodium chloride, silicon dioxide, titanium oxide, dicalcium phosphate dihydrate, calcium sulfate, calcium carbonate, alumina and kaolin. Binders can impart cohesive qualities to a tablet formulation and can be used to help a tablet remain intact after compression. Non-limiting examples of suitable binders include starch (including corn starch and pregelatinized starch), gelatin, sugars (e.g., glucose, dextrose, sucrose, lactose and sorbitol), celluloses, polyethylene glycol, waxes, natural and synthetic gums, e.g., acacia, tragacanth, sodium alginate, and synthetic polymers such as polymethacrylates and polyvinylpyrrolidone. Lubricants can also facilitate tablet manufacture; non-limiting examples thereof include magnesium stearate, calcium stearate, stearic acid, glyceryl behenate, and polyethylene glycol. Disintegrants can facilitate tablet disintegration after administration, and non-limiting examples thereof include starches, alginic acid, crosslinked polymers such as, e.g., crosslinked polyvinylpyrrolidone, croscarmellose sodium, potassium or sodium starch glycolate, clays, celluloses, starches, gums and the like. Non-limiting examples of suitable glidants include silicon dioxide, talc, and the like. Stabilizers can inhibit or retard drug decomposition reactions, including oxidative reactions. Surfactants can also include and can be anionic, cationic, amphoteric or nonionic. If desired, the tablets can also comprise nontoxic auxiliary substances such as pH buffering agents, preservatives, e.g., antioxidants, wetting or emulsifying agents, solubilizing agents, coating agents, flavoring agents, and the like.

In an embodiment, a softgel formulation is made with a gelatin mass for the outer shell, and a composition including one or more substances, for example prebiotics and/or probiotic microorganisms, for the capsule fill can be prepared. To make the gelatin mass, gelatin powder can be mixed with water and glycerin, heated, and stirred under vacuum. Additives, for example, flavors or colors, can be added to molten gelatin using a turbine mixer and transferred to mobile vessels. The gelatin mass can be kept in a steam-jacketed storage vessel at a constant temperature.

The encapsulation process can begin when the molten gel is pumped to a machine and two thin ribbons of gel are formed on either side of machine. These ribbons can then pass over a series of rollers and over a set of die that determine the size and shapes of capsules. A fill composition, for example a prebiotic and/or probiotic microorganism fill composition, can be fed to a positive displacement pump, which can dose the fill and inject it between two gelatin ribbons prior to sealing them together through the application of heat and pressure. To remove excess water, the capsules can pass through a conveyer into tumble dryers where a portion of the water can be removed. The capsules can then be placed on, for example, trays, which can be stacked and transferred into drying rooms. In the drying rooms, dry air can be forced over capsules to remove any excess moisture.

### 3. Release Formulations

Immediate-release formulations of an effective amount of a prebiotic composition can comprise one or more combinations of excipients that allow for a rapid release of a pharmaceutically active agent (such as from 1 minute to 1 hour after administration). In an embodiment an excipient can be microcrystalline cellulose, sodium carboxymethyl cellulose, sodium starch glycolate, corn starch, colloidal silica, Sodium Laurel Sulphate, Magnesium Stearate, Prosolve SMCC (HD90), croscarmellose Sodium, Crospovidone NF, Avicel PH200, and combinations of such excipients.

"Controlled-release" formulations (also referred to as sustained release (SR), extended-release (ER, XR, or XL), time-release or timed-release, controlled-release (CR), or continuous-release) refer to the release of a prebiotic composition from a dosage form at a particular desired point in time after the dosage form is administered to a subject. Controlled-release formulations can include one or more excipients, including but not limited to microcrystalline cellulose, sodium carboxymethyl cellulose, sodium starch glycolate, corn starch, colloidal silica, Sodium Laurel Sulphate, Magnesium Stearate, Prosolve SMCC (HD90), croscarmellose Sodium, Crospovidone NF, or Avicel PH200. Generally, controlled-release includes sustained but otherwise complete release. A sudden and total release in the large intestine at a desired and appointed time or a release in the intestines such as through the use of an enteric coating are both considered controlled-release. Controlled-release can occur at a predetermined time or in a predetermined place within the digestive tract. It is not meant to include a passive, uncontrolled process as in swallowing a normal tablet. Examples include, but are not limited to, those described in U.S. Pat. Nos. 3,845,770; 3,916,899; 3,536,809; 3,598,123; 4,008,719; 5,674,533; 5,059,595; 5,591,767; 5,120,548; 5,073,543; 5,639,476; 5,354,556; 5,733,556; 5,871,776; 5,902,632; and 5,837,284 each of which is incorporated herein by reference in its entirety.

In an embodiment a controlled release dosage form begins its release and continues that release over an extended period of time. Release can occur beginning almost immediately or can be sustained. Release can be constant, can increase or decrease over time, can be pulsed, can be continuous or intermittent, and the like. Generally, however, the release of at least one pharmaceutically active agent from a controlled-release dosage form will exceed the amount of time of release of the drug taken as a normal, passive release tablet. Thus, for example, while all of at least one pharmaceutically active agent of an uncoated aspirin tablet should be released within, for example, four hours, a controlled-release dosage form could release a smaller amount of aspirin over a period of six hours, 12 hours, or even longer. Controlled-release in accordance with the compositions and methods described herein generally means that the release occurs for a period of six hours or more, such as 12 hours or more.

In another embodiment a controlled release dosage refers to the release of an agent, from a composition or dosage form in which the agent is released according to a desired profile over an extended period of time. In an embodiment, controlled-release results in dissolution of an agent within 20-720 minutes after entering the stomach. In another embodiment, controlled-release occurs when there is dissolution of an agent within 20-720 minutes after being swallowed. In another embodiment, controlled-release occurs when there is dissolution of an agent within 20-720 minutes after entering the intestine. In another embodiment, controlled-release results in substantially complete dissolution after at least 1 hour following administration. In another embodiment, controlled-release results in substantially complete dissolution after at least 1 hour following oral administration. For example, controlled-release compositions allow delivery of an agent to a subject in need thereof over an extended period of time according to a predetermined profile. Such release rates can provide therapeutically effective levels of agent for an extended period of time and thereby provide a longer period of pharmacologic or diagnostic response as compared with conventional rapid release dosage forms. Such longer periods of response provide for many inherent benefits that are not achieved with immediate-release dosages. When used in connection with the dissolution profiles discussed herein, the term "controlled-release" refers to wherein all or less than all of the total amount of a dosage form, made according to methods and compositions described herein, delivers an active agent over a period of time greater than 1 hour.

When present in a controlled-release oral dosage form, the compositions described herein can be administered at a substantially lower daily dosage level than immediate-release forms.

In an embodiment, the controlled-release layer is capable of releasing about 30 to about 40% of the one or more active agents (e.g., prebiotic and/or probiotic) contained therein in the stomach of a subject in need thereof in about 5 to about 10 minutes following oral administration. In another embodiment, the controlled-release layer is capable of releasing about 90% of the one or more active agents (e.g., prebiotic and/or probiotic) is released in about 40 minutes after oral administration.

In some embodiments, the controlled-release layer comprises one or more excipients, including but not limited to silicified microcrystalline cellulose (e.g., HD90), croscarmellose sodium (AC-Di-Sol), hydroxyl methyl propyl cellulose, magnesium stearate, or stearic acid. In an embodiment, a controlled release formulation weighs between about 100 mg to 3 g.

Pharmaceutical carriers or vehicles suitable for administration of the compounds provided herein include all such carriers additional to those skilled in the art to be suitable for the particular mode of administration. In addition, the compositions can one or more components that do not impair the desired action, or with components that supplement the desired action, or have another action.

In another embodiment, an effective amount of the prebiotic is formulated in an immediate release form. In this embodiment the immediate-release form can be included in an amount that is effective to shorten the time to its maximum concentration in the blood. By way of example, certain immediate-release pharmaceutical preparations are taught in United States Patent Publication US 2005/0147710A1 entitled, "Powder Compaction and Enrobing," which is incorporated herein in its entirety by reference.

The dosage forms described herein can also take the form of pharmaceutical particles manufactured by a variety of methods, including but not limited to high-pressure homogenization, wet or dry ball milling, or small particle precipitation (nano spray). Other methods to make a suitable powder formulation are the preparation of a solution of active ingredients and excipients, followed by precipitation, filtration, and pulverization, or followed by removal of the solvent by freeze-drying, followed by pulverization of the powder to the desired particle size.

In a further aspect the dosage form can be an effervescent dosage form. Effervescent means that the dosage form, when mixed with liquid, including water and saliva, evolves a gas. Some effervescent agents (or effervescent couple) evolve gas by means of a chemical reaction which takes place upon exposure of the effervescent disintegration agent to water or to saliva in the mouth. This reaction can be the result of the reaction of a soluble acid source and an alkali monocarbonate or carbonate source. The reaction of these two general compounds produces carbon dioxide gas upon contact with water or saliva. An effervescent couple (or the individual acid and base separately) can be coated with a solvent protective or enteric coating to prevent premature reaction. Such a couple can also be mixed with previously lyophilized particles (such as a prebiotic). The acid sources can be any which are safe for human consumption and can generally include food acids, acid and hydrite antacids such as, for example: citric, tartaric, amalic, fumeric, adipic, and succinics. Carbonate sources include dry solid carbonate and bicarbonate salt such as, preferably, sodium bicarbonate, sodium carbonate, potassium bicarbonate and potassium carbonate, magnesium carbonate and the like. Reactants which evolve oxygen or other gasses and which are safe for human consumption are also included. In an embodiment citric acid and sodium bicarbonate are used.

In another aspect the dosage form can be in a candy form (e.g., matrix), such as a lollipop or lozenge. In an embodiment an effective amount of a prebiotic is dispersed within a candy matrix. In an embodiment the candy matrix comprises one or more sugars (such as dextrose or sucrose). In another embodiment the candy matrix is a sugar-free matrix. The choice of a particular candy matrix is subject to wide variation. Conventional sweeteners such as sucrose can be utilized, or sugar alcohols suitable for use with diabetic patients, such as sorbitol or mannitol can be employed. Other sweeteners, such as the aspartames, can also be easily incorporated into a composition in accordance with compositions described herein. The candy base can be very soft and fast dissolving, or can be hard and slower dissolving. Various forms will have advantages in different situations.

A candy mass composition comprising an effective amount of the prebiotic can be orally administered to a subject in need thereof so that an effective amount of the prebiotic will be released into the subject's mouth as the candy mass dissolves and is swallowed. A subject in need thereof includes a human adult or child.

In an embodiment a candy mass is prepared that comprises one or more layers which can comprise different amounts or rates of dissolution of the prebiotic. In an embodiment a multilayer candy mass (such as a lollipop) comprises an outer layer with a concentration of the prebiotic differing from that of one or more inner layers. Such a drug delivery system has a variety of applications.

The choices of matrix and the concentration of the drug in the matrix can be important factors with respect to the rate of drug uptake. A matrix that dissolves quickly can deliver drug into the subject's mouth for absorption more quickly than a matrix that is slow to dissolve. Similarly, a candy matrix that contains the prebiotic in a high concentration can release more of the prebiotic in a given period of time than a candy having a low concentration. In an embodiment a candy matrix such as one disclosed in U.S. Pat. No. 4,671,953 or US Application Publication No. 2004/0213828 (which are herein incorporated by reference in their entirety) is used to deliver the prebiotic.

The dosage forms described herein can also take the form of pharmaceutical particles manufactured by a variety of methods, including but not limited to high-pressure homogenization, wet or dry ball milling, or small particle precipitation (e.g., nGimat's NanoSpray). Other methods useful to make a suitable powder formulation are the preparation of a solution of active ingredients and excipients, followed by precipitation, filtration, and pulverization, or followed by removal of the solvent by freeze-drying, followed by pulverization of the powder to the desired particle size. In an embodiment the pharmaceutical particles have a final size of 3-1000 µm, such as at most 3, 4, 5, 6, 7, 8, 9, 10, 20, 30, 40, 50, 60, 70, 80, 90, 100, 150, 200, 250, 300, 350, 400, 450, 500, 550, 600, 650, 700, 750, 800, 850, 900, 950, 1000 µm. In another embodiment the pharmaceutical particles have a final size of 10-500 µm. In another embodiment the pharmaceutical particles have a final size of 50-600 µm. In another embodiment the pharmaceutical particles have a final size of 100--800 µm.

In an embodiment an oral dosage form (such as a powder, tablet, or capsule) is provided comprising a prebiotic composition comprising about 0.7 g of FOS, GOS, or other FOS, GOS, or other appropriate polysaccharide, about 0.2 g of lactose, about 0.01 g of glucose, about 0.01 g of galactose, about 0.1-0.2 g of a binder, about 0.1-0.2 g of a dispersant, about 0.1-0.2 g of a solubilizer, wherein the FOS, GOS, or other FOS, GOS, or other appropriate polysaccharide are composed of about 1-25% disaccharides, about 1-25% trisaccharides, about 1-25% tetrasaccharides, and about 1-25% pentasaccharides. The oral dosage form can be in the form of a powder, capsule, or tablet. Suitable amounts of binders, dispersants, and solubilizers are additional in the art for preparation of oral tablets or capsules.

In another embodiment an oral dosage form (such as a powder, tablet or capsule) is provided comprising a prebiotic composition comprising about 1-99.9% by weight of FOS, GOS, or other FOS, GOS, or other appropriate polysaccharide about 0.5-20% by weight of lactose, about 0.1-2% by weight of glucose, about 0.1-2% by weight of galactose, about 0.05-2% by weight of a binder, about 0.05-2% by weight of a dispersant, about 0.05-2% by weight of a solubilizer, wherein the FOS, GOS, or other FOS, GOS, or other appropriate polysaccharide are composed of about 1-25% by weight disaccharides, about 1-25% by weight trisaccharides, about 1-25% by weight tetrasaccharides, and about 1-25% by weight pentasaccharides.

In another embodiment an oral dosage form (such as a powder, tablet, or capsule) is provided comprising a prebiotic composition comprising about 1, 10, 20, 30, 40, 50, 60, 70, 80, 90, 95, 99.5, 100% by weight of FOS, GOS, or other FOS, GOS, or other appropriate polysaccharide about 0, 5, 10, 15, or 20% by weight of lactose, about 0.1, 0.5, 1, or 2% by weight of glucose, about 0.1, 0.5, 1, or 2% by weight of galactose, about 0.05, 0.1, 0.5, 1, or 2% by weight of a binder, about 0.05, 0.1, 0.5, 1, or 2% by weight of a dispersant, about 0.05, 0.1, 0.5, 1, or 2% by weight of a solubilizer, wherein the FOS, GOS, or other FOS, GOS, or other appropriate polysaccharide are composed of about 1, 5, 10, 15, 20, or 25% by weight disaccharides, about 1, 5, 10, 15, 20, or 25% by weight trisaccharides, about 1, 5, 10, 15, 20, or 25% by weight tetrasaccharides, and about 1, 5, 10, 15, 20, or 25% by weight pentasaccharides.

In another embodiment, an oral dosage form is provided comprising a prebiotic composition, wherein the oral dosage form is a syrup. The syrup can comprise about 1%, 5%, 10%, 15%, 20%, 25%, 30%, 35%, 40%, 45%, 50%, 55%, 60%, 65%, 70%, 75%, 80%, or 85% solid. The syrup can comprise about 15%, 20%, 25%, 30%, 35%, 40%, 45%, or 50% liquid, for example, water. The solid can comprise a prebiotic composition. The solid can be, for example, about 1-96%, 10-96%, 20-96%, 30-96%, 40-96%, 50-96%, 60-96%, 70-96%, 80-96%, or 90-96% prebiotic composition. The solid can be, for example, about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, or 96% prebiotic composition. In an embodiment a prebiotic composition comprises FOS, GOS, or other FOS, GOS, or other appropriate polysaccharide. In another embodiment a prebiotic composition comprises FOS, GOS, or other FOS, GOS, or other appropriate polysaccharide and another prebiotic. In another embodiment a prebiotic composition comprises FOS, GOS or other and inulin or GOS and FOS.

In an embodiment, the softgel capsule is about 0.25 mL, 0.5 mL, 1.0 mL, 1.25 mL, 1.5 mL, 1.75 mL, or 2.0 mL. In another embodiment, a softgel capsule comprises about 0.1 g to 2.0 g of prebiotic composition. In another embodiment, a softgel capsule comprises about 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, or 2.0 g of a prebiotic composition. In an embodiment the prebiotic composition comprises FOS, GOS, or other FOS, GOS, or other appropriate polysaccharide. In another embodiment the prebiotic composition consists essentially of FOS, GOS, or other FOS, GOS, or other appropriate polysaccharide. In another embodiment, a softgel capsule comprises FOS, GOS, or other FOS, GOS, or other appropriate polysaccharide and inulin or FOS.

In another embodiment, the prebiotic composition is delivered in a gelatin capsule containing an amount of FOS, GOS, or other FOS, GOS, or other appropriate polysaccharide within the ranges listed in Table 2. In another embodiment, the number of pills taken per day is within the ranges listed in Table 2.

**Table 2.**

| Exemplary GOS Dosing Units | | |
|---|---|---|
| Exemplary GOS Composition | | |
| Dosages in Gel Caps | | |
| Size | GOS/Pill (g) | # pills per day |
| 000 | 1-2 | 1-15 |
| 00 | 0.6-1.5 | 1-25 |
| 0 | 0.4-1.1 | 1-38 |
| 1 | 0.3-0.8 | 1-50 |
| 2 | 0.25-0.6 | 1-60 |
| 3 | 0.2-0.5 | 1-75 |
| 4 | 0.14-0.3 | 1-107 |

In another embodiment, a prebiotic composition is provided that does not contain a preservative. In another embodiment, a prebiotic composition is provided that does not contain an antioxidant. In another embodiment, a prebiotic composition is provided that does not contain a preservative or an antioxidant. In an embodiment a prebiotic composition comprising FOS, GOS, or other FOS, GOS, or other appropriate polysaccharide does not contain a preservative or an antioxidant.

In another embodiment, a prebiotic composition is formulated as a viscous fluid. In another embodiment, a prebiotic composition is formulated such that its water content is low enough that it does not support microbial growth. In an embodiment, this composition is an intermediate-moisture food, with a water activity between 0.6 and 0.85; in another embodiment this composition is a low-moisture food, with a water activity less than 0.6. Low-moisture foods limit microbial growth significantly and can be produced by one of ordinary skill in the art. For example, these products could be produced similarly to a liquid-centered cough drop. In another embodiment, a prebiotic composition is formulated as a viscous fluid without a preservative in a gel capsule. In another embodiment, a prebiotic composition comprising FOS, GOS, or other FOS, GOS, or other appropriate polysaccharide is a viscous fluid. In another embodiment, a prebiotic composition comprises a high percentage of FOS, GOS, or other FOS, GOS, or other appropriate polysaccharide that does not support microbial growth. In another embodiment, the prebiotic composition comprises FOS, GOS, or other FOS, GOS, or other appropriate polysaccharide and inulin or FOS.

In another embodiment, an oral dosage form is provided comprising a prebiotic composition, wherein the oral dosage form is a softgel. In an embodiment the softgel comprises a syrup. In an embodiment the syrup comprises a prebiotic composition. In an embodiment the prebiotic composition comprises FOS, GOS, or other FOS, GOS, or other appropriate polysaccharide. In another embodiment the prebiotic composition comprises more than 80% FOS, GOS, or other FOS, GOS, or other appropriate polysaccharide. In another embodiment the prebiotic composition comprises between 80-99.9% FOS, GOS, or other. In another embodiment the prebiotic composition comprises more than 80% FOS, GOS, or other FOS, GOS, or other appropriate polysaccharide. In another embodiment the prebiotic composition comprises about 80, 81, 82, 83, 84, 85, 86, 87, 88, 89, 90, 91, 92, 93, 94, 95, 96, 97, 98, 99, or 99.9% FOS, GOS, or other FOS, GOS, or other appropriate polysaccharide.

In an embodiment a FOS, GOS, or other FOS, GOS, or other appropriate polysaccharide composition is formulated for delivery in a soft gel capsule. In an embodiment a FOS, GOS, or other FOS, GOS, or other appropriate polysaccharide composition formulated for delivery in a soft gel capsule is a high percentage FOS, GOS, or other FOS, GOS, or other appropriate polysaccharide composition, such as a 90-100% FOS, GOS, or other FOS, GOS, or other appropriate polysaccharide composition (e.g., 90, 91, 92, 93, 94, 95, 96, 97, 98, 99 or 100% FOS, GOS, or other FOS, GOS, or other appropriate polysaccharide composition by weight). In another embodiment a FOS, GOS, or other FOS, GOS, or other appropriate polysaccharide composition formulated for delivery in a soft gel capsule comprises about 95% FOS, GOS, or other FOS, GOS, or other appropriate polysaccharide. In another embodiment a FOS, GOS, or other FOS, GOS, or other appropriate polysaccharide composition formulated for delivery in a soft gel capsule comprises about 96% FOS, GOS, or other FOS, GOS, or other appropriate polysaccharide. In another embodiment, the FOS, GOS, or other FOS, GOS, or other appropriate polysaccharide composition is formulated such that its water content is low enough that it does not support microbial growth. In another embodiment, the FOS, GOS, or other FOS, GOS, or other appropriate polysaccharide composition is formulated as a viscous fluid without a preservative in a gel capsule. In another embodiment, the FOS, GOS, or other FOS, GOS, or other appropriate polysaccharide composition is formulated as a viscous fluid without an antioxidant in a gel capsule. In another embodiment the soft gel capsule comprises about 0.1-2 g of a FOS, GOS, or other FOS, GOS, or other appropriate polysaccharide composition.

In another embodiment a prebiotic composition can be formulated as described, in U.S. Pat. No. 6,750,331, which is herein incorporated by reference in its entirety. A prebiotic composition can be formulated to comprise an oligosaccharide, a foaming component, a water-insoluble dietary fiber (e.g., cellulose or lignin), or a neutralizing component. In an embodiment a prebiotic composition can be in the form of a chewable tablet.

In an embodiment a foaming component can be at least one member selected from the group consisting of sodium hydrogencarbonate, sodium carbonate, and calcium carbonate. In an embodiment a neutralizing component can be at least one member selected from the group consisting of citric acid, L-tartaric acid, fumaric acid, L-ascorbic acid, DL-malic acid, acetic acid, lactic acid, and anhydrous citric acid. In an embodiment a water-insoluble dietary fiber can be at least one member selected from the group consisting of crystalline cellulose, wheat bran, oat bran, cone fiber, soy fiber, and beet fiber. The formulation can contain a sucrose fatty acid ester, powder sugar, fruit juice powder, and/or flavoring material.

Formulations of the provided invention can include additive components selected from various additional additives. Such additives include, for example, saccharides (excluding oligosaccharides), sugar alcohols, sweeteners and like excipients, binders, disintegrators, lubricants, thickeners, surfactants, electrolytes, flavorings, coloring agents, pH modifiers, fluidity improvers, and the like. Specific examples of the additives include wheat starch, potato starch, corn starch, dextrin and like starches; sucrose, glucose, fructose, maltose, xylose, lactose and like saccharides (excluding oligosaccharides); sorbitol, mannitol, maltitol, xylitol and like sugar alcohols; calcium phosphate, calcium sulfate and like excipients; starch, saccharides, gelatine, gum arabic, dextrin, methyl cellulose, polyvinylpyrrolidone, polyvinyl alcohol, hydroxypropylcellulose, xanthan gum, pectin, gum tragacanth, casein, alginic acid and like binders and thickeners; leucine, isoleucine, L-valine, sugar esters, hardened oils, stearic acid, magnesium stearate, talc, macrogols and like lubricants; CMC, CMC-Na, CMC-Ca and like disintegrators; polysorbate, lecithin and like surfactants; aspartame, alitame and like dipeptides; silicon dioxide and like fluidity improvers; and stevia, saccharin, and like sweeteners. The amounts of these additives can be properly selected based on their relation to other components and properties of the preparation, production method, etc.

In an embodiment, a FOS, GOS, or other FOS, GOS, or other appropriate polysaccharide composition is a chewable oral dosage formulation. In an embodiment the chewable formulation can comprises between about 1-99.9% FOS, GOS, or other FOS, GOS, or other appropriate polysaccharide. In an embodiment, a FOS, GOS, or other FOS, GOS, or other appropriate polysaccharide composition comprises about 80% FOS, GOS, or other FOS, GOS, or other appropriate polysaccharide about 5% L-ascorbic acid, about 2% anhydrous citric acid, about 3% sodium hydrogencarbonate, about 3% calcium carbonate, about 2% sucrose fatty acid, about 3% fruit juice powder, and about 2% potassium carbonate.

In another embodiment, a FOS, GOS, or other FOS, GOS, or other appropriate polysaccharide composition comprises about 85% FOS, GOS, or other FOS, GOS, or other appropriate polysaccharide, about 5% L-ascorbic acid, about 3% sodium hydrogencarbonate, about 2% sodium carbonate, about 2% sucrose fatty acid ester, about 2% fruit juice powder, and about 1% potassium carbonate.

In another embodiment, a FOS, GOS, or other FOS, GOS, or other appropriate polysaccharide composition comprises about 90% FOS, GOS, or other FOS, GOS, or other appropriate polysaccharide, about 2% L-ascorbic acid, about 1% anhydrous citric acid, about 2% sodium hydrogencarbonate, about 2% sodium carbonate, about 2% sucrose fatty acid ester, and about 1% potassium carbonate.

In another embodiment, a FOS, GOS, or other FOS, GOS, or other appropriate polysaccharide composition comprises about 95% FOS, GOS, or other FOS, GOS, or other appropriate polysaccharide, about 2% L-ascorbic acid, about 1% sodium hydrogencarbonate, and about 2% fruit juice powder. In another embodiment, a FOS, GOS, or other FOS, GOS, or other appropriate polysaccharide composition comprises about 95% FOS, GOS, or other FOS, GOS, or other appropriate polysaccharide and about 5% of L-ascorbic acid, anhydrous citric acid, sodium hydrogencarbonate, calcium carbonate, sucrose fatty acid, fruit juice powder, or potassium carbonate.

In another embodiment, a FOS, GOS, or other FOS, GOS, or other appropriate polysaccharide composition comprises about 95% FOS, GOS, or other FOS, GOS, or other appropriate polysaccharide and about 5% of L-ascorbic acid, anhydrous citric acid, sodium hydrogencarbonate, calcium carbonate, sucrose fatty acid, fruit juice powder, and potassium carbonate.

### Medical Foods

An alternate embodiment of the present invention is a formulation as a medical food.

The consuming public has come to understand that foods possess more than basic nutrition (protein, carbohydrate, fat, etc). For example, 95% of consumers agree that "certain foods have health benefits that go beyond basic nutrition and may reduce the risk of disease or other health concerns." More than 50% of consumers believe that foods can replace the use of drugs. Replacing the use of drugs may have the benefit of reducing the incidence of adverse side effects suffered by patients following a pharmaceutical drug treatment regimen. In fact, medical foods are assumed to be generally safe, as people have historically consumed these foods safely in non-medical contexts.

The compositions of the invention may be administered under the supervision of a medical specialist, or may be self-administered. Medical foods could take the form of nutritional shakes or other liquids or meal replacements or delivery by nasogastric enteral feeding tube. Medical foods of the present invention could also take the form of a powder capable of being consumed upon addition to suitable food or liquid.

A medical food formulation of the present invention could confer benefits of a synthetic composition of microbes isolated from nutritionally beneficial plants, as well as the benefits of prebiotics, or other nutritionally beneficial inclusions, but not consumed to obtain nutrition from them but rather to provide a metabolic function different than a foodstuff. For example, medical foods of the invention may also include at least one vitamin, or vitamin precursor. Preferred vitamins possess antioxidant properties and include vitamins A, C and E, and/or their biochemical precursors. Another embodiment of the medical foods of the invention also includes at least one trace element, preferably selected from the group consisting of zinc, manganese and selenium. Medical foods of the invention also may include at least one additional antioxidant selected from the group consisting of carotenoids, N-acetylcysteine and L-glutamine. It is additional to those of skill in the art how to construct medical foods containing these elements.

Medical foods of the present invention would include effective doses of microbes deemed useful for the indication and effective doses of any vitamin, prebiotic, or other beneficial additive not consumed to obtain nutrition but to add a therapeutic benefit mediated by the production of SCFA or other immuno-stimulant molecules when passing through the GI tract.

Typically, the dietary supplements and medical foods of the present invention are consumed at least once daily, and preferably administered two times per day, preferably once in the morning and once in the afternoon. A typical treatment regime for the dietary supplements or medical foods will continue for four to eight weeks. Depending on such factors as the medical condition being treated and the response of the patient, the treatment regime may be extended. A medical food of the present invention will typically be consumed in two servings per day as either a meal replacement or as a snack between meals.

Anyone perceived to be at risk from metabolic syndrome, obesity, T2D, digestive distress, bone loss, low bone density, chronic inflammation or already suffering from these or associated disorders, can potentially benefit from ingesting the compositions of the invention. According to the invention it is believed to be possible to effectively ameliorate symptoms and conditions associated with T2D, metabolic syndrome, obesity, digestive distress, bone loss, low bone density, or chronic inflammation with natural compounds, which do not show any severe side effects. Furthermore, the present methods are expected to be well-tolerated, for example without causing any discomfort or nausea, and simple to apply.

### Co-administration

One or more of the microbial entities described herein and/or additional compounds can be co-administered together. The term "co-administration" refers to two or more microbial entities and/or compounds administered in such a way that they exert their pharmacological effects for the same period of time. Such co-administration may be accomplished by simultaneous, contemporaneous or sequential administration of the two or more microbial entities and/or compounds. Compounds can include a therapeutic agent or a drug. A therapeutic agent or drug can be any agent used in the treatment of a human disease. For example in type 2 diabetes or metabolic syndrome, the compound can be an anti-diabetic medication including, but not limited to, metformin, Acarbose, Miglitol, Voglibose, Sitagliptin, Saxagliptin, Liraglutide, Pioglitazone, dipeptidyl peptidase-4 (DPP4)-inhibitors, glucagon-like peptide-1 (GLP-1) receptor analogs, alpha glucosidase inhibitors, thiazolidinedione, and sodium/glucose cotransporter 2 (SGLT2) inhibitors. In another example, the compound can be an anti-osteoporosis medication including, but not limited to, bisphosphonates (*e.g.,* alendronate, risedronate, ibandronate, zolendronate), biologics (*e.g.,* denosumab, romosozumab), selective estrogen receptor mediators (*e*.*g*., Raloxifene), or anabolic agents (*e*.*g*., teriparatide, abaloparatide).

In some embodiments, the probiotic compositions described herein are co-administered with an effective amount of an additional therapeutic agent or along with an effective drug regimen to improve the efficacy of said therapeutic agent or drug regimen.

In some embodiments, the probiotic compositions described herein are co-administered with an effective amount of an additional therapeutic agent or along with an effective drug regimen to reduce the side effects of said therapeutic agent or drug regimen.

Co-administration encompasses methods given or given in any combination of simultaneously, sequentially, intermittently and/or continuously. In one aspect, the present disclosure provides a method wherein of the two or more microbial entities and/or compounds is administered intermittently. In one aspect, the present disclosure provides a subject with the two or more microbial entities and/or compounds at least ten (10) minutes, fifteen (15) minutes, twenty (20) minutes between administrations. Minutes, thirty (30) minutes, forty (40) minutes, sixty (60) minutes, two (2) hours, three (3) hours, four (4) hours, six (6) hours, eight (8) hours, Ten (10) hours, twelve (12) hours, fourteen (14) hours, eighteen (18) hours, twenty-four (24) hours, thirty-six (36) hours, forty-eight (48) hours, three (3) days , Four (4), five (5), six (6), seven (7), eight (8), nine (9), ten (10), ten (11), twelve (12) days, thirteen (13) days, fourteen (14) days, three (3) weeks, or four (4) weeks of delayed administration. In such embodiments, delayed administration is continued wherein the two or more microbial entities and/or compounds is continued for a given period of time from about ten (10) minutes to about three hundred and sixty-five (365) days. Followed by a pattern that is not administered for a given period of time from about ten (10) minutes to about thirty (30) days. In one aspect, the present disclosure provides a method wherein of the two or more microbial entities and/or compounds is administered intermittently while the remainder is continuously given. The two or more microbial entities and/or compounds can be administered at different intervals.

In one aspect, the present disclosure provides a method of the two or more microbial entities and/or compounds is administered sequentially. In one aspect, the present disclosure provides a method of simultaneously administering of the two or more microbial entities and/or compounds.

In some embodiments, the present disclosure provides a method of administering the two or more microbial entities and/or compounds in one formulation. In some embodiments, the present disclosure provides a method of administering a combination in two (2) compositions, such as wherein the two or more microbial entities are separate from the formulation of a compound (*e.g.,* a drug or therapeutic agent), or wherein the two or more microbial entities are in separate formulations (e.g., a probiotic microorganism, such as a GRAS probiotic microorganism, is separate from the one or more microbial entities presented in Table 4 or 7).

The two or more microbial entities and/or compounds can be co-administered via different routes, *e.g.,* a probiotic composition can be administered orally and a drug or therapeutic agent can be administered intravenously, subcutaneously, or any other route appropriate for administration of the drug or therapeutic agent, as will be appreciated by one skilled in the art.

### EXAMPLES

Below are examples of specific embodiments for carrying out the present invention. The examples are offered for illustrative purposes only, and are not intended to limit the scope of the present invention in any way. Efforts have been made to ensure accuracy with respect to numbers used (e.g., amounts, temperatures, etc.), but some experimental error and deviation should, of course, be allowed for.

### Example 1: Microbial preparations and metagenomic analyses.

A sample set of 15 vegetables typically eaten raw was selected to analyze the microbial communities by whole genome shotgun sequencing and comparison to microbial databases. The 15 fruits and vegetable samples are shown in Table 3 and represent ingredients in typical salads or eaten fresh. The materials were sourced at the point of distribution in supermarkets selling both conventional and organic farmed vegetables, either washed and ready to eat or without washing.

The samples were divided into 50 g portions, thoroughly rinsed with tap water and blended for 30 seconds on phosphate buffer pH 7.4 (PBS) in a household blender. The resulting slurry was strained by serial use of a coarse household sieve and then a fine household sieve followed by filtration through a 40 µm sieve. The cell suspension containing the plant microbiota, chloroplasts and plant cell debris was centrifuged at slow speed (100 x g) 5 minutes for removing plant material and the resulting supernatant centrifuged at high speed (4000 x g) 10 minutes to pellet microbial cells. The pellet was resuspended in a plant cell lysis buffer containing a chelator such as EDTA 10 mM to reduce divalent cation concentration to less than, and a non-ionic detergent to lyse the plant cells without destroying the bacterial cells. The lysed material was washed by spinning down the microbial cells at 4000 x g for 10 minutes, and then resuspended in PBS and re-pelleted as above. For sample #12 (broccoli) the cell pellet was washed and a fraction of the biomass separated and only the top part of the pellet collected. This was deemed "broccoli juice" for analyses. The resulting microbiota prep was inspected under fluorescence microscopy with DNA stains to visualize plant and microbial cells based on cell size and DNA structure (nuclei for plants) and selected for DNA isolation based on a minimum ratio of 9:1 microbe to plant cells. The DNA isolation was based on the method reported by Marmur (Journal of Molecular Biology 3, 208-218; 1961), or using commercial DNA extraction kits based on magnetic beads such as Thermo Charge Switch resulting in a quality suitable for DNA library prep and free of PCR inhibitors.

The DNA was used to construct a single read 150 base pair libraries and a total of 26 million reads sequenced per sample according to the standard methods done by CosmosID (www.cosmosid.com) for samples #1 to #12 or 300 base pair-end libraries and sequenced in an Illumina NextSeq instrument covering 4 Gigabases per sample for samples #13 to #15. The unassembled reads were then mapped to the CosmosID for samples #1 to #12 or OneCodex for samples #13 to #15 to databases containing 36,000 reference bacterial genomes covering representative members from diverse taxa.

In addition to the shotgun metagenomics survey, relevant microbes were isolated from fruits and vegetables listed in Table 3 using potato dextrose agar or nutrient agar and their genomes sequenced to cover 50X. Their metabolic potential was analyzed by using genome-wide models. For example, a yeast isolated from blueberries was sequenced and its genome showed identity to *Aureobasidium subglaciale* assembled in contigs with an N50 of 71 Kb and annotated to code for 10, 908 genes. Similarly, bacterial genomes from the same sample were sequenced and annotated for strains with high identity to *Pseudomonas* and *Rahnella.*

Bacteria and fungi isolated from the fresh fruits and vegetables were formulated to upgrade probiotic microorganisms such as *Leuconostoc mesenteroides, Lactobacillus plantarum, Lactobacillus paracasei,* and/or *Lactobacillus brevis.* To do this, the genomes of the probiotic target strains were combined *in silico* with the genomes of helper strains that will enhance the production of short chain fatty acids such as acetate, propionate and butyrate. The helper strains are members represented in Table 4 or Table 7 and includes yeast such as *Saccharomyces, Debaromyces, Hanseniaspora,* and *Pichia* among others.

**Table 3. Samples analyzed.**

| **Sample number** | **sample description** |
|---|---|
| 1 | chard |
| 2 | red cabbage |
| 3 | organic romaine |
| 4 | organic celery |
| 5 | butterhead organic lettuce |
| 6 | organic baby spinach |
| 7 | crisp green gem lettuce |
| 8 | red oak leaf lettuce |
| 9 | green oak leaf lettuce |
| 10 | cherry tomato |
| 11 | crisp red gem lettuce |
| 12 | broccoli juice |
| 13 | broccoli head |
| 14 | blueberries |
| 15 | pickled olives |
| 16 | early girl tomatoes |
| 17 | organic brined olives |
| 18 | fermented cabbage |

### Results

For most samples, bacterial abundances of fresh material contain about 10^4 to 10^8 microbes per gram of vegetable as estimated by direct microscopy or viable counts. Diverse cell morphologies were observed including rods, elongated rods, cocci and fungal hyphae. Microorganisms were purified from host cells, DNA was isolated and sequenced using a shotgun approach mapping reads to 35,000 bacterial genomes using a k-mer method. All samples were dominated by gamma proteobacteria, primarily *Pseudontonadacea,* presumably largely endophytes as some samples were triple washed before packaging. *Pseudomonas* cluster was the dominant genera for several samples with 10-90% of the bacterial relative abundance detected per sample and mapped to a total of 27 different genomes indicating it is a diverse group. A second relevant bacterial strain identified was *Duganella zoogloeoides* ATCC 25935 as it was present in almost all the samples ranging from 1-6 % of the bacterial relative abundance detected per sample or can reach 29% of the bacterial relative abundance detected per sample in organic romaine. Red cabbage was identified to contain a relatively large proportion of lactic acid bacteria as it showed 22% *Lactobacillus crispatus,* a species commercialized as probiotic and recognized relevant in vaginal healthy microbial community. Another vegetable containing lactic acid bacteria was red oak leaf lettuce containing 1.5% of the bacterial relative abundance detected per sample *Lactobacillus reuteri.* Other bacterial species recognized as probiotics included *Bacillus, Bacteroidetes, Propionibacterium* and *Streptococcus.* A large proportion of the abundant taxa in most samples was associated with plant microbiota and members recognized to act as biocontrol agents against fungal diseases or growth promoting agents such as *Pseudomonas fluorescens.* The aggregated list of unique bacteria detected by the k-mer method is 318 (Table 4).

Blueberries contained a mixture of bacteria and fungi dominated by *Pseudomonas* and *Propionibacterium* but the yeast *Aureobasidium* was identified as a relevant member of the community. A lesser abundant bacterial species was *Rahnella.* Pickled olives are highly enriched in lactic acid bacteria after being pickled in brine allowing the endogenous probiotic populations to flourish by acidifying the environment and eliminating most of the acid-sensitive microbes including bacteria and fungi. This resulted in a large amount of *Lactobacillus* species and *Pediococcus* recognized as probiotics and related to obesity treatment.

The shotgun sequencing method allows for the analysis of the metagenome including genes coding for metabolic reactions involved in the assimilation of nutrient, fermentative processes to produce short chain fatty acids, flavonoids and other relevant molecules in human nutrition.

**Table 4. Bacteria identified in a 15 sample survey identified by whole genome matching to reference genomes. The fruits and vegetables were selected based on their recognition as part of the whole food plant based diet and some antidiabetic, anti-obesogenic, anti-inflammatory and bone health properties. There is general recognition of microbes in these vegetables relevant for plant health such as water stress resilience or biocontrol agents but not previously recognized for their use in human health.**

| Strain identified by k-mer based on entire genome | Strain number | Collection |
|---|---|---|
| *Acinetobacter baumannii* | - | |
| *Acinetobacter soli* | - | |
| *Acinetobacter* 41764 Branch | - | |
| *Acinetobacter* 41930 Branch | - | |
| *Acinetobacter* 41981 Branch | - | |
| *Acinetobacter* 41982 Branch | - | |
| *Acinetobacter baumannii 348935* | - | |
| *Acinetobacter baumannii* 40298 Branch | - | |
| *Acinetobacter beijerinckii* 41969 Branch | - | |
| *Acinetobacter beijerinckii* CIP 110307 | CIP 110307 | WFCC |
| *Acinetobacter bohemicus* ANC 3994 | - | |
| *Acinetobacter guillouiae* 41985 Branch | - | |
| *Acinetobacter guillouiae* 41986 Branch | - | |
| *Acinetobacter gyllenbergii* 41690 Branch | - | |
| *Acinetobacter haemolyticus* TG19602 | - | |
| *Acinetobacter harbinensis* strain HITLi 7 | - | |
| *Acinetobacter johnsonii* 41886 Branch | - | |
| *Acinetobacter johnsonii* ANC 3681 | - | |
| *Acinetobacter junii* 41994 Branch | - | |
| *Acinetobacter lwoffii* WJ10621 | - | |
| *Acinetobacter* sp 41945 Branch | - | |
| *Acinetobacter* sp 41674 Branch | - | |
| *Acinetobacter* sp 41698 Branch | - | |
| *Acinetobacter* sp ETR1 | - | |
| *Acinetobacter* sp NIPH 298 | - | |
| *Acinetobacter tandoii* 41859 Branch | - | |
| *Acinetobacter tjernbergiae* 41962 Branch | - | |
| *Acinetobacter towneri* 41848 Branch | - | |
| *Acinetobacter venetianus* VE C3 | - | |
| *Actinobacterium* LLX17 | - | |
| *Aeromonas bestiarum* strain CECT 4227 | CECT 4227 | CECT |
| *Aeromonas caviae* strain CECT 4221 | CECT 4221 | CECT |
| *Aeromonas hydrophila* 4AK4 | - | |
| *Aeromonas media* 37528 Branch | - | |
| *Aeromonas media* strain ARB 37524 Branch | - | |
| *Aeromonas salmonicida* subsp 37538 Branch | - | |
| *Aeromonas* sp ZOR0002 | - | |
| *Agrobacterium* 22298 Branch | - | |
| *Agrobacterium* 22301 Branch | - | |
| *Agrobacterium* 22313 Branch | - | |
| *Agrobacterium* 22314 Branch | - | |
| *Agrobacterium* sp ATCC 31749 | ATCC 31749 | ATCC |
| *Agrobacterium tumefaciens* 22306 Branch | | |
| *Agrobacterium tumefaciens* strain MEJ076 | - | |
| *Agrobacterium tumefaciens* strain S2 | - | |
| *Alkanindiges illinoisensis* DSM 15370 | DSM 15370 | WFCC |
| alpha proteobacterium L41A | - | |
| *Arthrobacter* 20515 Branch | - | |
| *Arthrobacter arilaitensis* Re117 | - | |
| *Arthrobacter chlorophenolicus* A6 | - | |
| *Arthrobacter nicotinovorans* 20547 Branch | - | |
| *Arthrobacter phenanthrenivorans* Sphe3 | - | |
| *Arthrobacter* sp 20511 Branch | - | |
| *Arthrobacter* sp PAO19 | - | |
| *Arthrobacter* sp W1 | - | |
| *Aureimonas* sp. Leaf427 | - | |
| *Aureobasidium pullulans* | - | |
| *Bacillaceae* Family 24 4101 12691 Branch | - | |
| *Bacillus* sp. LL01 | - | |
| *Bacillus* 12637 Branch | - | |
| *Bacillus aerophilus* strain C772 | - | |
| *Bacillus thuringiensis* serovar 12940 Branch | - | |
| *Brevundimonas nasdae* strain TPW30 | - | |
| *Brevundimonas* sp 23867 Branch | - | |
| *Brevundimonas* sp EAKA | - | |
| *Buchnera aphidicola* str 28655 Branch | - | |
| *Burkholderiales* Order 15 6136 Node 25777 | - | |
| *Buttiauxella agrestis* 35837 Branch | - | |
| *Candidatus Burkholderia verschuerenii* | - | |
| *Carnobacterium* 5833 Branch | - | |
| *Carnobacterium maltaromaticum* ATCC 35586 | ATCC 35586 | ATCC |
| *Chryseobacterium* 285 Branch | - | |
| *Chryseobacterium daeguense* DSM 19388 | DSM 19388 | WFCC |
| *Chryseobacterium formosense* | - | |
| *Chryseobacterium* sp YR005 | - | |
| *Clavibacter* 20772 Branch | - | |
| *Clostridium diolis* DSM 15410 | DSM 15410 | WFCC |
| *Comamonas* sp B 9 | - | |
| *Curtobacterium flaccumfaciens* 20762 Branch | - | |
| *Curtobacterium flaccumfaciens* UCD AKU | - | |
| *Curtobacterium* sp UNCCL17 | - | |
| *Deinococcus aquatilis* DSM 23025 | DSM 23025 | WFCC |
| *Debaromyces hansenii* ATCC 36239 | ATCC 25935 | ATCC |
| *Duganella zoogloeoides* ATCC 25935 | - | |
| *Dyadobacter* 575 Branch | - | |
| *Elizabethkingia anophelis* | - | |
| *Empedobacter falsenii* strain 282 | - | |
| *Enterobacter* sp 638 | - | |
| *Enterobacteriaceae* Family 9 3608 Node 35891 | - | |
| *Enterobacteriaceae* Family 9 593 Node 36513 | - | |
| *Epilithonimonas lactis* | - | |
| *Epilithonimonas tenax* DSM 16811 | DSM 16811 | WFCC |
| *Erwinia* 35491 Branch | - | |
| *Erwinia amylovora* 35816 Branch | - | |
| *Erwinia pyrifoliae* 35813 Branch | - | |
| *Erwinia tasmaniensis* Et1 99 | DSM 17950 | WFCC |
| *Escherichia coli* ISC11 | - | |
| *Exiguobacterium* 13246 Branch | - | |
| *Exiguobacterium* 13260 Branch | - | |
| *Exiguobacterium sibiricum* 255 15 | DSM 17290 | WFCC |
| *Exiguobacterium* sp 13263 Branch | - | |
| *Exiguobacterium undae* 13250 Branch | - | |
| *Exiguobacterium undae* DSM 14481 | DSM 14481 | WFCC |
| *Flavobacterium* 237 Branch | - | |
| *Flavobacterium aquatile* LMG 4008 | LMG 4008 | WFCC |
| *Flavobacterium chungangense* LMG 26729 | LMG 26729 | WFCC |
| *Flavobacterium daejeonense* DSM 17708 | DSM 17708 | WFCC |
| *Flavobacterium hibernum* strain DSM 12611 | DSM 12611 | WFCC |
| *Flavobacterium hydatis* | - | |
| *Flavobacterium johnsoniae* UW101 | ATCC 17061D-5 | ATCC |
| *Flavobacterium reichenbachii* | - | |
| *Flavobacterium soli* DSM 19725 | DSM 19725 | WFCC |
| *Flavobacterium* sp 238 Branch | - | |
| *Flavobacterium* sp EM1321 | - | |
| *Flavobacterium* sp MEB061 | - | |
| *Hanseniaspora uvarum* ATCC 18859 | - | |
| *Hanseniaspora occidentalis* ATCC 32053 | - | |
| *Herminiimonas arsenicoxydans* | - | |
| *Hymenobacter swuensis* DY53 | - | |
| *Janthinobacterium* 25694 Branch | - | |
| *Janthinobacterium agaricidamnosum* | DSM 9628 | WFCC |
| *Janthinobacterium lividum* strain RIT308 | - | |
| *Janthinobacterium* sp RA13 | - | |
| *Kocuria* 20614 Branch | - | |
| *Kocuria rhizophila* 20623 Branch | - | |
| *Lactobacillus acetotolerans* | - | |
| *Lactobacillus brevis* | - | |
| *Lactobacillus buchneri* | - | |
| *Lactobacillus futsaii* | - | |
| *Lactobacillus kefiranofaciens* | - | |
| *Lactobacillus panis* | - | |
| *Lactobacillus parafarraginis* | - | |
| *Lactobacillus plantarum* | - | |
| *Lactobacillus rapi* | - | |
| *Lactobacillus crispatus* 5565 Branch | - | |
| *Lactobacillus plantarum* WJL | - | |
| *Lactobacillus reuteri* 5515 Branch | - | |
| *Leuconostoc mesenteroides* ATCC 8293 | - | |
| *Luteibacter* sp 9135 | - | |
| *Massilia timonae* CCUG 45783 | - | |
| *Methylobacterium extorquens* 23001 Branch | - | |
| *Methylobacterium* sp 22185 Branch | - | |
| *Methylobacterium* sp 285MFTsu5 1 | - | |
| *Methylobacterium* sp 88A | - | |
| *Methylotenera versatilis 7* | - | |
| *Microbacterium laevaniformans* OR221 | - | |
| *Microbacterium oleivorans* | - | |
| *Microbacterium* sp MEJ108Y | - | |
| *Microbacterium* sp UCD TDU | - | |
| *Microbacterium testaceum* StLB037 | - | |
| *Micrococcus luteus* strain RIT304 | NCTC 2665 | NCTC |
| *Mycobacterium abscessus* 19573 Branch | - | |
| *Neosartorya fischeri* | - | |
| *Oxalobacteraceae bacterium* AB 14 | - | |
| *Paenibacillus* sp FSL 28088 Branch | - | |
| *Paenibacillus* sp FSL H7 689 | - | |
| *Pantoea* sp. SL1 M5 | - | |
| *Pantoea* 36041 Branch | - | |
| *Pantoea agglomerans* strain 4 | - | |
| *Pantoea agglomerans* strain 4 | - | |
| *Pantoea agglomerans* strain LMAE 2 | - | |
| *Pantoea agglomerans* Tx10 | - | |
| *Pantoea* sp 36061 Branch | - | |
| *Pantoea* sp MBLJ3 | - | |
| *Pantoea* sp SL1 M5 | - | |
| *Paracoccus* sp PAMC 22219 | - | |
| *Patulibacter minatonensis* DSM 18081 | DSM 18081 | WFCC |
| *Pectobacterium carotovorum* subsp *carotovorum* strain 28625 Branch | - | |
| *Pediococcus ethanolidurans* | - | |
| *Pediococcus pentosaceus* ATCC 33314 | - | |
| *Pedobacter* 611 Branch | - | |
| *Pedobacter agri* PB92 | - | |
| *Pedobacter borealis* DSM 19626 | DSM 19626 | WFCC |
| *Pedobacter kyungheensis* strain KACC 16221 | - | |
| *Pedobacter* sp R20 19 | - | |
| *Periglandula ipomoeae* | - | |
| *Planomicrobium glaciei* CHR43 | - | |
| *Propionibacterium acnes* | - | |
| *Propionibacterium* 20955 Branch | - | |
| *Propionibacterium acnes* 21065 Branch | - | |
| *Pseudomonas fluorescens* | - | |
| *Pseudomonas* sp. DSM 29167 | - | |
| *Pseudomonas* sp. Leaf15 | - | |
| *Pseudomonas syringae* | - | |
| *Pseudomonas* 39524 Branch | - | |
| *Pseudomonas* 39642 Branch | - | |
| *Pseudomonas* 39733 Branch | - | |
| *Pseudomonas* 39744 Branch | - | |
| *Pseudomonas* 39791 Branch | - | |
| *Pseudomonas* 39821 Branch | - | |
| *Pseudomonas* 39834 Branch | - | |
| *Pseudomonas* 39875 Branch | - | |
| *Pseudomonas* 39880 Branch | - | |
| *Pseudomonas* 39889 Branch | - | |
| *Pseudomonas* 39894 Branch | - | |
| *Pseudomonas* 39913 Branch | - | |
| *Pseudomonas* 39931 Branch | - | |
| *Pseudomonas* 39942 Branch | - | |
| *Pseudomonas* 39979 Branch | - | |
| *Pseudomonas* 39996 Branch | - | |
| *Pseudomonas* 40058 Branch | - | |
| *Pseudomonas* 40185 Branch | - | |
| *Pseudomonas abietaniphila* strain KF717 | - | |
| *Pseudomonas chlororaphis* strain EA105 | - | |
| *Pseudomonas cremoricolorata* DSM 17059 | DSM 17059 | WFCC |
| *Pseudomonas entomophila* L48 | - | |
| *Pseudomonas extremaustralis* 14 3 substr 14 3b | - | |
| *Pseudomonas fluorescens* BBc6R8 | - | |
| *Pseudomonas fluorescens* BS2 | ATCC 12633 | ATCC |
| *Pseudomonas fluorescens* EGD AQ6 | - | |
| *Pseudomonas fluorescens* strain AU 39831 Branch | - | |
| *Pseudomonas fluorescens* strain AU10973 | - | |
| *Pseudomonas fluorescens* strain AU14440 | - | |
| *Pseudomonas fragi* B25 | NCTC 10689 | NCTC |
| *Pseudomonas frederiksbergensis* strain SI8 | - | |
| *Pseudomonas fulva* strain MEJ086 | - | |
| *Pseudomonas fuscovaginae* 39768 Branch | - | |
| *Pseudomonas gingeri* NCPPB 3146 | NCPPB 3146 | NCPPB |
| *Pseudomonas lutea* | - | |
| *Pseudomonas luteola* XLDN4 9 | - | |
| *Pseudomonas mandelii* JR 1 | - | |
| *Pseudomonas moraviensis* R28 S | - | |
| *Pseudomonas mosselii* SJ10 | - | |
| *Pseudomonas plecoglossicida* NB 39639 Branch | - | |
| *Pseudomonas poae* RE*1 1 14 | - | |
| *Pseudomonas pseudoalcaligenes* AD6 | - | |
| *Pseudomonas psychrophila* HA 4 | - | |
| *Pseudomonas putida* DOT T1E | - | |
| *Pseudomonas putida* strain KF703 | - | |
| *Pseudomonas putida* strain MC4 5222 | - | |
| *Pseudomonas rhizosphaerae* | - | |
| *Pseudomonas rhodesiae* strain FF9 | - | |
| *Pseudomonas* sp 39813 Branch | - | |
| *Pseudomonas simiae* strain 2 36 | - | |
| *Pseudomonas simiae* strain MEB105 | - | |
| *Pseudomonas* sp 11 12A | - | |
| *Pseudomonas* sp 2 922010 | - | |
| *Pseudomonas* sp CF149 | - | |
| *Pseudomonas* sp Eur1 9 41 | - | |
| *Pseudomonas* sp LAMO17WK12 I2 | - | |
| *Pseudomonas* sp PAMC 25886 | - | |
| *Pseudomonas* sp PTA1 | - | |
| *Pseudomonas* sp R62 | - | |
| *Pseudomonas* sp WCS374 | - | |
| *Pseudomonas synxantha* BG33R | - | |
| *Pseudomonas synxantha* BG33R | - | |
| *Pseudomonas syringae* 39550 Branch | - | |
| *Pseudomonas syringae* 39596 Branch | - | |
| *Pseudomonas syringae* 40123 Branch | - | |
| *Pseudomonas syringae* CC 39499 Branch | - | |
| *Pseudomonas syringae* pv panici str LMG 2367 | - | |
| *Pseudomonas syringae* strain mixed | - | |
| *Pseudomonas tolaasii* 39796 Branch | - | |
| *Pseudomonas tolaasii* PMS117 | - | |
| *Pseudomonas veronii* 1YdBTEX2 | - | |
| *Pseudomonas viridiflava CC1582* | - | |
| *Pseudomonas viridiflava* strain LMCA8 | - | |
| *Pseudomonas viridiflava* TA043 | - | |
| *Pseudomonas viridiflava* UASWS0038 | - | |
| *Rahnella* 35969 Branch | - | |
| *Rahnella* 35970 Branch | - | |
| *Rahnella* 35971 Branch | - | |
| *Rahnella aquatilis* HX2 | - | |
| *Rahnella* sp WP5 | - | |
| *Raoultella ornithinolytica* | - | |
| *Rhizobiales* Order 22324 Branch | - | |
| *Rhizobium* sp YR528 | - | |
| *Rhodococcus fascians* A76 | - | |
| *Rhodococcus* sp BS 15 | - | |
| *Saccharomyces cerevisiae* | DSM 10542 | WFCC |
| *Sanguibacter keddieii* DSM 10542 | - | |
| *Serratia fonticola* AU 35657 Branch | - | |
| *Serratia fonticola* AU AP2C | - | |
| *Serratia liquefaciens* ATCC 27592 | ATCC 27592 | ATCC |
| *Serratia* sp H 35589 Branch | - | |
| *Shewanella* 37294 Branch | - | |
| *Shewanella baltica* 37301 Branch | - | |
| *Shewanella baltica* 37315 Branch | - | |
| *Shewanella baltica* OS 37308 Branch | - | |
| *Shewanella baltica* OS 37312 Branch | - | |
| *Shewanella baltica* OS185 | - | |
| *Shewanella baltica* OS223 | - | |
| *Shewanella baltica* OS678 | - | |
| *Shewanella oneidensis* MR 1 | - | |
| *Shewanella putrefaciens* HRCR 6 | - | |
| *Shewanella* sp W3 18 1 | - | |
| *Sphingobacterium* sp ML3W | - | |
| *Sphingobium japonicum* BiD32 | - | |
| *Sphingobium xenophagum* 24443 Branch | - | |
| *Sphingomonas echinoides* ATCC 14820 | ATCC 14820 | ATCC |
| *Sphingomonas parapaucimobilis* NBRC 15100 | ATCC 51231 | ATCC |
| *Sphingomonas paucimobilis* NBRC 13935 | ATCC 29837 | ATCC |
| *Sphingomonas phyllosphaerae* 5 2 | - | |
| *Sphingomonas* sp 23777 Branch | - | |
| *Sphingomonas* sp STIS6 2 | - | |
| *Staphylococcus* 6317 Branch | - | |
| *Staphylococcus equorum* UMC CNS 924 | - | |
| *Staphylococcus* sp 6275 Branch | - | |
| *Staphylococcus* sp 6240 Branch | - | |
| *Staphylococcus* sp OJ82 | - | |
| *Staphylococcus xylosus* strain LSR 02N | - | |
| *Stenotrophomonas* 14028 Branch | - | |
| *Stenotrophomonas* 42816 Branch | - | |
| *Stenotrophomonas maltophilia* 42817 Branch | - | |
| *Stenotrophomonas maltophilia* PML168 | - | |
| *Stenotrophomonas maltophilia* strain ZBG7B | - | |
| *Stenotrophomonas rhizophila* | - | |
| *Stenotrophomonas* sp RIT309 | - | |
| *Streptococcus gallolyticus* subsp *gallolyticus* TX20005 | - | |
| *Streptococcus infantarius* subsp *infantarius* 2242 Branch | - | |
| *Streptococcus infantarius* subsp *infantarius* | ATCC BAA 102 | ATCC |
| *Streptococcus macedonicus* ACA DC 198 | ATCC BAA-249 | ATCC |
| *Streptomyces olindensis* | - | |
| *Variovorax paradoxus* 110B | - | |
| *Variovorax paradoxus* ZNC0006 | - | |
| *Variovorax* sp CF313 | - | |
| *Vibrio fluvialis* 44473 Branch | - | |
| *Xanthomonas campestris* 37936 Branch | - | |
| *Xanthomonas campestris* pv *raphani 756C* | - | |

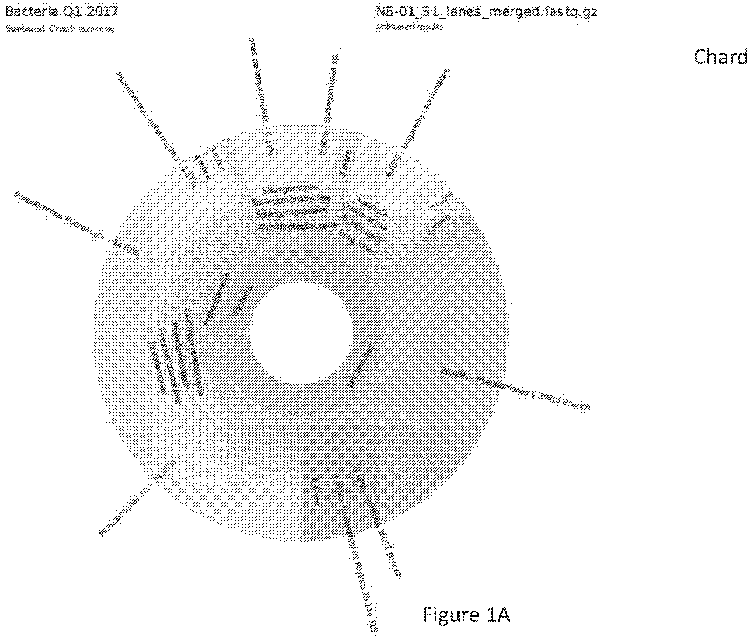

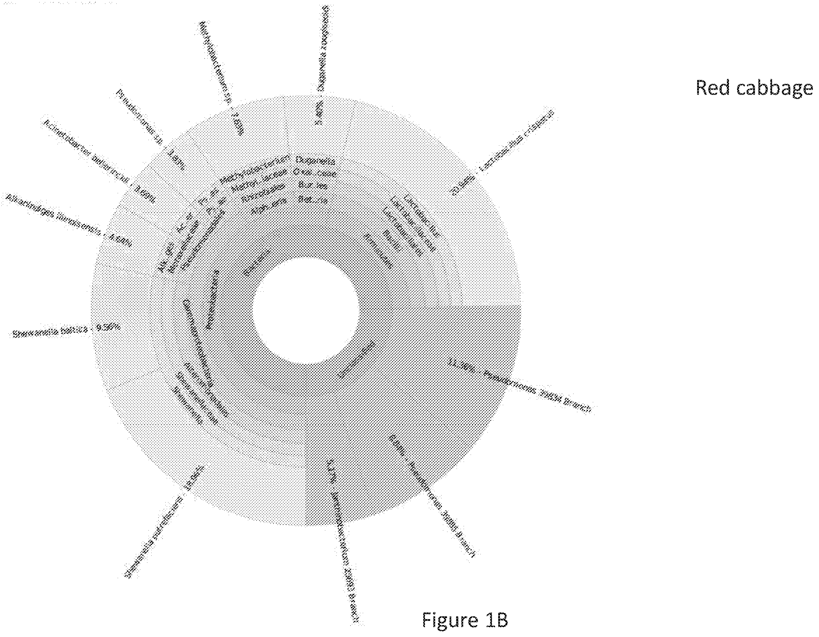

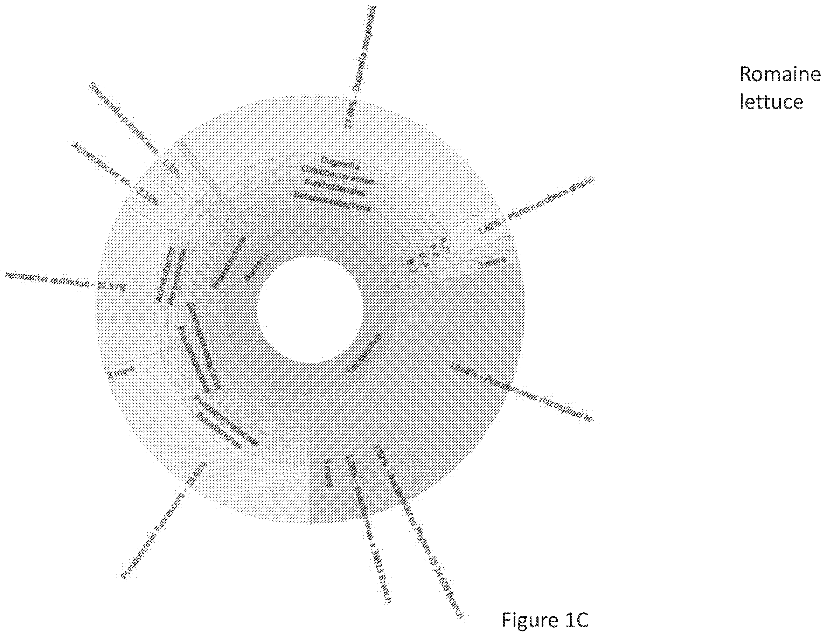

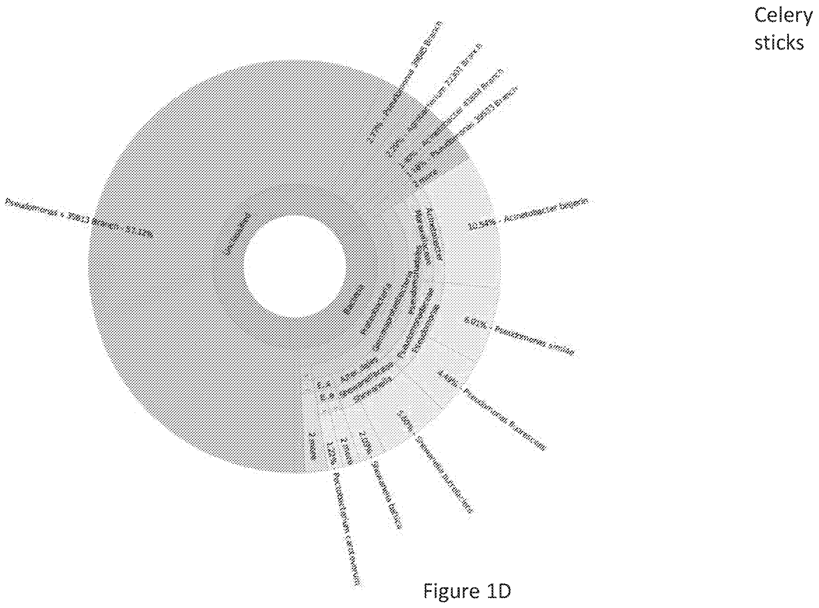

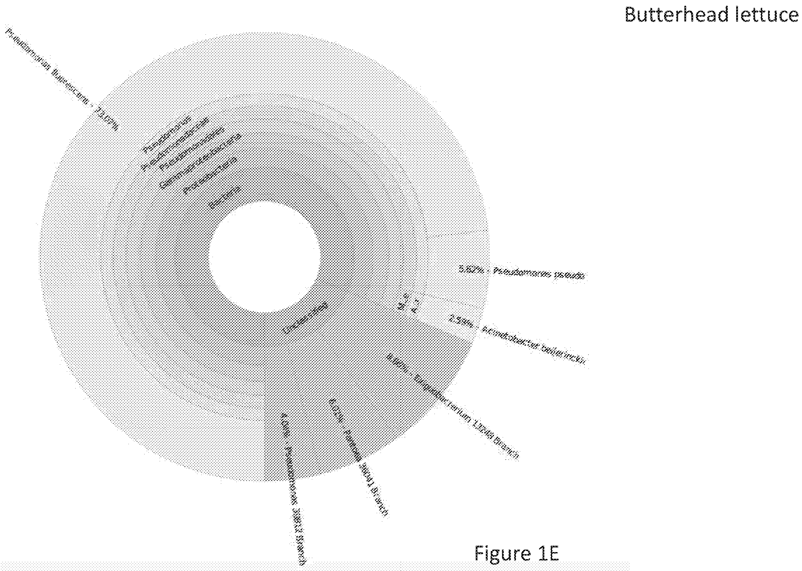

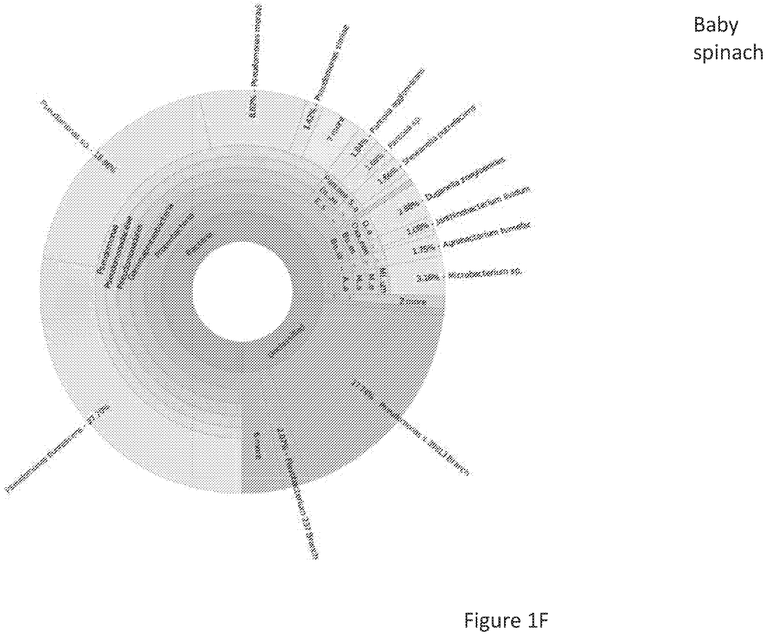

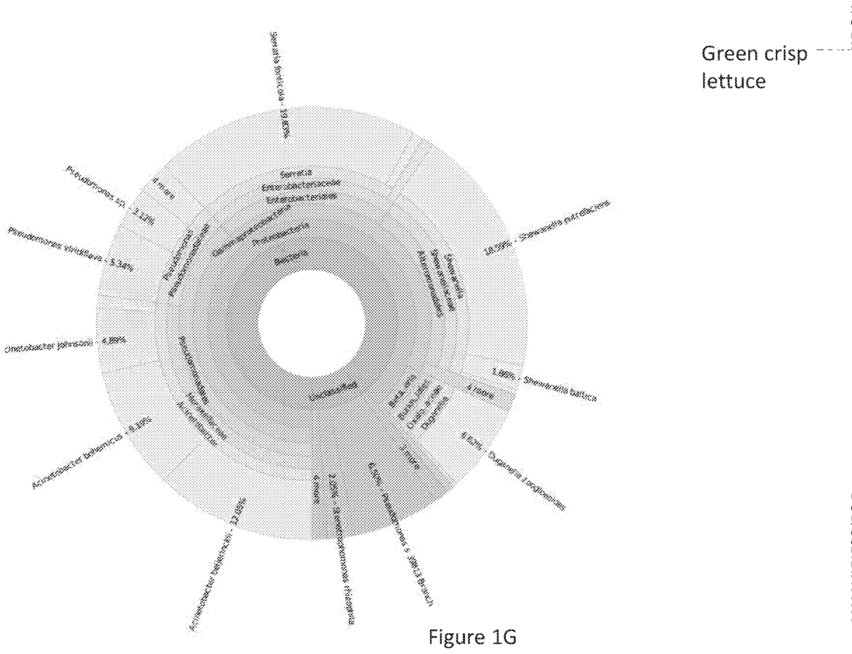

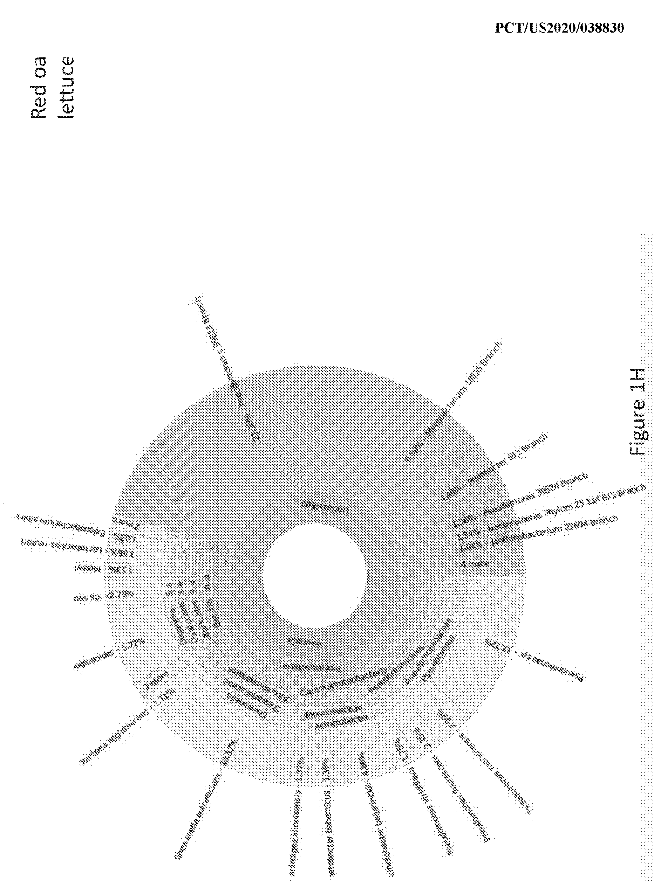

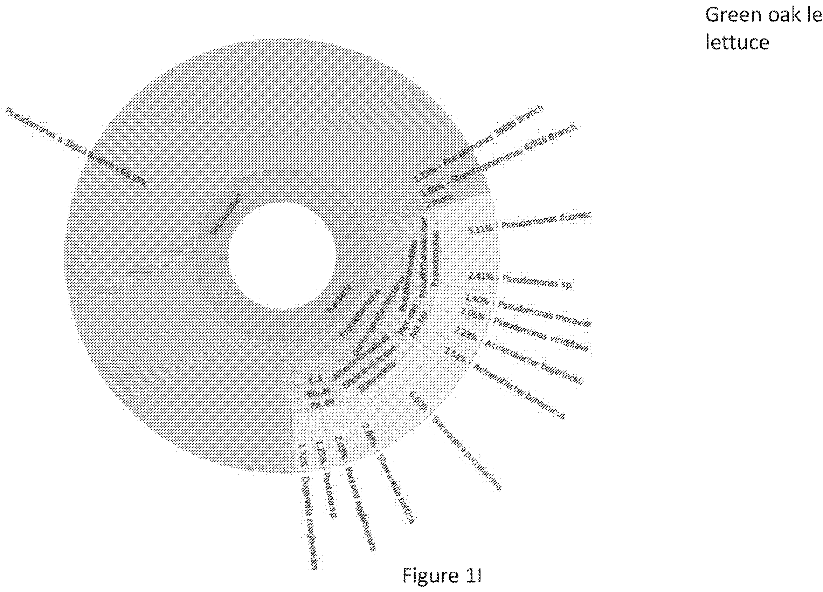

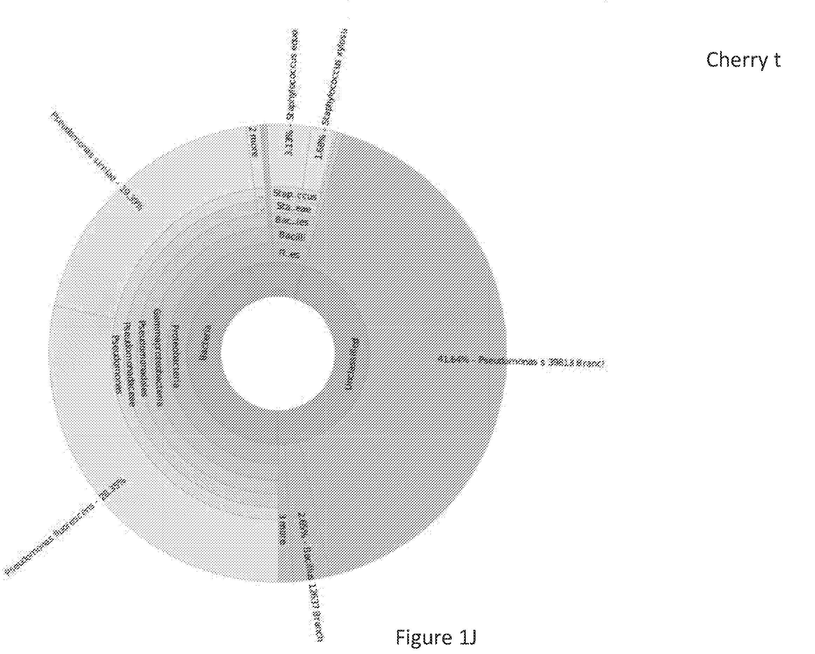

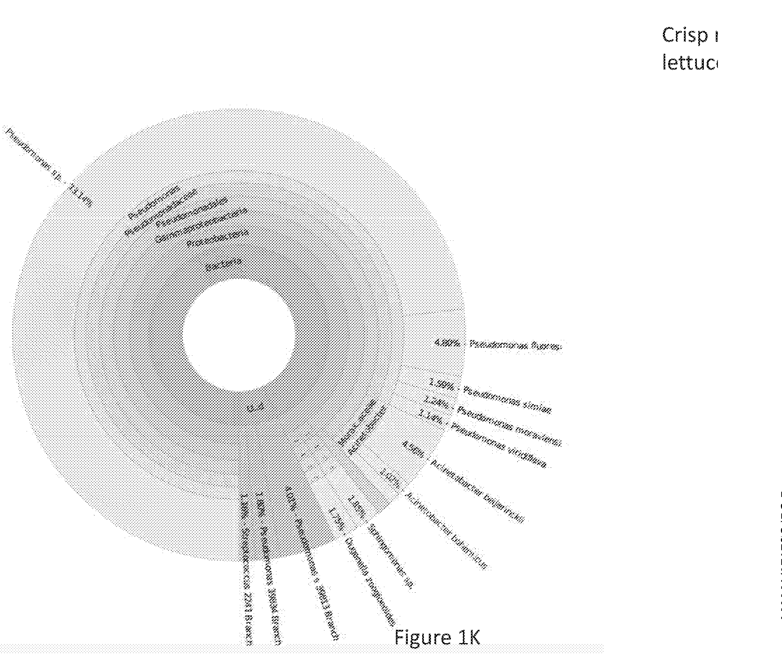

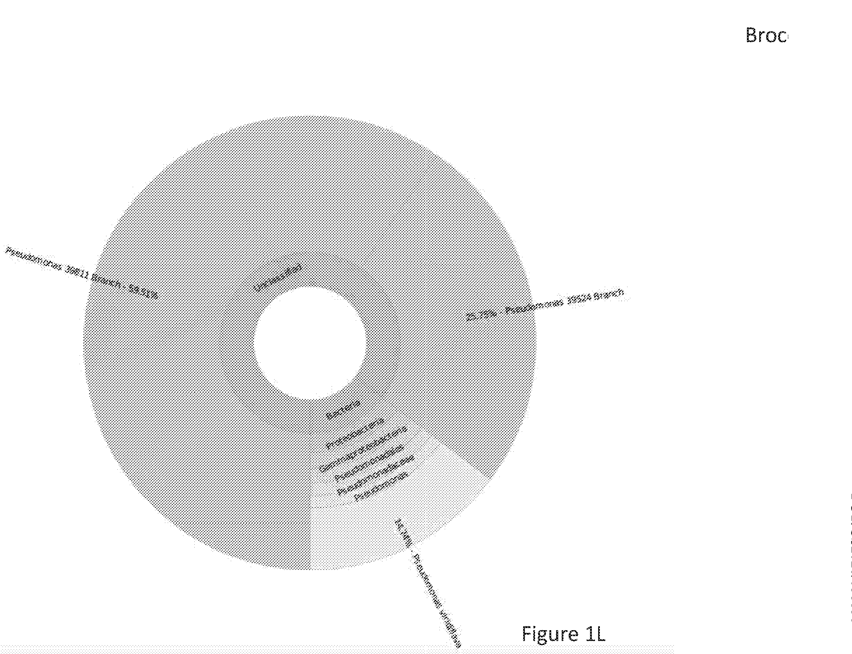

Figure 1 shows bacterial diversity observed in a set of 12 plant-derived samples as seen by a community reconstruction based on mapping the reads from a shotgun sequencing library into the full genomes of a database containing 36,000 genomes by the k-mer method (CosmosID). The display corresponds to a sunburst plot constructed with the relative abundance for each corresponding genome identified and their taxonomic classification. The genomes identified as unclassified have not been curated in the database with taxonomic identifiers and therefore not assigned to a group. This does not represent novel taxa and it is an artifact of the database updating process.

More specifically, Figure 1A shows bacterial diversity observed in a green chard. The dominant group is gamma proteobacteria with different *Pseudomonas* species. The members of the group "unclassified" are largely gamma proteobacteria not included in the hierarchical classification as an artifact of the database annotation.

Figure 1B shows bacterial diversity in red cabbage. There is a large abundance of *Lactobacillus* in the sample followed by a variety of *Pseudomonas* and *Shewanella.*

Figure 1C shows bacterial diversity in romaine lettuce. *Pseudomonas* and *Duganella* are the dominant groups. A member of the *Bacteroidetes* was also identified.

Figure 1D shows bacterial diversity in celery sticks. This sample was dominated by a *Pseudomonas* species that was not annotated yet into the database and therefore appeared as "unclassified" same for *Agrobacterium* and *Acinetobacter.*

Figure 1E shows bacterial diversity observed in butterhead lettuce grown hydroponically. The sample contains relatively low bacterial complexity dominated by *Pseudomonas fluorescens and other groups.* Also, there is a 9% abundance of *Exiguobacterium.*

Figure 1F shows bacterial diversity in organic baby spinach. The samples were triple-washed before distribution at the point of sale and therefore it is expected that must of the bacteria detected here are endophytes. Multiple *Pseudomonas* species observed in this sample including *P. fluorescens* and other shown as "unclassified."

Figure 1G shows bacterial diversity in green crisp gem lettuce. This variety of lettuce showed clear dominance of gamma proteobacteria and with *Pseudomonas, Shewanella, Serratia* as well as other groups such as *Duganella.*

Figure 1H shows bacterial diversity in red oak leaf lettuce. There is a relative high diversity represented in this sample with members of *Lactobacillus, Microbacterium, Bacteroidetes, Exiguobacterium* and a variety of *Pseudomonas.*

Figure 1I shows bacterial diversity in green oak leaf lettuce. It is dominated by a single *Pseudomonas* species including *fluorescens* and mostly gamma proteobacteria.

Figure 1J shows bacterial diversity in cherry tomatoes. It is dominated by 3 species of *Pseudomonas* comprising more than 85% of the total diversity on which *P. fluorescens* comprises 28% of bacterial diversity.

Figure 1K shows bacterial diversity in crisp red gem lettuce. Dominance by a single *Pseudomonas* species covering 73% of the bacterial diversity, on which *P. fluorescens* comprises 5% of bacterial diversity.

Figure 1L shows bacterial diversity in broccoli juice. The sample is absolutely dominated by 3 varieties of *Pseudomonas.*

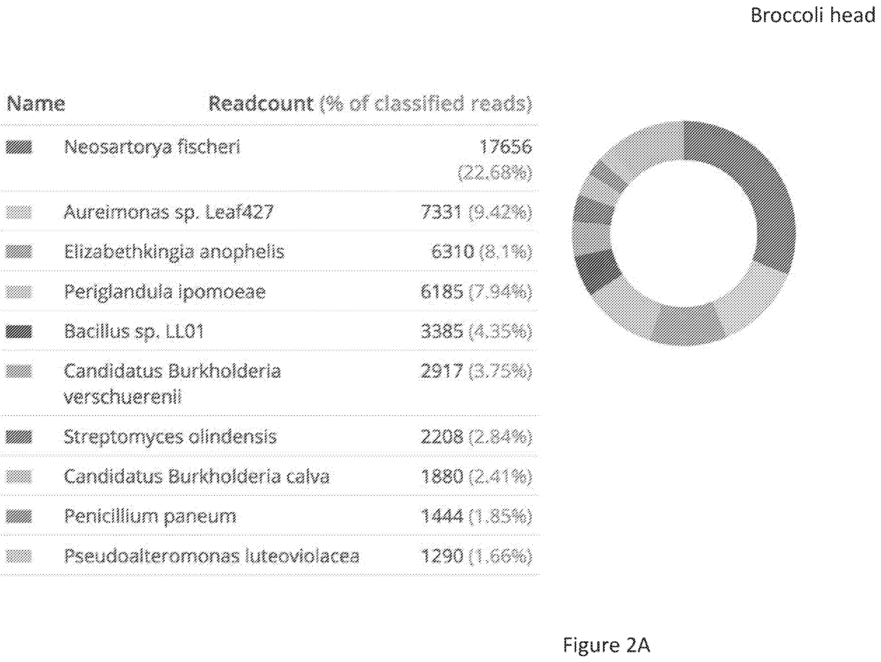

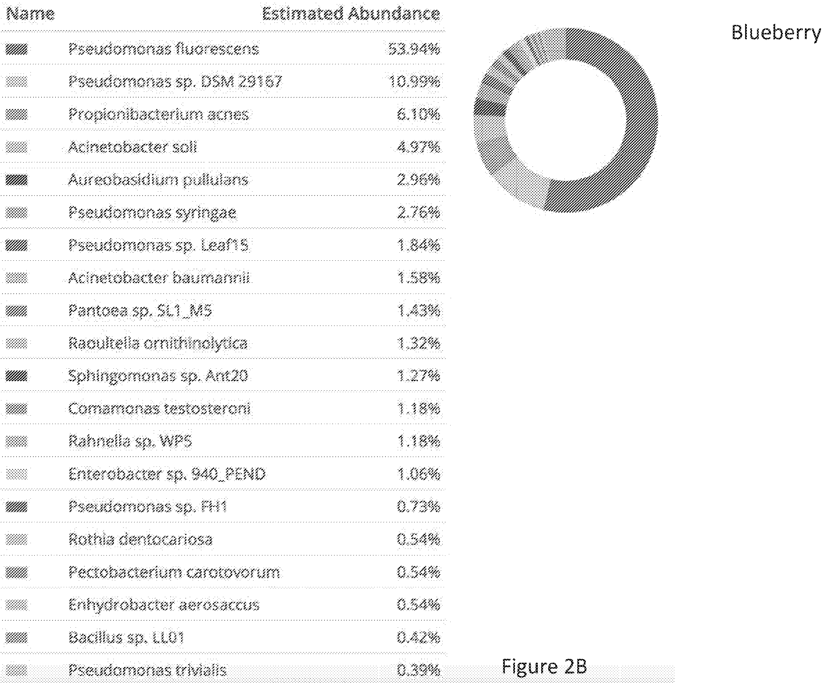

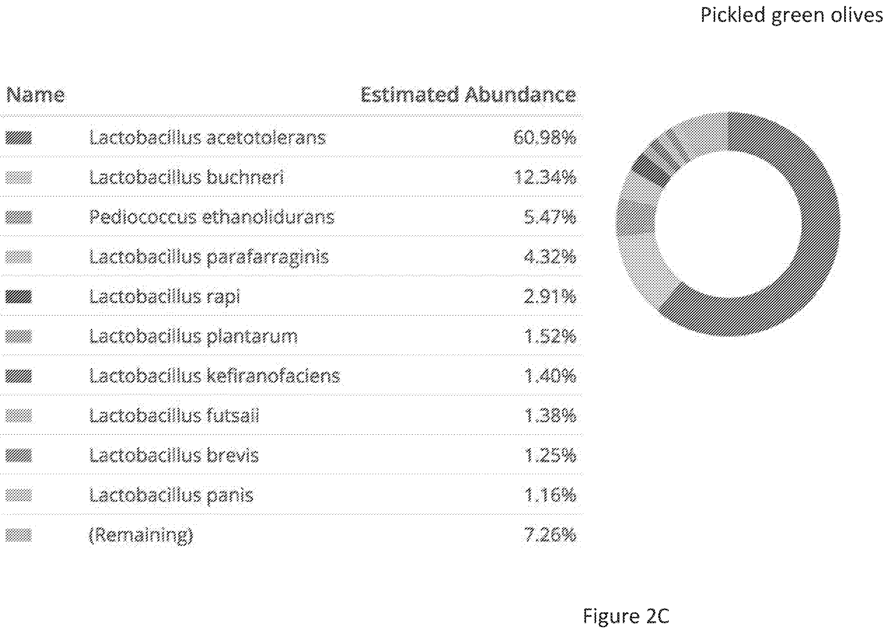

Figure 2 shows taxonomic composition of blueberries, pickled olives and broccoli head. More specifically, Figure 2A shows taxonomic composition of broccoli head showing a diversity of fungi and bacteria distinct from the broccoli juice dominated by few *Pseudomonas* species.

Figure 2B shows taxonomic composition of blueberries including seeds and pericarp (peel) as seen by shotgun sequencing showing dominance of *Pseudomonas* and strains isolated and sequenced.

Figure 2C shows taxonomic composition of pickled olives showing a variety of lactic acid bacteria present and dominant. Some of the species are recognized as probiotics.

### Example 2: In silico modeling outputs for different assemblages and DMA formulation.

To generate *in silico* predictions for the effect of different microbial assemblages with a human host a genome-wide metabolic analysis was performed with formulated microbial communities selected from the Agora collection (Magnusdottir et al. (2016) Generation of genome-scale metabolic reconstructions for 773 members of the human gut microbiota. Nat. Biotech. 35, 81-89) and augmented with the genomes of bacterial members detected in the present survey. These simulations predicted the "fermentative power" of each assemblage when simulated under different nutritional regimes including relatively high carbon availability (carbon replete) or carbon limited conditions when using plant fibers such as inulin, oligofructose and others as carbon source.

### Example 2.1 Metabolites in samples.

The method used for DNA sequencing the sample-associated microbiomes enabled to search for genes detected in the different vegetables related to propionate, butyrate, acetate and bile salt metabolism. This was done by mapping the reads obtained in the samples to reference genes selected for their intermediate role in the synthesis or degradation of these metabolites. There were organisms present in some of the 18 analyzed samples that matched the target pathways indicating their metabolic potential to produce desirable metabolites. Table 5 shows metabolites in samples.

**Table 5 - Metabolites in samples**

| **NAME OF ENZYME** | **ASSOCIATED METABOLITE** | **GENE SYMBOL** | **PATHWAY** | **E.C. NUMBER** | **COMMENTS** |
|---|---|---|---|---|---|
| ACETOLACTATE SYNTHASE I | (S)-2-ACETOLACTATE | | BUTANOATE METABOLISM | 2.2.1.6 | BUTYRATE PRODUCTION |
| ACETATE KINASE | PROPIONATE | ACKA | PROPANOATE METABOLISM | 2.7.2.1 | PROPIONATE |
| ACETYL-COA SYNTHETASE | PROPIONATE | AACS | PROPANOATE METABOLISM | 6.2.1.1 | PROPIONATE |
| ACETYL-COA HYDROLASE | ACETATE | | PYRUVATE METABOLISM | 3.1.2.1 | ACETATE |
| BILE SALT TRANSPORTER | BILE SALTS | ACR3 | BILE SALT TRANSPORT | | BILE SALT TOLERANCE |

### Example 2.2 Combined probiotic assemblages formulation

Microbes in nature interact with multiple other groups and form consortia that work in synergy exchanging metabolic products and substrates resulting in thermodynamically favorable reactions as compared to their individual metabolism. For example, in the human colon, the process for plant fiber depolymerization, digestion and fermentation into butyrate is achieved by multiple metabolic groups working in concert. This metabolic synergy is reproduced in the combined probiotic assemblages concept where strains are selected to be arrayed based on their ability to synergize to produce an increased amount of SCFA when grown together in the presence of substrates such as plant fibers. Defined Microbial Assemblage (DMA) is a combination of microorganisms that includes bacteria, fungi and prebiotic plant fibers. The consortium can exhibit some synergistic features but can be optimized by including additional strains.

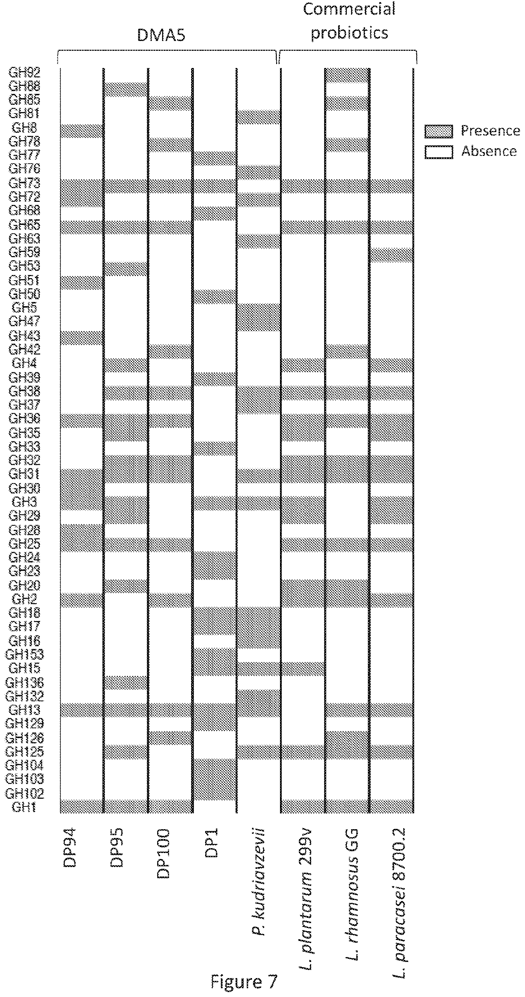

To illustrate this process, a set of 40 bacterial and fungal strains were isolated from food sources and their genomes were sequenced. The assembled and annotated genomes were then used to formulate *in silico* assemblages considering the human host as one of the metabolic members. Assuming a diet composed of lipids, different carbohydrates and proteins, the metabolic fluxes were predicted using an unconstrained model comparing the individual strain production of acetate, propionate and butyrate and compared to the metabolic fluxes with the assemblage. In addition to the genes related to short chain fatty acid metabolism the strains selected for *in vivo* testing were defined microbial assemblage 5 (DMA5) (DMA5 comprised: DP1 *(Pseudomonas sp.),* DP94 *(Lactobacillus brevis),* DP93 *(Leuconostoc mesenteroides),* DP100 *(Lactobacillus plantarum),* and DP102 (*Pichia krudriavzevii*) were analyzed for their genes related to plant fiber breakdown (glycosyl hydrolases) and compared to those found in reference probiotic strains (*Lactobacillus plantarum* 299V, *rahmnosus* LGG, and *paracasei* 8700.2) (Figure7). Whole-genome sequencing of microbial isolates from DMA5 was performed using an Illumina MiSeq instrument and a MiSEQ reagent v2 kit for 500 cycles (2 x 250 bp paired end run). Raw reads were initially trimmed at both 5' and 3' ends based on a PHRED score cutoff of 20 using SolexaQA++ (Cox et al. 2010). Reads shorter than 50 bp after trimming were discarded. Quality-filtered reads were *de novo* assembled using IDBA-UD with pre-corrections (Peng et al. 2010). Assembled contigs from the strains and genome sequences from commercial probiotics were used to predict CAZymes at the whole-genome level using run-dbcan v2.0.6 (Zhang et al. 2018) and the dbCAN CAZyme database (Yin et al. 2012). Reference probiotic microorganisms include *L. plantarum* 299v, *L. rhamnosus* GG, and *L. paracasei 8700.2.* Overall, GH102, GH103, GH104, GH129, GH132, GH136, GH153, GH16, GH17, GH18, GH23, GH24, GH28, GH30, GH33, GH37, GH39, GH43, GH47, GH5, GH50, GH51, GH53, GH63, GH68, GH76, GH77, GH8, GH81, and GH88 were present in DMA5 and absent in the genome of reference probiotic microorganisms (Figure 7).

It was observed that several gene families necessary to break down a variety of plant cell wall polymers were present in DP1 (*Pseudomonas sp*) and DP102 (*Pichia krudriavzevii*) that were missing in the reference probiotics (*Lactobacillus plantarum* 299V, *rahmnosus* LGG, and *paracasei* 8700.2), leading to enhanced functionality of the assemblage as a whole compared to the reference probiotics alone (Figure 7).

### Computational metabolic modeling

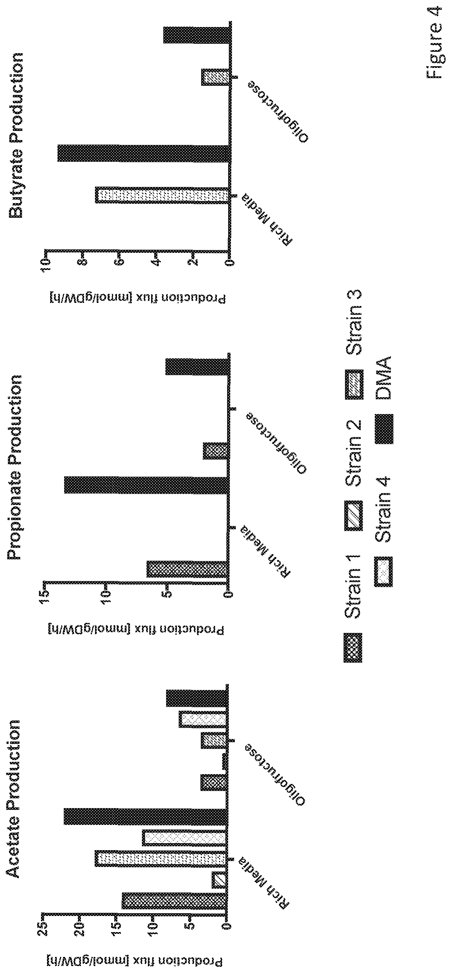

In a first model, 4 strains were combined into a combined probiotic assemblages. Metabolic predictions are shown in Figure 4. Strains 1-4 were predicted to produce acetate as single cultures but the combination into a combined probiotic assemblage was predicted to increase the flux when modeled on replete media and decrease the flux when modeled on plant fibers. Strain 4 was predicted to utilize the fibers better than the other three strains to produce acetate. Strain 1 was the only member of the assemblage predicted to produce propionate and when modeled with the other three strains the predicted flux doubled in replete media and quadrupled in the fiber media illustrating the potential metabolic synergy from the assemblage. Strain 3 was the only member of the assemblage predicted to produce butyrate and when modeled with the other three strains the predicted flux increased slightly in replete media and doubled in the fiber media illustrating the potential metabolic synergy from the assemblage.

**Table 6. Strains from first DMA model.**

| | |
|---|---|
| Strain 1 - | DP6 *Bacillus cereus-like* |
| Strain 2 - | DP9 *Pediococcus pentosaceus-like* |
| Strain 3 - | *Clostridium butyricum* DSM 10702 |
| Strain 4 - | DP1 *Pseudomonas fluorescens-like* |

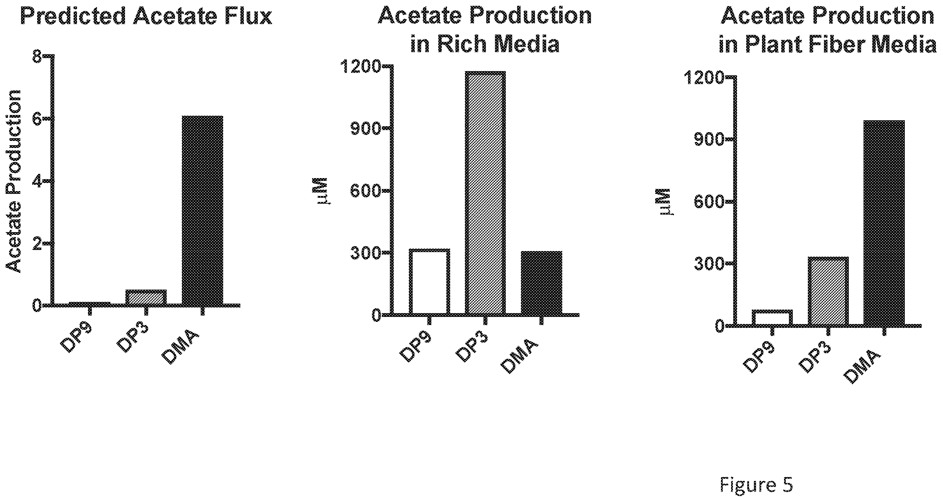

Substrate availability plays an important role in the establishment of synergistic interactions. Carbon limitation in presence of plant fibers favors fiber depolymerization and fermentation to produce SCFA. Conversely, carbon replete conditions can prevent the establishment of synergistic metabolism to degrade fibers as it is not favored thermodynamically when the energy available from simple sugars is available. To illustrate this, combined probiotic assemblages were formulated containing two strains of lactic acid bacteria and a metabolic prediction was run assuming a limited media with plant fibers. According to the model, the predicted flux of *Leuconostoc* was higher than *Pediococcus.* When combined into a probiotic assemblage, the flux increased five-fold compared to the single strains alone (Figure 5 left panel),. When tested in the lab and measured by gas chromatography, the acetate production increased 3 fold compared to the single strains when grown on media with plant fibers (Figure 5 right panel). However, when grown on carbon replete media with available simple sugars, acetate production was correspondingly higher compared to the plant fiber media but there was no benefit of synergistic acetate production when the two strains were grown together into a combined probiotic assemblages (Figure 5 middle panel).

In addition to acetate, propionate, and butyrate some strains produce other isomers. For example, strains DP1 related to *Pseudomonas fluorescens* and DP5 related to *Debaromyces hansenii* (yeast) produce isobutyrate when grown in carbon-replete media as single strains, however there is metabolic synergy when tested together as combined probiotic assemblages measured as an increase in the isobutyric acid production (data not shown).

### In vitro short chain fatty acid production

To demonstrate the predictive synergies with strains in a defined consortium, a combination of strains generally considered to be probiotic (DP94 *(Lactobacillus brevis),* DP93 *(Leuconostoc mesenteroides),* and DP100 (*Lactobacillus plantarum*)) were combined with the yeast DP64 *(Hanseniaspora uvarum)* and referred to as defined microbial assemblage 4 (DMA4), or combined with the probiotic-upgrading strains DP1 (*Pseudomonas fluorescens*) and DP102 (*Pichia kudriavzevii*) and referred to as DMA5 and tested for the ability to produce acetate from plant fiber-based media. Strains were cultured anaerobically, at 37°C, in a medium with prebiotic fibers. Each strain was inoculated with 1.00E+09 CFUs at a normalized volume. Each strain was cultured in a combination and alone. Culture supernatant was sampled 48 hours after inoculation and analyzed by gas chromatography to observe acetate production. After culturing the microbes, samples are centrifuged, and supernatants are acidified and SCFAs are extracted into ethyl acetate in preparation for detection of SCFAs using GC-FID. Standards were prepared by diluting a pre-mixed Fatty Acid Test Standard to create standards of 5000 µM, 1000 µM, and 200 µM.

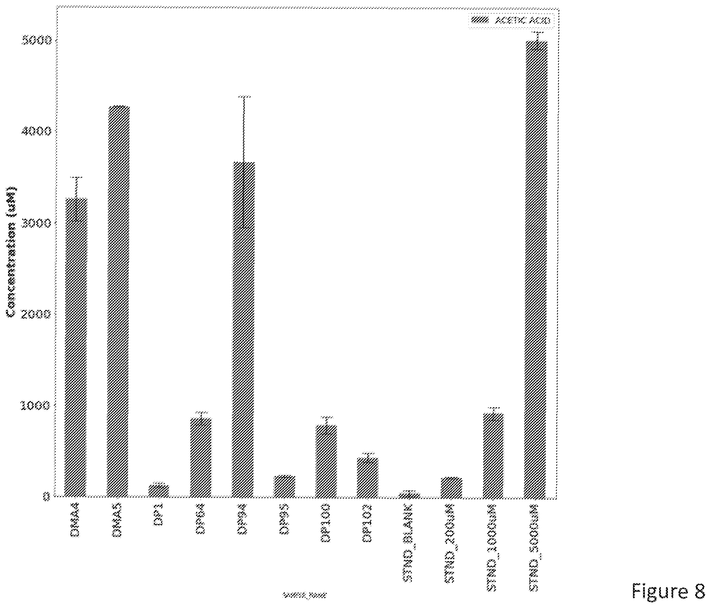

As shown in Figure 8, it was observed that there was a synergistic increase in acetate production by the addition of DP1 (*Pseudomonas sp*) and replacing DP64 *(Hanseniaspora uvarum)* by DP102 (*Pichia kudriavzevii*) to the set of probiotic strains. The addition of DP1 *(Pseudomonas sp.)* and DP102 (*Pichia kudriavzevii)* to the lactic acid bacteria strains increased overall acetate production as compared to lactic acid bacteria combinations alone or with the yeast DP64 (*Hanseniaspora uvarum*)*.* Thus, the function of probiotic microorganism was improved by adding new and novel microbes to the assemblage or generating new and novel assemblages of probiotics, leading to a synergistic production of short chain fatty acids.

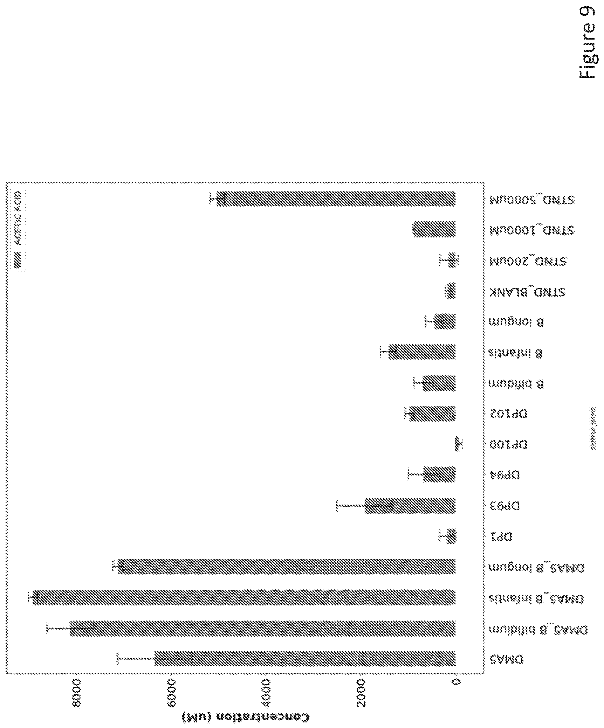

Another probiotic microorganism genus is *Bifidobacterium* with the species *bifidum, infantis* and *longum* consumed as commercial probiotics. The consortium DMA5 (DP1 *(Pseudomonas sp.),* DP94 *(Lactobacillus brevis),* DP93 *(Leuconostoc mesenteroides*), DP100 *(Lactobacillus plantarum*), and DP102 (*Pichia krudriavzevii*) was combined with each one of these Bifidobacteria species (*bifidum, infantis* and *longum*) and the short chain fatty acid production measured. As shown in Figure 9, a synergistic effect with each of the three strains was observed illustrating another example of enhancement of a known probiotic strain by a rational combination together with other helper strains identified herein.

The process of combined probiotic assemblages validation described herein and summarized in the following method are applied to find other candidates applicable to other products:
1. Define a suitable habitat where microbes present with the desirable attributes are abundant based on ecological hypotheses. For example, fresh vegetables are additional to have anti-inflammatory effects when consumed in a whole-food plant based diet, and therefore, it is likely they harbor microbes that can colonize the human gut.
2. Apply a selection filter to isolate and characterize only those microbes capable of a relevant gut function. For example, tolerate acid shock, bile salts and low oxygen.
3. Selected strains are then cultivated *in vitro* and their genomes sequenced at 100X coverage to assemble, annotate and use in predictive genome-wide metabolic models.
4. Metabolic fluxes are generated with unconstrained models that consider multiple strains and the human host to determine the synergistic effects from multiple strains when it is assumed they are co-cultured under a simulated substrate condition.
5. Predicted synergistic combinations are then tested in the laboratory for validation. Single strains are grown to produce a biomass and the spent growth media removed after reaching late log phase. The washed cells are then combined in combined probiotic assemblages with 2-10 different strains per combined probiotic assemblage and incubated using a culture media with plant fibers as substrates to produce short chain fatty acids to promote gut health.
6. The combined probiotic assemblages are then analyzed by gas chromatography to quantify the short chain fatty acid production where the synergistic effect produces an increased production in the combined assemblage as compared to the individual contributions.
7. The selected probiotic assemblages with the best SCFA production are tested in preclinical models, for example in ovariectomized mice (OVX) to recapitulate postmenopausal osteoporosis.

### Example 3. Gut simulation experiments

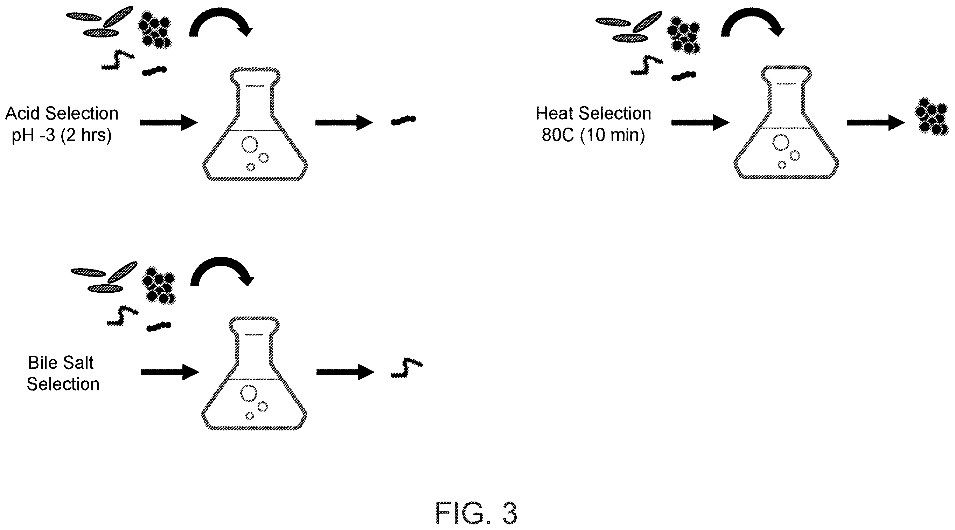

An *in vitro,* system that mimics various sections of the gastrointestinal tract was used to identify probiotic strains of interest. Isolates of interest were incubated in the presence of conditions that mimic particular stresses in the gastro-intestinal tract (such as low pH or bile salts), heat shock, or metformin. After incubation, surviving populations were recovered. Representative isolates identified by this methodology are shown in Table 7 and the design layout in Figure 3.

**Table 7: Strains resistant to gut stress, listed with heat shock tolerance, acid shock tolerance, and isolation temperature.**

| **Strain number** | **Heat shock** | **Isolation temp** | **Acid shock (pH 3) 2hr** | **Genus** | **species** |
|---|---|---|---|---|---|
| DP39 | No | 25 | No | *Agrobacterium* | *tumefaciens* |
| DP14 | No | 25 | Yes | *Arthrobacter* | *luteolus* |
| DP52 | No | 25 | No | *Arthrobacter* | *sp.* |
| DP28 | No | 25 | Yes | *Aureobasidium* | *pullulans* |
| DP4 | No | 25 | No | *Aureobasidium* | *pullulans* |
| DP10 | Yes | 25 | No | *Bacillus* | *velezensis* |
| DP13 | No | 25 | Yes | *Bacillus* | *mycoides* |
| DP48 | Yes | 25 | No | *Bacillus* | *paralicheniformis* |
| DP49 | Yes | 25 | No | *Bacillus* | *gibsonii* |
| DP55 | Yes | 25 | No | *Bacillus* | *megaterium* |
| DP57 | Yes | 25 | No | *Bacillus* | *mycoides* |
| DP6 | Yes | 25 | No | *Bacillus* | *cereus* |
| DP60 | Yes | 25 | No | *Bacillus* | *simplex* |
| DP65 | No | 25 | No | *Bacillus* | *sp.* |
| DP67 | Yes | 25 | No | *Bacillus* | *sp.* |
| DP68 | Yes | 25 | No | *Bacillus* | *atrophaeus* |
| DP69 | Yes | 25 | No | *Bacillus* | *sp.* |
| DP70 | No | 25 | No | *Bacillus* | *tequilensis* |
| DP72 | Yes | 25 | No | *Bacillus* | *sp.* |
| DP73 | Yes | 37 | No | *Bacillus* | *amyloliquefaciens* |
| DP74 | Yes | 25 | No | *Bacillus* | *coagulans* |
| DP75 | No | 37 | No | *Bacillus* | *valezensis* |
| DP81 | Yes | 37 | No | *Bacillus* | *sp.* |
| DP82 | Yes | 37 | No | *Bacillus* | *velezensis* |
| DP86 | No | 30 | No | *Bacillus* | *velezensis* |
| DP88 | No | 30 | No | *Bacillus* | *velezensis* |
| DP89 | No | 30 | No | *Bacillus* | *subtilis* |
| DP91 | No | 30 | No | *Bacillus* | *sp.* |
| DP92 | No | 30 | No | *Bacillus* | *tequilensis* |
| DP77 | Yes | 25 | No | *Bacillus* | *megaterium* |
| DP83 | Yes | 37 | No | *Bacillus* | *amyloliquefaciens* |
| DP21 | No | 25 | No | *Candida* | *santamariae* |
| DP41 | Yes | 37 | No | *Corynebacterium* | *mucifaciens* |
| DP47 | No | 25 | Yes | *Cronobacter* | *dublinensis* |
| DP16 | No | 25 | No | *Cryptococcus* | *laurentii* |
| DP15 | No | 25 | No | *Curtobacterium* | *sp.* |
| DP19 | No | 25 | No | *Curtobacterium* | *pusillum* |
| DP5 | No | 37 | No | *Debaromyces* | *hansenii* |
| DP50 | No | 25 | No | *Enterobacter* | *sp.* |
| DP85 | No | 30 | No | *Enterococcus* | *faecium* |
| DP23 | No | 25 | No | *Erwinia* | *billingiae* |
| DP33 | No | 25 | No | *Erwinia* | *persicinus* |
| DP62 | No | 25 | No | *Erwinia* | *sp.* |
| DP78 | No | 25 | No | *Erwinia* | *rhapontici* |
| DP24 | No | 25 | No | *Filobasidium* | *globisporum* |
| DP32 | No | 25 | No | *Hafnia* | *paralvei* |
| DP2 | No | 37 | No | *Hanseniaspora* | *occidentalis* |
| DP64 | No | 25 | No | *Hanseniaspora* | *uvarum* |
| DP66 | No | 25 | No | *Hanseniaspora* | *occidentalis* |
| DP8 | No | 25 | No | *Hanseniaspora* | *opuntiae* |
| DP44 | No | 25 | No | *Herbaspirillum* | *sp.* |
| DP43 | No | 25 | No | *Janthinobacterium* | *sp.* |
| DP58 | No | 25 | No | *Janthinobacterium* | *svalbardensis* |
| DP51 | No | 25 | No | *Klebsiella* | *aerogenes* |
| DP59 | No | 25 | No | *Kosakonia* | *cowanii* |
| DP100 | No | 30 | No | *Lactobacillus* | *plantarum* |
| DP90 | No | 30 | No | *Lactobacillus* | *plantarum* |
| DP94 | No | 30 | No | *Lactobacillus* | *brevis* |
| DP95 | No | 30 | No | *Lactobacillus* | *paracasei* |
| DP96 | No | 30 | No | *Lactobacillus* | *casei* |
| DP87 | No | 30 | No | *Lactobacillus* | *plantarum* |
| DP97 | No | 30 | No | *Lactococcus* | *garvieae* |
| DP98 | No | 30 | No | *Lactococcus* | *garvieae* |
| DP61 | No | 25 | No | *Lelliottia* | *sp.* |
| DP3 | No | 25 | No | *Leuconostoc* | *mesenteroides* |
| DP93 | No | 30 | No | *Leuconostoc* | *mesenteroides* |
| DP26 | No | 25 | No | *Methylobacterium* | *sp.* |
| DP54 | No | 25 | No | *Methylobacterium* | *adhaesivum* |
| DP80 | No | 25 | No | *Methylobacterium* | *adhaesivum* |
| DP12 | No | 25 | Yes | *Microbacterium* | *sp.* |
| DP30 | No | 25 | Yes | *Microbacterium* | *testaceum* |
| DP84 | No | 25 | No | *Microbacterium* | *sp.* |
| DP76 | No | 25 | No | *Ochrobactrum* | *sp.* |
| DP56 | Yes | 25 | No | *Paenibacillus* | *lautus* |
| DP35 | No | 25 | Yes | *Pantoea* | *ananatis* |
| DP36 | No | 25 | Yes | *Pantoea* | *vagans* |
| DP40 | No | 37 | No | *Pantoea* | *sp.* |
| DP46 | No | 25 | Yes | *Pantoea* | *agglomerans* |
| DP101 | No | 30 | No | *Pediococcus* | *pentosaceus* |
| DP9 | No | 25 | No | *Pediococcus* | *pentosaceus* |
| DP25 | No | 25 | No | *Penicillium* | *solitum* |
| DP102 | No | 30 | No | *Pichia* | *krudriavzevii* |
| DP7 | No | 25 | No | *Pichia* | *fermentans* |
| DP34 | No | 25 | Yes | *Plantibacter* | *flavus* |
| DP29 | No | 25 | Yes | *Pseudoclavibacter* | *helvolus* |
| DP1 | No | 25 | No | *Pseudomonas* | *fluorescens* |
| DP11 | No | 25 | No | *Pseudomonas* | *putida* |
| DP18 | No | 25 | No | *Pseudomonas* | *sp.* |
| DP37 | No | 25 | No | *Pseudomonas* | *rhodesiae* |
| DP42 | No | 37 | No | *Pseudomonas* | *lundensis* |
| DP53 | No | 25 | No | *Pseudomonas* | *fragi* |
| DP63 | No | 25 | Yes | *Pseudomonas* | *azotoformans* |
| DP79 | No | 25 | No | *Pseudomonas* | *fragi* |
| DP17 | No | 25 | No | *Rahnella* | *aquatilis* |
| DP22 | No | 25 | No | *Rahnella* | *sp.* |
| DP38 | No | 25 | No | *Rhodococcus* | *sp.* |
| DP71 | No | 25 | No | *Rhodosporidium* | *babjevae* |
| DP45 | No | 25 | No | *Sanguibacter* | *keddieii* |
| DP27 | No | 25 | No | *Sphingomonas* | *sp.* |
| DP31 | No | 25 | Yes | *Sporisorium* | *reilianum* |
| DP20 | No | 25 | No | *Stenotrophomonas* | *rhizophila* |
| DP99 | No | 30 | No | *Weissella* | *cibaria* |

### Example 4. Monitoring the effect of upgraded probiotic assemblages on microbial flora of a mammal

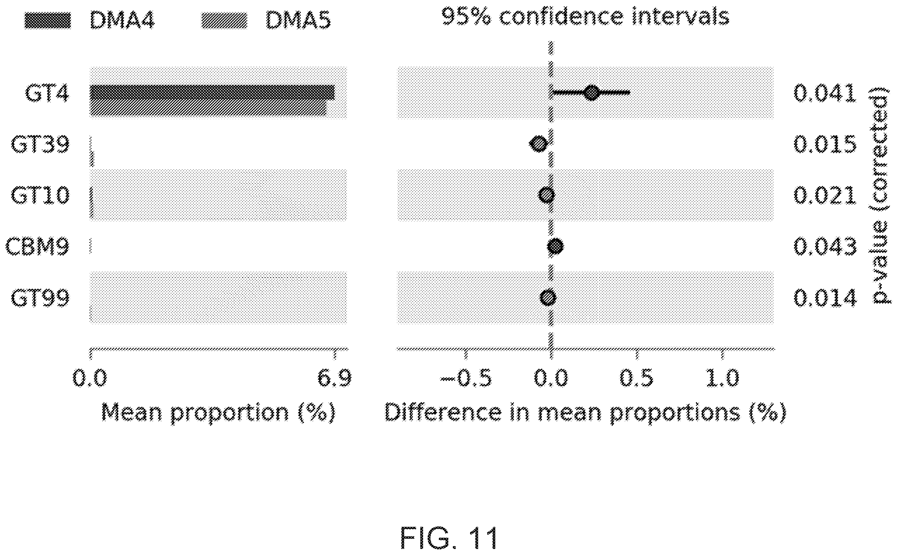

Alterations of the gut microbiota have been linked with changes in the host homeostasis such as metabolic diseases. In order to evaluate alterations in the gut microbiota composition in obese individuals, fecal samples were collected from ovariectomized mice (OXV) and the gut microbiota was characterized (Figures 11 - 14). Briefly, DNA was extracted using the Zymo Quick-DNA Fecal/Soil Microbe Kit and quantified using a Qubit 2.0 flurometer with the dsDNA HS assay kit. Metagenomic libraries were prepared using the Illumina Nextera XT DNA library prep kit and an equimolar mixture of the libraries was sequenced on an Illumina NextSeq instrument on a 2 x 150 bp paired end run. Raw reads from the sequencing run were analyzed using SolexaQA (Cox et al. 2010) for trimming and removing of Illumina adaptors using a Phred score cutoff of 20 and minimum fragment length of 50 bp. Taxonomic classification of the short-read metagenomes was determined using MetaPhlan2, which uses clade-specific marker genes from approximately 17,000 reference genomes to estimate the relative abundance of microbial members present in the sample (Troung et al. 2015).

### Example 4.1 Comparison of glycosyl hydrolase families identified in microbial strains from DMA4 and DMA5

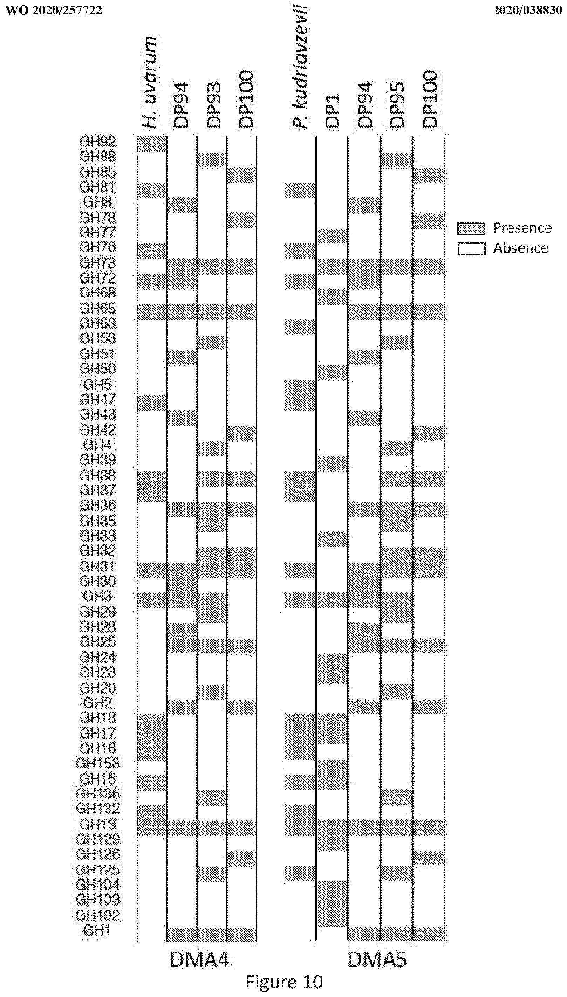

Whole-genome sequencing of microbial isolates from DMA4 and DMA5 was performed to further understand the functional capacity of these strains in breaking down complex carbohydrates from plants. The abundance of carbohydrate-active enzymes (CAZymes), a group of enzymes that are involved into the breakdown of plant cell walls with potential for further conversion to beneficial metabolites in the human intestinal tract was investigated. We focused on glycosyl hydrolases (GHs), which are enzymes that hydrolyze the glycosidic bond leading to the formation of carbohydrate hemiacetal. Assembled contigs were used as input data to predict CAZymes at the whole-genome level using run-dbcan v2.0.6 (Zhang et al. 2018) and the dbCAN CAZyme database (Yin et al. 2012). Overall, the GH families GH102, GH103, GH104, GH129, GH153, GH23, GH24, GH33, GH39, GH5, GH50, GH63, GH68, and GH77 were present in microbial strains from DMA5 and absent in the ones from DMA4 (Figure 10). The increased number of GH families in DMA5 provide a greater ability to utilize plant fibers and to metabolize a variety of carbohydrate polymers resulting in a more efficient conversion of these polymers into beneficial metabolites. One of the strains contributing to the unique GHs in DMA5 is DP1 *Pseudomonas fluorescens,* possibly in part due to its origin of isolation from edible plant tissues.

### Example 4.2 Differential abundance of CAZYmes families in mice stool at week 6 between OVX mice treated with DMA4 and DMA5.

Figure 11 shows the mean proportion differences and confidence intervals at 95% of CAZYmes families between DMA4 and DMA5 groups at week 6. The families with differential abundance are glycosyl transferases (GT) such as GT4 (e.g., 1,2-diacylglycerol 3-glucosyltransferase, diglucosyl diacylglycerol synthase, trehalose phosphorylase, NDP-Glc: α-glucose α-glucosyltransferase / α,α-trehalose synthase), GT39 (α-mannosyltransferase), GT10 (galactoside α-1,3/1,4-L-fucosyltransferase, galactoside α-1,3-L-fucosyltransferase, glycoprotein α-1,3-L-fucosyltransferase), CBM39 (β-1,3-glucan binding function), and GT99 (3-deoxy-β-D-manno-oct-2-ulosonic acid transferase). There are biological implications on these differences that, without wishing to be bound by theory, can explain the improved bone benefit on DMA5. For example, GT39 deficiency is a disorder in the attachment of the first mannose to dolichol-PP-GlcNAc2 at the outside of the ER resulting in dolicholglycan accumulation in fibroblasts. Serum transferrin isoelectrofocusing shows a type 1 pattern, and lipid-linked oligosaccharide analysis of fibroblasts an accumulation of GlcNAc2-PP-dolichol. The clinical picture is mainly characterized by severe psychomotor disability in humans among other symptoms.

### Example 4.3 Relative abundance values of L-rhamnose degradation pathway in the gut microbiota of OVX mice at week 6 treated with DMA4 and DMA5.

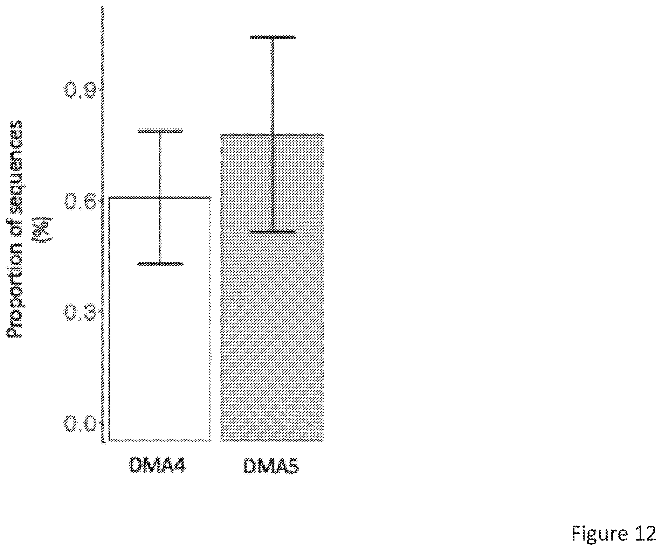

Rhamnose is a plant polymer that has been associated to bone health when there is an increased degradation. Conversely, in osteoporosis mice models there is an increase in rhamnose biosynthesis when there is a loss of bone mineral density. In order to compare the rhamnose degradation in stool from DMA4 vs. DMA5 groups at week 6, relative abundances of genes related to L-rhamnose degradation pathway in individual mouse were calculated by mapping of sequencing reads against UniRef90 using HuMANN2 and characterizing gene families (Franzosa et al. 2018). The data shows the average per group and the standard deviation of the relative abundance estimated in the metagenomic samples. The bar plot also indicated an increase of L-rhamnose degradation pathway in the DMA5 group (Figure 12). Accordingly, the results demonstrate an increased relative amount of genes related to rhamnose degradation in the group treated with DMA5 than in DMA4 that can be associated to the benefit in bone mineral density.

### Example 4.4 Differential abundant metabolic pathways in stool of OVX mice at week 6 between DMA4 and DMA5 treatments.

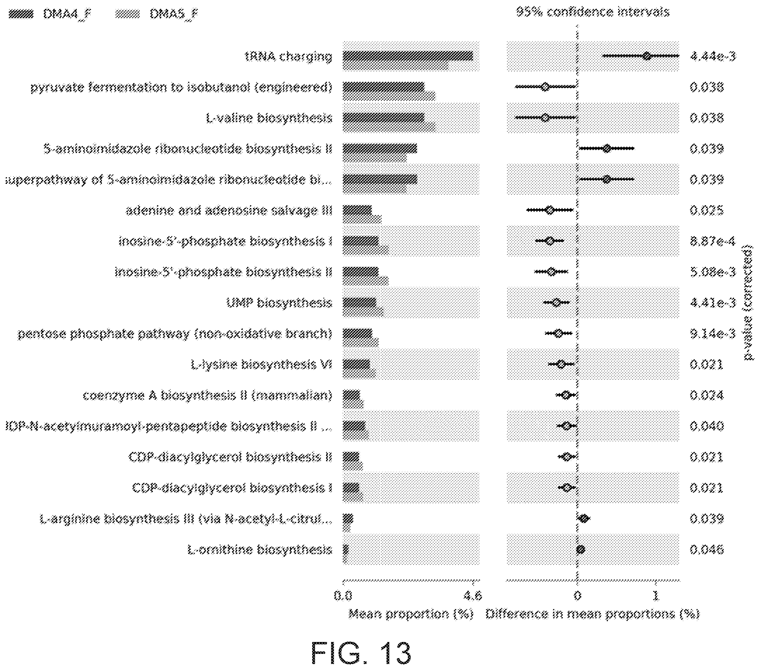

The shotgun sequencing approach allowed to compare functional genes and pathways between experimental treatments. Metabolic pathways from the gut microbial community were identified using HuMANN2 (Franzosa et al. 2018) and the UniRef90 database. The plot shows the mean proportion differences and confidence intervals at 95% of metabolic pathways between DMA4 and DMA5 groups at week 6. Some pathways were depleted in DMA5 with respect to DMA4 indicating a potential beneficial effect. For example, L-arginine biosynthesis III is part of a pathway that has been seen enriched in OVX mice compared to sham and therefore making this a signature for the loss of bone mineral density. Other pathways were enriched in DMA5 compared to DMA4. Among them, pyruvate fermentation to isobutanol, CDP-diacylglycerol biosynthesis, and L-lysine biosynthesis VI and L-valine biosynthesis was seen (Figure 13). The differences revealed additional anabolic capacity and a connection to bone health. For example, CDP-diacylglycerol is a key intermediate in the synthesis of phosphatidylinositol (PI) and cardiolipin (CL), and both PI and CL have highly specialized roles in cells.

### Example 4.5 Comparison of enzymes involved in vitamin biosynthesis identified in microbial strains from DMA4 and DMA5.

Cobalamin (vitamin B12) is an essential cofactor for humans synthetized by some bacteria and archaea and it has been involved in mediating microbe-microbe interactions and host-microbe communication (Fang et al. 2017). Cobalamin deficiency can result in anemia, nervous system disease, and gastrointestinal and psychiatric disorders (Briani et al. 2013, Isaac et al. 2015). Folate has been previously identified to show anti-inflammatory effects and mediate DNA methylation process (Kok et al. 2018). Folate deficiency has been associated to increased risk of development postmenopausal breast cancer (Sellers et al. 2001) and colorectal cancer (Terry et al. 2002). Gene pathways associated with folate production have been identified in commercial probiotic strains including Lb. plantarum WCFS1 (Laiño et al.2014, Li et al.2016) and *Bifidobacterium adolescentis* (D'Aimmo et al.2012, Sugahara et al.2015). Accordingly, each of these vitamins was explored.

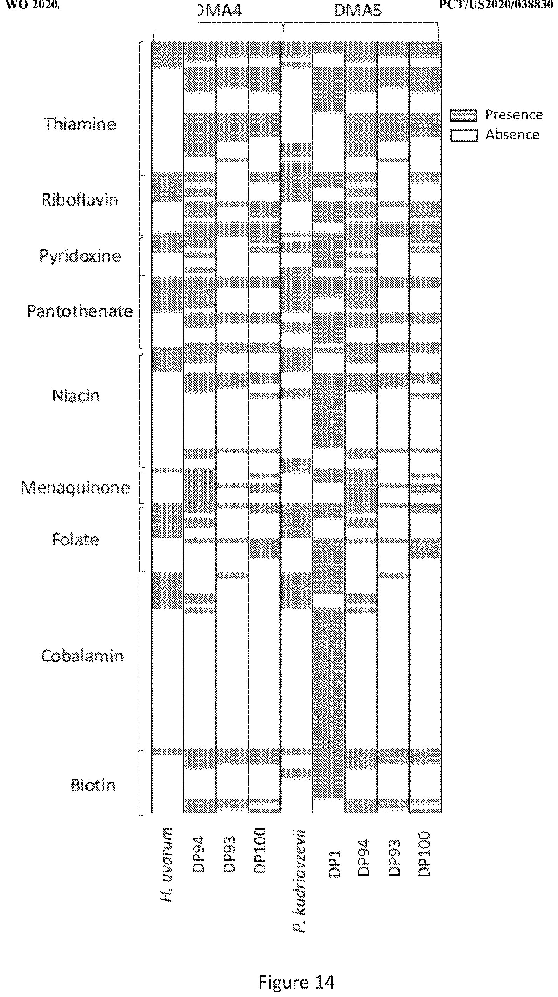

Sequencing reads from the strain genomes were analyzed using FMAP (Kim et al. 2016) in order to predict KEGG Orthologies (KOs) in their genomes. The list of KOs for each metabolic pathway was previously identified in human fecal metagenomes (Dan et al. 2019). As shown in Figure 14, the heatmap demonstrates the presence (grey color) and absence (white color) of KEGG Orthologies (KOs) that are involved in each vitamin pathway. Overall, microbial genomes from DMA5 demonstrated a higher number of enzymes involved in vitamin biosynthesis including Cobalamin, Niacin, Biotin, Pantothenate, Thiamine, and Folate.

### Example 5: Identification of Upgraded Probiotic Assemblages

Additional probiotic microorganisms were combined with strains described herein, allowing synergy in the production of SCA, production of other bone anabolic molecules such as vitamin K2, modification of estrogen-like compounds from the diet leading to improved bone health, or promoting an increase calcium absorption along the gastrointestinal tract. The additional probiotic microorganisms were formulated into a probiotic upgraded assemblage composed of 4 modules: i) a variety of plant derived fibers, ii) a microbe digesting the fiber to produce acetate, iii) a microbe taking up acetate to produce butyrate, iv) a microbe that can produce a bone health promoting molecule such as vitamin K2. There are instances where microbes may belong to more than one module, including the commercial probiotic strain that is being upgraded.

Using whole genome sequencing, a large database of microbial genomes that can be mined for various functional capacities has been generated. For example, microbes with the ability to break down dietary fiber (prebiotics), produce SCFA from fiber breakdown products, or vitamin K2 can be identified as lead candidates to upgrade additional probiotic microorganisms.

After identifying strains with potential to synergize with additional probiotic microorganism in SCFA production as well as strains with the capacity to produce vitamin K2, modify estrogen-like compounds, metabolic models were used to predict SCFA production *in silico.* These predictions allowed for the screening of many combinations of microbes to be tested *in vitro* for synergy, where the SCFA production combined is greater than the sum of the individual components. Strains from Table 4 or Table 7 were nominated both by the ability to produce SCFA, and either produce vitamin K2 or modify estrogen-like compounds.

Candidate probiotic assemblages were then tested *in vitro* for growth compatibility by streaking one culture onto an agar plate with other members of the DMA and assessing for zones of inhibition. Probiotic assemblages were then tested for by growing the microbes alone or together in equal proportions for 24-48 hours and measuring their SCFA production using a gas chromatographer (GC) equipped with a flame ionization detector (GC-FID) and comparing to the retention times of purified SCFA standards. If the SCFA production of the probiotic assemblage is greater than the sum of the parts, that assemblage was a candidate to be tested *in vivo.* The assemblages were then screened for vitamin K2 production by analyzing their genomes for the presence of the menaquinone-7 pathway. Strains in the assemblage survival and activity in the host GI tract were tested *in vitro* by exposing the cells to an acidic shock at pH 3 for 2 hours to simulate the passage through the stomach, followed by exposure to bile salts in the growth medium and incubation under low oxygen or anoxic conditions using an anaerobic chamber or Hungate tubes with anoxic headspace. Population dynamics in each assemblage were measured by agar plating CFUs in selective media.

The above analysis resulted in the generation of DMA4 and DMA5 below:
DMA4: DP64 (*Hanseniaspora uvarum*), DP94 *(Lactobacillus brevis),* DP93 (*Leuconostoc mesenteroides*), and DP100 *(Lactobacillus plantarum).*
DMA 5: DP1 *(Pseudomonas sp.),* DP94 *(Lactobacillus brevis),* DP93 (*Leuconostoc mesenteroides*), DP100 *(Lactobacillus plantarum),* and DP102 (*Pichia krudriavzevii*).

Once an assemblage is formed based on SCFA synergy between the additional probiotic strain and strains from Table 4 or Table 7, as well as vitamin K2 production, they are tested for the ability to increase TGF-beta in colonic cell lines such as Caco-2 cells. A TGF-beta rich environment promotes Treg production in the colon, so if a probiotic assemblage can promote TGF-beta in colonic cell lines, they will likely promote Treg production in an animal model. Additionally, the assemblages are evaluated based on their ability to decrease inflammation in colonic cell lines. Here, Caco-2 cells are treated with an inflammatory mediator such as TNF either alone or in the presence of supernatant from the assemblage. Decreasing colonic inflammation leads to improved overall health. The assemblages that can both create a TGF-beta rich environment as well as decrease inflammation are good candidates to test in an animal model of osteoporosis to determine if they can prevent steroid depletion induced bone loss.

### Example 6. Improving Bone Health using Upgraded Probiotic Assemblage treatment

**Experimental Design:** Lead candidate defined microbial assemblages (DMAs) were evaluated for their therapeutic efficacy in an ovariectomized (OVX) mouse model of postmenopausal osteoporosis. All mice were group housed with 5 mice per cage in individually ventilated cages (IVCs) specifically designed for germ free husbandry [59, 60]. At 12-weeks of age, mice were weighed, had baseline feces collected, and underwent OVX (N=30) or sham (N=10) surgery to deplete estrogen levels and commence the bone resorption process as previously described [61]. Prior to surgery, mice received a DXA scan to evaluate baseline BMD. 1- day post-surgery, mice began a daily oral gavage regimen (200 µL) of water (negative control), DMA4, or DMA5 and continued for 6-weeks. Bi-weekly fecal samples were collected to monitor the composition of the gut microbiome over time. Finally, on the last day of the study, mice received a second DXA scan to evaluate BMD, followed by euthanasia.

DMA4 comprised: DP64 *(Hanseniaspora uvarum),* DP94 *(Lactobacillus brevis),* DP93 *(Leuconostoc mesenteroides),* and DP100 *(Lactobacillus plantarum).*
DMA 5 comprised: DP1 *(Pseudomonas sp.),* DP94 *(Lactobacillus brevis),* DP93 *(Leuconostoc mesenteroides),* DP100 *(Lactobacillus plantarum),* and DP102 (*Pichia krudriavzevii*).

*Tissue collection and analysis:* At the time of sacrifice, the uterus is removed and weighed to confirm that the ovaries were successfully removed, and estrogen was depleted following OVX surgery. Serum is collected to analyze bone turnover markers including C-terminal telopeptide of collagen (CTX), a marker of resorption, and osteocalcin, a marker of formation, as previously described [5], as well as inflammatory cytokines and osteoclastogenic markers including TNF, IL-1, IFNγ, IL-17, and RANKL. Serum CTX and osteocalcin are analyzed by rodent specific ELISA (immunodiagnostic systems) while inflammatory markers are analyzed by multiplex assay at EveTechnologies (Calgary, AB). Tissues are then collected from each mouse to evaluate the impact of our therapeutic intervention on intestinal barrier integrity and inflammation, as well as bone quantity and strength. Colonic tissues is isolated and flash frozen for RNA and protein evaluation of tight junctions and claudins, critical mediators of gut barrier integrity, as well as inflammatory cytokine levels including TNF, IL-17, IFNγ and IL-1, markers of Th-17 and macrophage mediated inflammation [5, 58]. Cecal contents are removed and flash frozen for downstream metagenomic sequencing and SCFA analysis by GC-FID to determine how our DMA impacted the composition and function of the gut microbiome. Finally, both femurs and the lumbar spine are removed and processed skeletal analysis. Here, distal femurs and vertebra are analyzed by MicroCT and histomorphometry to observe changes in cortical and trabecular morphometry and cellularity. Additionally, one femur from each mouse is subjected to biomechanical testing including 3-point bending to determine the impact of probiotic assemblage therapy on skeletal strength.

*Metagenomic Analysis:* Comprehensive functional analysis of the fecal microbiome identifies functions enriched in response to probiotic assemblage gavage. Shotgun sequencing libraries of fecal DNA are created using Illumina Nextera Flex and sequenced on an Illumina HiSeqX followed by standard metagenomic pipelines.

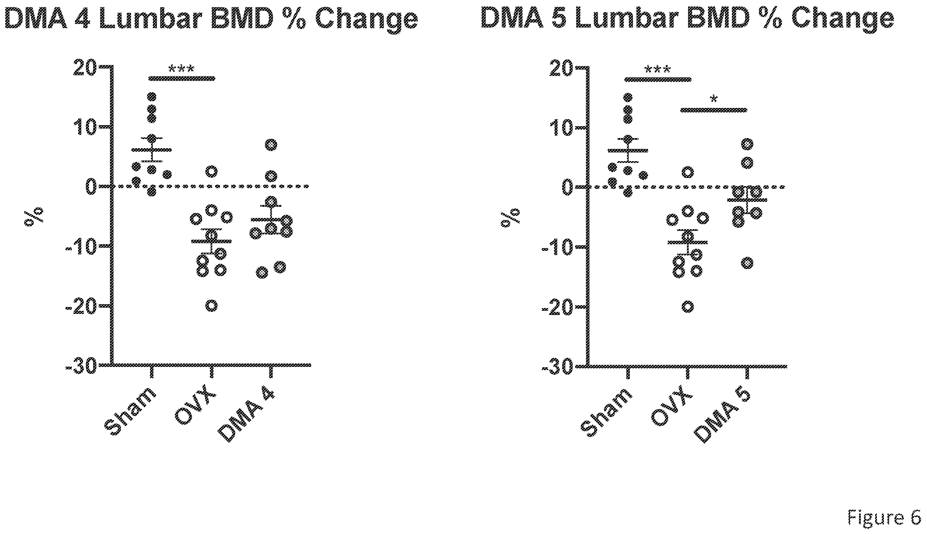

DXA analysis of the lumbar spine revealed a significant decrease in bone mineral density (BMD) in OVX mice compared to control 6 weeks after surgery (Figure 6). OVX mice treated with DMA 5 were significantly protected from the ovx induced bone loss as observed in Figure 6B, where DMA4 provided no protection against loss of BMD following OVX surgery (Figure 6A). The results indicate that the combination of microbes in DMA5 offers superior protection against steroid depletion induced bone loss than the combination of microbes in DMA4.

### Example 7. Improving Metabolism/weight loss with Upgraded Probiotic Assemblage treatment

Using whole genome sequencing, a large database of microbial genomes that can be mined for various functional capacities has been generated. For example, microbes with the ability to break down dietary fiber (prebiotics), produce SCFA from fiber breakdown products, or ClpB as lead candidates to upgrade additional probiotic commercial probiotics can be identified.

After identifying strains with potential to synergize with additional probiotic strains in SCFA production as well as strains with the capacity to produce ClpB, metabolic models are used to measure SCFA production *in silico.* These predictions allow for screening of many combinations of microbes to be tested *in vitro* for synergy, where the SCFA production combined is greater than the sum of the individual components. Strains are nominated both by the ability to produce SCFA and produce ClpB.

Candidate assemblages are tested as described in the previous example.

Once an assemblage is nominated based on SCFA synergy between the additional probiotic strain and strain or strains from table 4 or table 7, they are tested for the ability to decrease inflammation in colonic cell lines. Here, Caco-2 cells are treated with an inflammatory mediator such as TNF either alone or in the presence of supernatant from the probiotic assemblage. Decreasing colonic inflammation leads to improved overall health. Additionally, the probiotic assemblages are tested for their ability to improve barrier function in Caco-2 cells by analyzing tight and gap junction gene expression and protein levels after treating with probiotic assemblage supernatant. It has been shown that improving barrier function can decrease systemic inflammation that leads to improved metabolic parameters. Finally, the probiotic assemblages are evaluated based on their capacity to induce glucagon-like peptide 1 (GLP-1) production. GLP-1 is an incretin that leads to decreased blood glucose levels by enhancing insulin secretion. SCFAs like butyrate have been shown to promote GLP-1 production in the colon. The probiotic assemblages that can decrease inflammation, improve gut barrier function, and increase GLP-1 production are the good candidates to test in an animal model of osteoporosis to determine whether they can induce weight loss, prevent weight gain, decrease adiposity, and/or improve metabolic parameters.

Our lead candidate probiotic assemblages are evaluated for their therapeutic efficacy in a diet induced obesity (DIO) model of type 2 diabetes. Male (DIO) and low-fat diet control C57BL/6J mice are purchased from the Jackson Laboratories (Jax) at 16 weeks of age and were singly housed in individually ventilated cages (IVCs) (Allentown Inc) in a room with a 12-hour light/dark schedule. At Jax, mice are placed on either a low-fat diet (10% kcal, D12450B) or high-fat diet (60% kcal, D12492) (Open Source Diets; Research Diets Inc.) at 5-weeks of age and remained on those respective diets for the duration of the experiment. Mice are allowed to acclimate for 2-weeks prior to the experimental commencement. At 18-weeks of age, test articles are provided to the mice via oral gavage. Control groups are provided sterile water at a dose of 5mL/kg body weight. Upgraded probiotic assemblages are provided at a dose of 5x10⁹ CFUs/dose. Mice are gavaged with test articles daily for 6-weeks.

*Oral glucose* tolerance *test (OGTT).* After 5 weeks of dosing mice with test article, an OGTT is performed. Here, mice are fasted for 6 hours after which fasting blood glucose levels are measured via tail vein blood using a glucometer (One-Touch Ultra II). Mice are then dosed with an oral glucose bolus (2g/kg) via oral gavage, and blood glucose is measured at 20, 40, 60, and 120 minutes post gavage.

*Insulin Tolerance Test (ITT).* 6 weeks after the first dose of test material, mice are fasted for 4-hours and a baseline blood glucose level measurement are recorded using a glucometer (One-Touch Ultra II). Following baseline measurements, mice receive an intraperitoneal (IP) injection of insulin (10mL/kg at a concentration of 0.1U/mL). After injection, blood glucose is measured at 15, 30, 60, 90, and 120 minutes via tail vein blood.

*Body composition.* Body fat percentage and lean mass are determined using Dual Energy x-ray Absorptiometry (DEXA) scan (PIXImus2 Mouse Densitometer; GE) 12 weeks after DMM surgery. Prior to DEXA scans, mice are anesthetized via intraperitoneal injection of ketamine (60 mg/kg) and xylazine (4 mg/kg).

### Example 8. Using Upgraded Probiotic Assemblages to reduce chronic inflammation

Aging is associated with increased inflammation that has a systemic negative impact on the body. Many additional probiotic strains have been associated decreasing inflammation and the most commonly used microbes are various species of *Bifidobacterium, Lactobacillus, and Bacillus* including *Bifidobacterium bifidum, Bifidobacterium pseudolongum, Bifidobacterium longum, Bifidobacterium adolescentis, Bifidobacterium animalis, Bifidobacterium breve, Lactobacillus reuteri, Lactobacillus rhamnosus GG, Lactobacillus paracasei, Lactobacillus plantarum, Bacillus clausii,* and *Bacillus subtilis.* All of these strains have been shown to decrease inflammation through the generation of short chain fatty acids (SCFA) including acetate, propionate, and butyrate.

We have the ability to upgrade these additional probiotic microbes with strains isolated from edible plants with the capacity to synergize in the production of SCFA. The additional probiotic strains are upgraded into an upgraded probiotic assemblage composed of 3 modules: i) a variety of plant derived fibers, ii) a microbe digesting the fiber to produce acetate, iii) a microbe taking up acetate to produce butyrate. There are instances where microbes may belong to more than one module, including the additional probiotic strain that is being upgraded.

Using whole genome sequencing, a large database of microbial genomes that can be mined for various functional capacities has been generated. For example, microbes with the ability to break down dietary fiber (prebiotics) and produce SCFA from fiber breakdown products can be identified.

After identifying strains with potential to synergize with additional probiotic strains in SCFA production, metabolic models can be used to measure SCFA production *in silico.* These predictions allow screening of many combinations of microbes to be tested *in vitro* for synergy, where the SCFA production combined is greater than the sum of the individual components. Strains from table 4 or table 7 collection are nominated by the ability to synergize in the production of SCFA.

Candidate upgraded probiotic assemblages are nominated as described in the above examples.

Once an upgraded probiotic assemblage is assembled based on SCFA synergy between the additional probiotic strain and strains from table 4 or table 7, they are tested for the ability to decrease inflammation in colonic cell lines. Here, Caco-2 cells are treated with an inflammatory mediator such as TNF either alone or in the presence of supernatant from the upgraded probiotic assemblage. Decreasing colonic inflammation leads to improved overall health. Additionally, the upgraded probiotic assemblages are tested for their ability to improve barrier function in Caco-2 cells by analyzing tight and gap junction gene expression and protein levels after treating with upgraded probiotic assemblage supernatant. It has been shown that improving barrier function can decrease systemic inflammation.

Our lead candidate upgraded probiotic assemblage are evaluated for their therapeutic efficacy in a mouse model of aging. Briefly, 12-month old male and female C57BL/6J mice arrive at the facility, at which point they undergo a 2-week acclimation period. All mice are group housed with 5 mice per cage in individually ventilated cages (IVCs) specifically designed for germ free husbandry [59, 60]. At 12.5-months of age, mice are weighed, have baseline feces collected, and have blood collected to evaluate a panel of inflammatory cytokines. Mice then begin a daily oral gavage regimen (200 µL) of water (negative control), or a upgraded probiotic assemblagecomposition at a concentration of about 5x10⁹ CFUs/dose and continue for 2-months. Monthly fecal samples are collected to monitor the composition of the gut microbiome over time. At the end of the experiment, blood samples are again collected to evaluate the change in inflammatory markers that are indicative of health.

### Example 9. Upgraded Probiotic Assemblage Improves the Efficacy of a Type 2 Diabetes Drug When Used in Combination

The upgraded probiotic assemblages described herein can be used in combination with FDA approved type 2 diabetes drugs including but not limited to, metformin, Acarbose, Miglitol, Voglibose, Sitagliptin, Saxagliptin, Liraglutide, Pioglitazone, dipeptidyl peptidase-4 (DPP4)-inhibitors, glucagon-like peptide-1 (GLP-1) receptor analogs, alpha glucosidase inhibitors, thiazolidinedione, and sodium/glucose cotransporter 2 (SGLT2) inhibitors, to improve the efficacy and/or reduce the side effects of said drug.

To test the combination of the upgraded probiotic assemblages with anti-type 2 diabetes drugs, male diet induced obese (DIO) and low-fat diet control C57BL/6J mice are purchased from the Jackson Laboratories (Jax) at 16 weeks of age and are singly housed in individually ventilated cages (IVCs) in a room with a 12-hour light/dark schedule. At Jax, mice are placed on either a low-fat diet (10% kcal, D12450B) or high-fat diet (60% kcal, D12492) (Open Source Diets; Research Diets Inc.) at 5-weeks of age and remain on those respective diets for the duration of the experiment. Mice are allowed to acclimate for 2-weeks prior to the experimental commencement. At 18-weeks of age, test articles are provided to the mice via oral gavage. Control groups are provided sterile water at a dose of 5mL/kg body weight. Metformin treatment is provided at a dose of 100mg/kg body weight either independently, or in combination with the upgraded probiotic assemblage. Upgraded probiotic assemblages are provided at a dose of 5x10⁹ CFUs/dose. Mice receive a daily oral gavage of saline (control), metformin, upgraded probiotic assemblage, or upgraded probiotic assemblage in combination with metformin, to quantify the ability of the upgraded probiotic assemblage to improve metformin efficacy. Daily gavages continue for 8 weeks, at which point glucose tolerance tests and insulin tolerance tests are performed to evaluate the metabolic health of each mouse. Each week, mice are weighed, and fecal samples are collected to evaluate changes in the microbial composition over time. At sacrifice, adipose tissue depots, blood, liver, small intestine, and colonic tissue from each mouse are collected for downstream mechanistic analysis.

*Oral glucose tolerance test (OGTT).* After 8 weeks of dosing mice with test article, an OGTT is performed. Here, mice are fasted for 6 hours after which fasting blood glucose levels were measured via tail vein blood using a glucometer (One-Touch Ultra II). Mice are then dosed with an oral glucose bolus (2g/kg) via oral gavage, and blood glucose is measured at 20, 40, 60, and 120 minutes post gavage.

*Insulin Tolerance Test (ITT).* 8 weeks after the first dose of test material, mice are fasted for 4-hours and a baseline blood glucose level measurement is recorded using a glucometer (One-Touch Ultra II). Following baseline measurements, mice receive an intraperitoneal (IP) injection of insulin (10mL/kg at a concentration of 0.1U/mL). After injection, blood glucose is measured at 15, 30, 60, 90, and 120 minutes via tail vein blood.

*Body composition.* Body fat percentage is determined using Dual Energy x-ray Absorptiometry (DEXA) scan (PIXImus2 Mouse Densitometer; GE) 8 weeks after initiation of treatment. Prior to DEXA scans, mice are anesthetized via intraperitoneal injection of ketamine (60 mg/kg) and xylazine (4 mg/kg).

### Example 10. Upgraded Probiotic Assemblage Improves the Efficacy of an Osteoporosis Drug When Used in Combination

The upgraded probiotic assemblages described herein can be used in combination with FDA approved drugs for treating osteoporosis including but not limited to, bisphosphonates (alendronate, risedronate, ibandronate, zolendronate), biologics (denosumab, romosozumab), selective estrogen receptor mediators (Raloxifene), or anabolic agents (teriparatide, abaloparatide), to improve the efficacy and/or reduce the side effects of said drug.

To test the combination of the upgraded probiotic assemblages with anti-osteoporosis drugs, lead candidate upgraded probiotic assemblages are evaluated for their therapeutic efficacy in an ovariectomized (OVX) mouse model of postmenopausal osteoporosis either alone, or in combination with alendronate. All mice are group housed with 5 mice per cage in individually ventilated cages (IVCs) specifically designed for germ free husbandry [59, 60]. At 12-weeks of age, mice are weighed, have baseline feces collected, and undergo OVX (N=40) or sham (N=10) surgery to deplete estrogen levels and commence the bone resorption process as previously described [61]. Prior to surgery, mice receive a DXA scan to evaluate baseline BMD. 1- day post-surgery, mice begin a daily oral gavage regimen (200 µL) of water (negative control), alendronate, upgraded probiotic assemblage, or alendronate plus upgraded probiotic assemblage, and continue for 6-weeks. Bi-weekly fecal samples are collected to monitor the composition of the gut microbiome over time. Finally, on the last day of the study, mice received a second DXA scan to evaluate BMD, followed by euthanasia.

*Tissue collection and analysis:* At the time of sacrifice, the uterus is removed and weighed to confirm that the ovaries were successfully removed, and estrogen was depleted following OVX surgery. Serum is collected to analyze bone turnover markers including C-terminal telopeptide of collagen (CTX), a marker of resorption, and osteocalcin, a marker of formation, as previously described, as well as inflammatory cytokines and osteoclastogenic markers including TNF, IL-1, IFNγ, IL-17, and RANKL. Serum CTX and osteocalcin are analyzed by rodent specific ELISA (immunodiagnostic systems) while inflammatory markers are analyzed by multiplex assay at EveTechnologies (Calgary, AB). Tissues are then collected from each mouse to evaluate the impact of our therapeutic intervention on intestinal barrier integrity and inflammation, as well as bone quantity and strength. Colonic tissues is isolated and flash frozen for RNA and protein evaluation of tight junctions and claudins, critical mediators of gut barrier integrity, as well as inflammatory cytokine levels including TNF, IL-17, IFNγ and IL-1, markers of Th-17 and macrophage mediated inflammation [5, 58]. Cecal contents are removed and flash frozen for downstream metagenomic sequencing and SCFA analysis by GC-FID to determine how the upgraded probiotic assemblage impacted the composition and function of the gut microbiome. Finally, both femurs and the lumbar spine are removed and processed skeletal analysis. Here, distal femurs and vertebra are analyzed by MicroCT and histomorphometry to observe changes in cortical and trabecular morphometry and cellularity. Additionally, one femur from each mouse is subjected to biomechanical testing including 3-point bending to determine the impact of probiotic assemblage therapy on skeletal strength.

*Metagenomic Analysis:* Comprehensive functional analysis of the fecal microbiome identifies functions enriched in response to probiotic assemblage gavage. Shotgun sequencing libraries of fecal DNA are created using Illumina Nextera Flex and sequenced on an Illumina HiSeqX followed by standard metagenomic pipelines.

All references, issued patents and patent applications cited within the body of the instant specification are hereby incorporated by reference in their entirety, for all purposes.

### REFERENCES

Smith and Waterman. Comparison of biosequences. Adv. Appl. Math. 2:482 (1981)
Needleman and Wunsch. A General Method Applicable to the Search for Similarities in the Amino Acid Sequence of Two Proteins. J. Mol. Biol. 48:443 (1970)
Pearson and Lipman. Improved Tools for Biological Sequence Comparison. Proc. Nat'l. Acad. Sci. USA 85:2444 (1988)
Altschul et al. Basic local alignment search tool. J. Mol. Biol., 215:403-410 (1990)
Schoch CL, et al. Nuclear ribosomal internal transcribed spacer (ITS) region as a universal DNA barcode marker for Fungi. Proc Natl Acad Sci USA 109:6241-6246 (2012)
Rathbone et al. Modified-Release Drug Delivery Technology (Drugs and the Pharmaceutical Sciences Book 184) 2nd Edition, Marcel Dekker, Inc. New York, N.Y. (2002).
The Handbook of Pharmaceutical Excipients. 6th edition. American Pharmaceutical Association (1986)
Remington's pharmaceutical sciences, 17th edition. Edited by Alfonso R. Gennaro. Mack Publishing Co., 20th and Northampton Streets, Easton, PA 18042 (1985)
Magnusdottir et al. Generation of genome-scale metabolic reconstructions for 773 members of the human gut microbiota. Nat. Biotech. 35, 81-8 (2016)
Cox et al. SolexaQA: At-a-glance quality assessment of Illumina second-generation sequencing data. BMC Bioinformatics. 11:485 (2010)
Peng Y., et al. IDBA-A Practical Iterative de Bruijn Graph De Novo Assembler. Lisbon: RECOMB; (2010)
Zhang et al. dbCAN2: a meta server for automated carbohydrate-active enzyme annotation. Nucleic Acids Res. 46(W1):W95-W101 (2018)
Yin et al. dbCAN: a web resource for automated carbohydrate-active enzyme annotation. Nucleic Acids Res. 40 (2012)
Truong et al. MetaPhlAn2 for enhanced metagenomic taxonomic profiling. Nature Methods. 12:10 (2015)
Franzosa et al. Species-level functional profiling of metagenomes and metatranscriptomes Nat. Methods. 15. 962-968 (2018)
Kim et al. FMAP: Functional Mapping and Analysis Pipeline for metagenomics and metatranscriptomics studies. BMC Bioinf. 17:420 (2016)
Das et al. Metagenomic analysis of microbe-mediated vitamin metabolism in the human gut microbiome. BMC Genomics. 20(1):208 (2019)
Fang H. et al. Microbial production of vitamin B 12: a review and future perspectives. Microb Cell Fact. 16(1):15 (2017)
Briani C et al. Cobalamin Deficiency: Clinical Picture and Radiological Findings. Nutrients. 5:11, 4521-4539. (2013)
Issac I, et al. Vitamin B12 Deficiency: An Important Reversible Co-Morbidity in Neuropsychiatric Manifestations. Indian Journal of Psychiatric Medicine. 37:1, 26-29. (2015)
Kok D, et al. Bacterial folate biosynthesis and colorectal cancer risk: more than just a gut feeling. Critical Reviews in Food Science and Nutrition. 60:2, 244-256 (2018)
Terry P, et al. Dietary intake of folic acid and colorectal cancer risk in a cohort of women. International Journal of Cancer. 97:6, 864-867 (2002)
D'Aimmo MR., et. al. The potential of bifidobacteria as a source of natural folate. Journal of Applied Microbiology. 112:975-984 (2012).
Sugahara H., et. al. Probiotic Bifidobacterium longum alters gut luminal metabolism through modification of the gut microbial community. Scientific Reports. 13548 (2015).
Li, P., Gu, Q., Zhou, Q. Complete genome sequence of Lactobacillus plantarum LZ206, a potential probiotic strain with antimicrobial activity against food-borne pathogenic microorganisms. Journal of Biotechnology. 238:(52-55). (2016).
Laiño JE., et. al. Applicability of a Lactobacillus amylovorus strain as a co-culture for natural folate bio-enrichment of fermented milk. International Journal of Food Microbiology. 191:10-16. (2014).

### ADDITIONAL SEQUENCES

| |
|---|
| **Seq ID No 166 (DP67 16S)** |
| TCGAGCGGACAGATGGGAGCTTGCTCCCTGATGTTAGCGGCGGACGGGTGAGTAACACGTGGG TAACCTGCCTGTAAGACTGGGATAACTCCGGGAAACCGGGGCTAATACCGGATGCTTGTTTGAA CCGCATGGTTCAAACATAAAAGGTGGCTTCGGCTACCACTTACAGATGGACCCGCGGCGCATTA GCTAGTTGGTGAGGTAATGGCTCACCAAGGCAACGATGCGTAGCCGACCTGAGAGGGTGATCG GCCACACTGGGACTGAGACACGGCCCAGACTCCTACGGGAGGCAGCAGTAGGGAATCTTCCGC AATGGACGAAAGTCTGACGGAGCAACGCCGCGTGAGTGATGAAGGTTTTCGGATCGTAAAGCT CTGTTGTTAGGGAAGAACAAGTGCCGTTCAAATAGGGCGGCACCTTGACGGTACCTAACCAGAA AGCCACGGCTAACTACGTGCCAGCAGCCGCGGTAATACGTAGGTGGCAAGCGTTGTCCGGAATT ATTGGGCGTAAAGGGCTCGCAGGCGGTTTCTTAAGTCTGATGTGAAAGCCCCCGGCTCAACCGG GGAGGGTCATTGGAAACTGGGGAACTTGAGTGCAGAAGAGGAGAGTGGAATTCCACGTGTAGC GGTGAAATGCGTAGAGATGTGGAGGAACACCAGTGGCGAAGGCGACTCTCTGGTCTGTAACTG ACGCTGAGGAGCGAAAGCGTGGGGAGCGAACAGGATTAGATACCCTGGTAGTCCACGCCGTAA ACGATGAGTGCTAAGTGTTAGGGGGTTTCCGCCCCTTAGTGCTGCAGCTAACGCATTAAGCACT CCGCCTGGGGAGTACGGTCGCAAGACTGAAACTCAAAGGAATTGACGGGGGCCCGCACAAGCG GTGGAGCATGTGGTTTAATTCGAAGCAACGCGAAGAACCTTACCAGGTCTTGACATCCTCTGAC ACCCTAGAGATAGGGCTTCCCTTCGGGG |
| **Seq ID No 167 (DP68 16S)** |
| TGCAGTCGAGCGGACAGATGGGAGCTTGCTCCCTGATGTTAGCGGCGGACGGGTGAGTAACAC GTGGGTAACCTGCCTGTAAGACTGGGATAACTCCGGGAAACCGGGGCTAATACCGGATGCTTGT TTGAACCGCATGGTTCAAACATAAAAGGTGGCTTCGGCTACCACTTACAGATGGACCCGCGGCG CATTAGCTAGTTGGTGAGGTAATGGCTCACCAAGGCAACGATGCGTAGCCGACCTGAGAGGGT GATCGGCCACACTGGGACTGAGACACGGCCCAGACTCCTACGGGAGGCAGCAGTAGGGAATCT TCCGCAATGGACGAAAGTCTGACGGAGCAACGCCGCGTGAGTGATGAAGGTTTTCGGATCGTA AAGCTCTGTTGTTAGGGAAGAACAAGTGCCGTTCAAATAGGGCGGCACCTTGACGGTACCTAAC CAGAAAGCCACGGCTAACTACGTGCCAGCAGCCGCGGTAATACGTAGGTGGCAAGCGTTGTCC GGAATTATTGGGCGTAAAGGGCTCGCAGGCGGTTTCTTAAGTCTGATGTGAAAGCCCCCGGCTC AACCGGGGAGGGTCATTGGAAACTGGGGAACTTGAGTGCAGAAGAGGAGAGTGGAATTCCACG TGTAGCGGTGAAATGCGTAGAGATGTGGAGGAACACCAGTGGCGAA |
| **Seq ID No 168 (DP69 16S)** |
| TGCAGTCGAGCGGACAGATGGGAGCTTGCTCCCTGATGTTAGCGGCGGACGGGTGAGTAACAC GTGGGTAACCTGCCTGTAAGACTGGGATAACTCCGGGAAACCGGGGCTAATACCGGATGCTTGT TTGAACCGCATGGTTCAAACATAAAAGGTGGCTTCGGCTACCACTTACAGATGGACCCGCGGCG CATTAGCTAGTTGGTGAGGTAATGGCTCACCAAGGCAACGATGCGTAGCCGACCTGAGAGGGT GATCGGCCACACTGGGACTGAGACACGGCCCAGACTCCTACGGGAGGCAGCAGTAGGGAATCT TCCGCAATGGACGAAAGTCTGACGGAGCAACGCCGCGTGAGTGATGAAGGTTTTCGGATCGTA AAGCTCTGTTGTTAGGGAAGAACAAGTGCCGTTCAAATAGGGCGGCACCTTGACGGTACCTAAC CAGAAAGCCACGGCTAACTACGTGCCAGCAGCCGCGGTAATACGTAGGTGGCAAGCGTTGTCC GGAATTATTGGGCGTAAAGGGCTCGCAGGCGGTTTCTTAAGTCTGATGTGAAAGCCCCCGGCTC AACCGGGGAGGGTCATTGGAAACTGGGGAACTTGAGTGCAGAAGAGGAGAGTGGAATTCCACG TGTAGCGGTG |
| **Seq ID No 169 (DP70 16S)** |
| TGCAAGTCGAGCGGACAGATGGGAGCTTGCTCCCTGATGTTAGCGGCGGACGGGTGAGTAACA CGTGGGTAACCTGCCTGTAAGACTGGGATAACTCCGGGAAACCGGGGCTAATACCGGATGGTTG TTTGAACCGCATGGTTCAAACATAAAAGGTGGCTTCGGCTACCACTTACAGATGGACCCGCGGC GCATTAGCTAGTTGGTGAGGTAACGGCTCACCAAGGCAACGATGCGTAGCCGACCTGAGAGGG TGATCGGCCACACTGGGACTGAGACACGGCCCAGACTCCTACGGGAGGCAGCAGTAGGGAATC TTCCGCAATGGACGAAAGTCTGACGGAGCAACGCCGCGTGAGTGATGAAGGTTTTCGGATCGTA AAGCTCTGTTGTTAGGGAAGAACAAGTACCGTTCGAATAGGGCGGTACCTTGACGGTACCTAAC CAGAAAGCCACGGCTAACTACGTGCCAGCAGCCGCGGTAATACGTAGGTGGCAAGCGTTGTCC GGAATTATTGGGCGTAAAGGGCTCGCAGGCGGTTTCTTAAGTCTGATGTGAAAGCCCCCGGCTC AACCGGGGAGGGTCATTGGAAACTGGGGAACTTGAGTGCAGAAGAGGAGAGTGGAATTCCACG TGTAGCGGTGAAATGCGTAGAGATGTGGAGGAACACCAGTGGCGAAGGCGACTCTCTGGTCTG TAACTGACGCTGANGAGCGAAAGCGTGGGGAGCGAACAGGATTAGATACCCTGGTAGTCCACG CCGTAAACGATGAGTGCTAAGTGTTA |
| **Seq ID No 170 (DP71 16S)** |
| TTACTTGGAGTCCGAACTCTCACTTTTTAACCCTGTGCATCTGTTAATTGGAATAGTAGCTCTTC GGAGTGAACCACCATTCACTTATAAAACACAAAGTCTATGAATGTATACAAATTTATAACAAAA CAAAACTTTCAACAACGGATCTCTTGGCTCTCGCATCGATGAAGAACGCAGCGAAATGCGATAC GTAATGTGAATTGCAGAATTCAGTGAATCATCGAATCTTTGAACGCACCTTGCGCTCCTTGGTAT TCCGAGGAGCATGCCTGTTTGAGTGTCATGAAATCTTCAACCCACCTCTTTCTTAGTGAATCTGG TGGTGCTTGGTTTCTGAGCGCTGCTCTGCTTCGGCTTAGCTCGTTCGTAATGCATTAGCATCCGC AACCGAACTTCGGATTGACTTGGCGTAATAGACTATTCGCTGAGGATTCTAGTTTACTAGAGCC GAGTTGGGTTAAAGGAAGCTCCTAATCCTAAAGTCTATTTTTGATTAGATCTCAAATCAGGTAG GACTACCCGCTGAACTTAAGCATATCAATAAGCGGAGGAAAAGAAACTAACAAGGATTCCCCT AGTAGCGGCGAGCGAAGCGGGAAGAGCTCAAATTTATAATCTGGCACCTTCGGTGTCCGAGTTG TAATCTCTAGAAGTGTTTTCCGCGTTGGACCGCACACAAGTCTGTTGGAATACAGCGGCATAGT GGTGAAACCCCCGTATATGGTGCGGACGCCCAGCGCTTTGTGATACACTTTCAATGAGTCGAGT TGTTTGGGAATGCAGCTCAAATTGGGTGGTAAATTCCATCTAAAGCTAAATATTGGCGAGAGAC CGATAGCGAACAAGTACCGTGAGGGAAAGATGAAAAGCACTTTGGAAAGAGAGTTAACAGTAC GTGAAATTGTTGGAA |

Aspects and features of the present disclosure are set out in the following numbered clauses which contain the subject matter of the claims of the parent application as filed.
1. A probiotic composition comprising a plurality of viable microbial entities,
   comprising:
   a. a first microbial entity, wherein the first microbial entity is a gamma proteobacterium, a fungus, or a lactic acid bacterium presented in Table 4 or Table 7;
   b. a second microbial entity, wherein the second microbial entity is a probiotic microorganism; and
   c. at least one prebiotic compound, wherein the prebiotic compound comprises a prebiotic polysaccharide or a prebiotic fiber, and
   wherein at least one of the first or second microbial entities comprises an agriculturally-derived microbial entity, and wherein the first and second microbial entities are distinct species.
2. The probiotic composition of clause 1, wherein the first and second microbial entity are capable of a first functional interaction.
3. The probiotic composition of clause 2, wherein the first functional interaction comprises synthesis of a beneficial microbially-produced compound.
4. The probiotic composition of clause 3, wherein the beneficial microbially-produced compound comprises a short chain fatty acid or vitamin selected from the group consisting of: acetate, propionate, butyrate, vitamin B12, K2, folate, and combinations thereof.
5. The probiotic composition of any one of clauses 1-4, wherein the composition comprises at least two distinct microbial species selected from Table 4 or Table 7.
6. The probiotic composition of any one of clauses 1-4, wherein the composition comprises at least 3, at least 4, at least 5, or at least 6 distinct microbial species selected from Table 4 or Table 7.
7. The probiotic composition of any one of clauses 1-6, wherein the composition comprises at least one gamma proteobacterium, at least one fungus, and at least one lactic acid bacterium.
8. The probiotic composition of clause 7, wherein each of the at least one gamma proteobacterium, the at least one fungus, and the at least one lactic acid bacterium are selected from Table 4 or Table 7.
9. The probiotic composition of any one of clauses 2-8, wherein the prebiotic compound is capable of being metabolized by at least one of the viable microbial entities present in the probiotic composition.
10. The probiotic composition of clause 9, wherein the prebiotic compound is capable of being metabolized by the first or the second microbial entity.
11. The probiotic composition of any one of clauses 9 or 10, wherein the prebiotic compound is capable of being metabolized to produce acetate, propionate, butyrate, or combinations thereof.
12. The probiotic composition of any one of the preceding clauses, wherein the second microbial entity is selected from the group consisting of: a *Lactobacillus sp., a Bifidobacterium sp., a Bacillus sp.,* and a *Clostridium sp..*
13. The probiotic composition of any one of the preceding clauses, wherein the second microbial entity is selected from the group consisting of: *Lactobacillus reuteri, Lactobacillus paracasei, Lactobacillus salivarius Lactobacillus plantarum,Lactobacillus acidophilus, Lactobacillus reuteri protectis, Lactobacillus bulgaricus, Lactobacillus rhamnosus, Lactobacillus casei, Lactobacillus delbreukeii, Bifidobacterium infantis, Bifidobacterium longum, Bifidobacterium bifidum, Bifidobacterium breve, Streptococcus thermophilus, Escherichia coli Nissle, Lactococcus lactis, Bacillus subtilis, Bacillus clausii, Bacillus coagulans, Clostridium butyricum, Akkermansia muciniphila, Hafnia alvei, and Saccharomyces boulardii.*
14. The probiotic composition of any one of the preceding clauses, wherein at least one of the viable microbial entities comprises a nucleic acid sequence having at least about 97% identity at the 16S or ITS locus with a sequence shown in SEQ ID NO: 1-170.
15. The probiotic composition of any one of the preceding clauses, wherein the first microbial entity is isolated from a food or food product.
16. The probiotic composition of clause 15, wherein the first microbial entity is isolated from a fruit or vegetable.
17. The probiotic composition of clause 15 or 16, wherein the first microbial entity is isolated from a pickled food.
18. The probiotic composition of clause 15 or 16, wherein the first microbial entity is isolated from a fermented food.
19. The probiotic composition of clause 15 or 16, wherein the first microbial entity is isolated from a raw agricultural product.
20. The probiotic composition of any one of the preceding clauses, wherein the first microbial entity is isolated from edible tissues of an agricultural product.
21. The probiotic composition of any one of the preceding clauses, wherein the first microbial entity is isolated from an extract, fraction, tissue or portion of an agricultural product.
22. The probiotic composition of any one of the preceding clauses, wherein the second microbial entity is a generally regarded as safe (GRAS) probiotic microorganism or probiotic microorganism approved for human consumption.
23. The probiotic composition of any one of the preceding clauses, wherein the second microbial entity is *Lactobacillus plantarum.*
24. The probiotic composition of any one of the preceding clauses, wherein the second microbial entity is *Lactobacillus brevis.*
25. A probiotic assemblage comprising a first purified microbial entity isolated from a first plant-based sample selected from the group consisting of the samples presented in Table 3, a second purified microbial entity isolated from a second plant-based sample from selected from the group consisting of the samples presented in Table 3, wherein the first purified microbial entity is artificially-associated with the second purified microbial entity, and a third microbial entity, wherein the third microbial entity is a probiotic microorganism.
26. The probiotic assemblage of clause 25, wherein the composition is capable of a first functional interaction.
27. The probiotic assemblage of clause 26, wherein the first functional interaction comprises synthesis of a beneficial microbially-produced compound.
28. The probiotic assemblage of clause 27, wherein the wherein the beneficial microbially-produced compound comprises a short chain fatty acid or vitamin selected from the group consisting of: acetate, propionate, butyrate, vitamin B12, K2, folate, and combinations thereof.
29. The probiotic assemblage of any one of clauses 25-28, further comprising a prebiotic polysaccharide or a prebiotic fiber.
30. The probiotic assemblage of clause 29, wherein the prebiotic polysaccharide is oligofmctose or fmctooligosaccharide.
31. The probiotic assemblage of clause 29, wherein the prebiotic polysaccharide comprises a plant or plant extract.
32. The probiotic assemblage of any one of clauses 29-31, wherein the prebiotic polysaccharide or the prebiotic fiber is capable of being metabolized by at least one microbial entities present in the probiotic composition, optionally by the first and/or the second purified microbial entities.
33. The probiotic assemblage of any one of clauses 29-32, wherein the prebiotic polysaccharide or the prebiotic fiber is capable of being metabolized to produce acetate, propionate, butyrate or combinations thereof.
34. The probiotic composition of any one of clauses 25-33, wherein the probiotic microorganism is selected from the group consisting of: a *Lactobacillus sp., a Bifidobacterium sp., a Bacillus sp.,* or a *Clostridium sp.,* optionally wherein the probiotic microorganism is selected from the group consisting of: *Lactobacillus reuteri, Lactobacillus paracasei, Lactobacillus salivarius Lactobacillus plantarum, Lactobacillus acidophilus, Lactobacillus reuteri protectis, Lactobacillus bulgaricus, Lactobacillus rhamnosus, Lactobacillus casei, Lactobacillus delbreukeii, Bifidobacterium infantis, Bifidobacterium longum, Bifidobacterium bifidum, Bifidobacterium breve, Streptococcus thermophilus, Escherichia coli Nissle, Lactococcus lactis, Bacillus subtilis, Bacillus clausii, Bacillus coagulans, Clostridium butyricum, Akkermansia muciniphila, Hafnia alvei,* and *Saccharomyces boulardii.*
35. The probiotic composition of any one of clauses 25 to 33, wherein at least one of the first or the second purified microbial entities comprises a nucleic acid sequence having at least about 97% identity at the 16S or ITS locus with a sequence shown in SEQ ID NO: 1-170.
36. A probiotic composition comprising at least one probiotic microorganism, and at least one second microbial entity classified as a gamma proteobacterium, fungus, or lactic acid bacterium, optionally wherein the at least one second microbial entity is as a gamma proteobacterium, fungus, or lactic acid bacterium presented in Table 4 or Table 7, and wherein the at least one probiotic microorganism and the at least one second microbial entity are capable of combining with a food product to become a functional food.
37. The probiotic composition of clause 36, further comprising a prebiotic polysaccharide or a prebiotic fiber providing a carrier for the probiotic composition and to enhance the taste and flavor properties of the food product.
38. The probiotic composition of clause 36 or 37, further comprising a prebiotic polysaccharide or a prebiotic fiber in dry or liquid form, wherein the prebiotic polysaccharide or the prebiotic fiber acts as a cryoprotectant.
39. The probiotic composition of any one of clauses 36-38, wherein the probiotic composition can be combined with a dairy product, optionally wherein the dairy product is selected from the group consisting of: yogurt, shake, smoothie, and cheese.
40. The probiotic composition of any one of clauses 37-39, wherein the prebiotic polysaccharide or the prebiotic fiber is in powdered form, and optionally provides flavorings, and wherein the prebiotic polysaccharide or the prebiotic fiber is capable of being mixed in a liquid to flavor the liquid, optionally wherein the flavor is selected from the group consisting of grape, strawberry, cranberry, blueberry, lime, lemon, and chocolate.
41. The probiotic composition of any one of clauses 37-40, wherein the prebiotic polysaccharide or the prebiotic fiber comprises a plant or plant extract in the form of a juice, smoothie, shake, dry powder, tablet, granules, pellet, emulsion, paste, jelly, ferment, syrup, or brine.
42. The probiotic composition of any one of clauses 36-41, wherein the prebiotic polysaccharide or the prebiotic fiber is derived from an agricultural product farmed using organic practices.
43. The probiotic composition of any one of clauses 36 to 42, wherein the at least one probiotic microorganism is selected from the group consisting of: a *Lactobacillus sp., a Bifidobacterium sp., a Bacillus sp.,* and a *Clostridium sp.*
44. A fermented probiotic composition comprising a *Pediococcus pentosaceus* microbial entity and/or a *Leuconostoc mesenteroides* microbial entity, a non-lactic acid bacteria microbial entity presented in Table 4 or Table 7, and a third microbial entity comprising a probiotic microorganism, wherein the fermented probiotic composition is encapsulated in a capsule or a microcapsule adapted for enteric delivery.
45. The fermented probiotic composition of clause 44, further comprising a prebiotic polysaccharide or a prebiotic fiber.
46. The fermented probiotic composition of clause 45, wherein the prebiotic polysaccharide is oligofructose or fructooligosaccharide.
47. The fermented probiotic composition of clause 45, wherein the prebiotic polysaccharide comprises a plant or plant extract.
48. The fermented probiotic composition of any one of clauses 44-47, wherein the prebiotic polysaccharide or the prebiotic fiber is capable of being metabolized by at least one of the microbial entities present in the fermented probiotic composition.
49. The fermented probiotic composition of any one of clauses 44-47, wherein the prebiotic polysaccharide or the prebiotic fiber is capable of being metabolized by at least one of the microbial entities present in the probiotic composition to produce acetate, propionate, butyrate, or combinations thereof.
50. A fermented probiotic composition comprising a *Pichia kudriavzevii* microbial entity and/or a *Leuconostoc mesenteroides* microbial entity, a non-lactic acid bacteria microbial entity presented in Table 4 or Table 7, and a third microbial entity comprising a probiotic microorganism, wherein the fermented probiotic composition is encapsulated in a capsule or a microcapsule adapted for enteric delivery.
51. The fermented probiotic composition of clause 50, further comprising a prebiotic polysaccharide or a prebiotic fiber.
52. The fermented probiotic composition of clause 51, wherein the prebiotic polysaccharide is oligofructose or fructooligosaccharide.
53. The fermented probiotic composition of clause 51, wherein the prebiotic polysaccharide comprises a plant or plant extract.
54. The fermented probiotic composition of any one of clauses 51-53, wherein the prebiotic polysaccharide or the prebiotic fiber is capable of being metabolized by at least one of the microbial entities present in the fermented probiotic composition.
55. The fermented probiotic composition of any one of clauses 51-54, wherein the prebiotic polysaccharide or the prebiotic fiber is capable of being metabolized by at least one microbial entity present in the probiotic composition to produce acetate, propionate, butyrate, or combinations thereof.
56. Any of the above compositions further comprising a drug.
57. The composition of clause 56, wherein the drug is selected from the group consisting of: Metformin, Acarbose, Miglitol, Voglibose, Sitagliptin, Saxagliptin, Liraglutide, Pioglitazone, dipeptidyl peptidase-4 (DPP4)-inhibitors, glucagon-like peptide- 1 (GLP-1) receptor analogs, alpha glucosidase inhibitors, thiazolidinedione, sodium/glucose cotransporter 2 (SGLT2) inhibitors, bisphosphonates, biologies (, selective estrogen receptor mediators, and anabolic agents.
58. A method for improving the efficacy of a probiotic microorganism in improving or maintaining health in a human subject, the method comprising administering any of the above compositions.
59. A method for treating Type 2 diabetes, the method comprising administering any of the above compositions.
60. A method for treating osteoporosis, the method comprising administering any of the above compositions.
61. A method for improving the efficacy of a probiotic microorganism in improving or maintaining health in a human subject, the method comprising co-administration of the probiotic microorganism to the subject in combination with an upgraded probiotic composition comprising a microbial entity, wherein the microbial entity is a gamma proteobacterium, a fungus, or a lactic acid bacterium presented in Table 4 or Table 7, and wherein the upgraded probiotic composition comprises a prebiotic polysaccharide or a prebiotic fiber.
62. A method for treating Type 2 diabetes, the method comprising co-administration of the probiotic microorganism to the subject in combination with an upgraded probiotic composition comprising a microbial entity, wherein the microbial entity is a gamma proteobacterium, a fungus, or a lactic acid bacterium presented in Table 4 or Table 7, and wherein the upgraded probiotic composition comprises a prebiotic polysaccharide or a prebiotic fiber.
63. A method for treating osteoporosis, the method comprising co-administration of the probiotic microorganism to the subject in combination with an upgraded probiotic composition comprising a microbial entity, wherein the microbial entity is a gamma proteobacterium, a fungus, or a lactic acid bacterium presented in Table 4 or Table 7, and wherein the upgraded probiotic composition comprises a prebiotic polysaccharide or a prebiotic fiber.
64. The method of any one of clauses 61-63, wherein the probiotic microorganism and the upgraded probiotic composition are co-formulated.
65. A method to formulate an upgraded probiotic assemblage, wherein the method comprises artificially-associating a first purified microbial entity isolated from a first plant-based sample selected from the group consisting of the samples presented in Table 3, a second purified microbial entity isolated from a second plant-based sample from selected from the group consisting of the samples presented in Table 3, and a third microbial entity, wherein the third microbial entity is a probiotic microorganism, wherein the upgraded probiotic assemblage is predicted using a computational simulation to modulate production of one or more branched chain fatty acids, short chain fatty acids, and/or flavones in a mammalian gut.
66. The probiotic composition or method of any one of the preceding clauses, wherein the probiotic microorganism is selected from the group consisting of: *Lactobacillus reuteri, Lactobacillus paracasei, Lactobacillus salivarius Lactobacillus plantarum, Lactobacillus acidophilus, Lactobacillus reuteri protectis, Lactobacillus bulgaricus, Lactobacillus rhamnosus, Lactobacillus casei, Lactobacillus delbreukeii, Bifidobacterium infantis, Bifidobacterium longum, Bifidobacterium bifidum, Bifidobacterium breve, Streptococcus thermophilus, Escherichia coli Nissle, Lactococcus lactis, Bacillus subtilis, Bacillus clausii, Bacillus coagulans, Clostridium butyricum, Akkermansia muciniphila, Hafnia alvei,* and *Saccharomyces boulardii.*
67. The probiotic composition or method of any one of the preceding clauses, wherein the probiotic microorganism is a generally regarded as safe (GRAS) probiotic microorganism.
68. The probiotic composition or method of any one of the preceding clauses, wherein the probiotic microorganism is approved for human consumption.
69. The probiotic composition or method of any one of the preceding clauses, wherein the probiotic microorganism is *Lactobacillus plantarum.*
70. The probiotic composition or method of any one of the preceding clauses, wherein the probiotic microorganism is *Lactobacillus brevis.*
71. The probiotic composition or method of any one of the preceding clauses, wherein the prebiotic polysaccharide or the prebiotic fiber acts as a cryoprotectant.
72. The probiotic composition or method of clause 71, wherein no other cryoprotectant is present other than the prebiotic polysaccharide or the prebiotic fiber.
73. The probiotic composition or method of any one of the preceding clauses, wherein the microbial entity selected from Table 4 or Table 7 comprises a nucleic acid sequence having at least 97% identity at the 16S or ITS locus with a sequence shown in SEQ ID NO: 1-170.
74. The probiotic composition or method of any one of the preceding clauses, wherein the microbial entity selected from Table 4 or Table 7 comprises a nucleic acid sequence having at least 98% identity at the 16S or ITS locus with a sequence shown in SEQ ID NO: 1-170.
75. The probiotic composition or method of any one of the preceding clauses, wherein the microbial entity selected from Table 4 or Table 7 comprises a nucleic acid sequence having at least 99% identity at the 16S or ITS locus with a sequence shown in SEQ ID NO: 1-170.
76. The probiotic composition or method of any one of the preceding clauses, wherein the microbial entity selected from Table 4 or Table 7 comprises a nucleic acid sequence having 100% identity at the 16S or ITS locus with a sequence shown in SEQ ID NO: 1-170.
77. The probiotic composition or method of any one of the preceding clauses, wherein each of the microbial entities selected from Table 4 or Table 7 comprise a nucleic acid sequence having at least 97% identity at the 16S or ITS locus with a sequence shown in SEQ ID NO: 1-170.
78. The probiotic composition or method of any one of the preceding clauses, wherein each of the microbial entities selected from Table 4 or Table 7 comprise a nucleic acid sequence having at least 98% identity at the 16S or ITS locus with a sequence shown in SEQ ID NO: 1-170.
79. The probiotic composition or method of any one of the preceding clauses, wherein each of the microbial entities selected from Table 4 or Table 7 comprise a nucleic acid sequence having at least 99% identity at the 16S or ITS locus with a sequence shown in SEQ ID NO: 1-170.
80. The probiotic composition or method of any one of the preceding clauses, wherein each of the microbial entities selected from Table 4 or Table 7 comprise a nucleic acid sequence having 100% identity at the 16S or ITS locus with a sequence shown in SEQ ID NO: 1-170.
81. The probiotic composition or method of any one of the preceding clauses, wherein the microbial entities selected from Table 4 or Table 7 comprise each of the microbial entities having a nucleic acid sequence having at least 97% identity at the 16S or ITS locus with SEQ ID NO: 1, SEQ ID NO: 157, SEQ ID NO: 158, SEQ ID NO: 163, and SEQ ID NO: 165.
82. The probiotic composition or method of any one of the preceding clauses, wherein the microbial entities selected from Table 4 or Table 7 comprise each of the microbial entities having a nucleic acid sequence having at least 98% identity at the 16S or ITS locus with SEQ ID NO: 1, SEQ ID NO: 157, SEQ ID NO: 158, SEQ ID NO: 163, and SEQ ID NO: 165.
83. The probiotic composition or method of any one of the preceding clauses, wherein the microbial entities selected from Table 4 or Table 7 comprise each of the microbial entities having a nucleic acid sequence having at least 99% identity at the 16S or ITS locus with SEQ ID NO: 1, SEQ ID NO: 157, SEQ ID NO: 158, SEQ ID NO: 163, and SEQ ID NO: 165.
84. The probiotic composition or method of any one of the preceding clauses, wherein the microbial entities selected from Table 4 or Table 7 comprise each of the microbial entities having a nucleic acid sequence having 100% identity at the 16S or ITS locus with SEQ ID NO: 1, SEQ ID NO: 157, SEQ ID NO: 158, SEQ ID NO: 163, and SEQ ID NO: 165.

## Claims

1. A probiotic composition comprising a plurality of viable microbial entities, comprising:
a. a first microbial entity, wherein the first microbial entity is the fungus *Pichia kudriavzevii;*
b. a second microbial entity, wherein the second microbial entity is *Lactobacillus plantarum* or *Lactobacillus brevis;* and
c. at least one prebiotic compound, wherein the prebiotic compound comprises a prebiotic polysaccharide or a prebiotic fiber.

2. The probiotic composition of claim 1, wherein the first and second microbial entity are capable of synthesis of a beneficial microbially-produced compound, optionally wherein the beneficial microbially-produced compound comprises a short chain fatty acid or vitamin selected from the group consisting of: acetate, propionate, butyrate, vitamin B12, K2, folate, and combinations thereof.

3. The probiotic composition of claim 2, wherein the beneficial microbially-produced compound is acetate.

4. The probiotic composition of any one of claims 1-3, wherein the composition comprises at least 3 distinct microbial species selected from Table 4 or Table 7.

5. The probiotic composition of any one of claims 1-4, wherein the composition comprises at least 4 distinct microbial species selected from Table 4 or Table 7.

6. The probiotic composition of any one of claims 2-5, wherein the prebiotic compound is capable of being metabolized by at least one of the viable microbial entities present in the probiotic composition, optionally wherein the prebiotic compound is capable of being metabolized to produce acetate, propionate, butyrate, or combinations thereof.

7. The probiotic composition of any one of the preceding claims, wherein at least one of the viable microbial entities comprises a nucleic acid sequence having at least about 97% identity at the 16S or ITS locus with a sequence shown in SEQ ID NO: 1-170.

8. The probiotic composition of any one of the preceding claims, wherein the second microbial entity is *Lactobacillus plantarum.*

9. The probiotic composition of any one of the preceding claims, wherein the second microbial entity is *Lactobacillus brevis.*

10. The probiotic assemblage of claim 1, wherein the prebiotic polysaccharide is oligofructose.

11. The composition of any preceding claim for use in treating Type 2 diabetes.

12. The composition of any preceding claim for use in treating osteoporosis.

13. The probiotic composition of any one of the preceding claims, wherein the prebiotic polysaccharide or the prebiotic fiber acts as a cryoprotectant, optionally wherein no other cryoprotectant is present other than the prebiotic polysaccharide or the prebiotic fiber.

14. The probiotic composition of any one of the preceding claims, wherein the first microbial entity comprises a nucleic acid sequence having at least 97%, at least 98%, at least 99%, or 100% identity at the ITS locus with SEQ ID NO: 165.

15. A probiotic microbial assemblage comprising *Lactobacillus brevis, Leuconostoc mesenteroides, Lactobacillus plantarum* and a yeast, wherein the yeast is *Pichia kudriavzevii.*
